# EUROPEAN PATENT APPLICATION

(11) **EP 4 273 140 A1**
(43) Date of publication of application: **08.11.2023**
(21) Application number: 22749997.7
(22) Date of filing: 28.01.2022
(51) Int. Cl.: C07D 401/12, A61K 31/506, A61K 31/505, A61K 31/444, A61K 31/4439, A61P 35/00, A61P 11/06, A61P 25/28, A61P 37/00, C07D 403/12

(54) **NOVEL COMPOUND AS PROTEIN KINASE INHIBITOR**

(30) Priority: 02.02.2021 KR 20210014929
(71) Applicant: Lg Chem, Ltd., Seoul 07336 (KR)
(72) Inventor: CHO, Joong Heui, Daejeon 34122 (KR); HAM, Young Jin, Daejeon 34122 (KR); KIM, Jung Joon, Daejeon 34122 (KR)
(74) Representative: Hoffmann Eitle
(86) International application number: PCT/KR2022/001641
(87) International publication number: WO 2022/169248

(57) **Abstract**

The present invention relates to a novel compound as a protein kinase inhibitor.

## Description

### TECHNICAL FIELD

### [Cross-reference to Related Applications]

The present application claims the benefit of priority based on Korean Patent Application No. 2021-0014929, filed on February 2, 2021, the entire contents of which are incorporated herein by reference.

### [Technical Field]

The present invention relates to a novel compound as protein kinase inhibitors. More particularly, the present invention relates to a novel compound as C-MET and/or RON inhibitors.

### BACKGROUND ART

At least 400 or more protein kinases are known, which catalyze the transphosphorylation from adenosine triphosphate (ATP) to a protein substrate. Since the specific amino acid in a target protein to which a phosphate group is transferred is tyrosine, serine or threonine, protein kinase enzymes are commonly referred to as protein tyrosine kinases (PTKs) or serine/threonine kinases (STKs).

Protein kinases are composed of a structurally relevant large group which is required for controlling a wide variety of intracellular signaling pathways. The signaling pathway includes many other signaling pathways by the transphosphorylation to a target protein. The phosphorylation process operates as a molecular on/off switch which may control the biological function of a target protein or protein complex. The proper performance of protein kinases in the signaling pathways is activation or deactivation of metabolizing enzymes, regulatory proteins, receptors, cytoskeleton proteins, ion channels and pumps, transcription factors, or the like. The inappropriate regulatory signaling due to defective regulation of protein phosphorylation is related to numerous diseases including inflammation, cancer, allergy/asthma, immunological disorder, central nervous system diseases, angiogenesis, or the like.

Most kinases include similar 250-300 amino acid catalyst sites. Kinases may be classified according to substrates that they phosphorylate, and in each of kinase groups, overall corresponding sequence motives have been confirmed.

Meanwhile, a mesenchymal-epithelial transition factor (c-MET) is referred to as a tyrosine protein kinase receptor or a hepatocellular growth factor receptor, and the c-MET is expressed in endothelial, epithelial, and mesenchymal cells. The c-MET is reported to be related to the progression of specific tumors, and the overexpression of c-MET is known to appear in numerous tumor types in addition to colon, breast, kidney, lung, hemangioma, squamous cell myeloid leukemia, melanoma, glioblastoma and astrocytoma.

In addition, a receptor originated from nantes (RON) which is one of tyrosine protein kinase receptors, is referred to as a macrophage stimulating 1 receptor (MST1R), and is reported to accelerate the invasion and metastasis of cancer cells. The overexpression of the RON is also known to appear in various tumor types.

The activation of the tyrosine protein kinases including the c-MET, the RON, or the like in tumor cells, increases the proliferation, invasion and metastasis of tumor cells and also increases the resistance to the apoptosis and cytotoxic therapy of tumor cells. Accordingly, selective small molecule kinase regulators targeting tyrosine protein kinases such as c-Met and RON are expected to have therapeutic possibility for the treatment of cancers, wherein the activation of the c-Met receptor and/or the RON receptor plays an important role in the occurrence and progression of a primary tumor and secondary metastasis. Accordingly, constant study on various inhibitors for suppressing the activation of tyrosine protein kinases including a c-MET receptor and/or RON, is being conducted.

### DISCLOSURE OF THE INVENTION

### TECHNICAL PROBLEM

In an aspect, the present invention intends to provide a novel compound having protein kinase inhibitory activity.

In another aspect, the present invention intends to provide a compound useful for the prevention and treatment of cancers.

In another aspect, the present invention intends to provide a compound useful for the prevention and treatment of immune-related diseases.

In another aspect, the present invention intends to provide a compound useful as c-MET and/or RON inhibitors.

### TECHNICAL SOLUTION

In order to accomplish the above-described objects,

in an aspect, the present invention provides a compound of Formula 1 below or a pharmaceutically acceptable salt thereof.

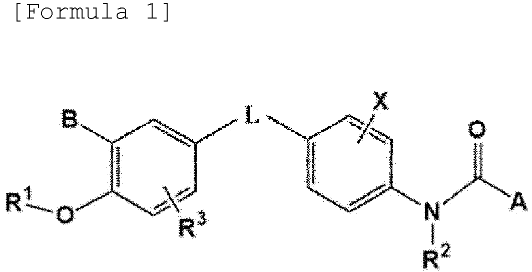

In Formula 1,
L is a direct linkage, O or NR⁴,
R¹ is hydrogen, C₂-C₆ alkyl, substituted C₁-C₆ alkyl, C₅-C₇ aryl, or substituted C₅-C₇ aryl, where a substituent of the substituted C₂-C₆ alkyl and the substituted C₅-C₇ aryl is selected from the group consisting of C₂-C₆ alkyl, C₆-C₁₀ aryl, halogen, C₁-C₆ haloalkyl, C₁-C₆ alkoxy, C₁-C₆ alkylthio, hydroxy, -COOH, C₁-C₆ alkylcarbonyl, C₂-C₆ alkyloxycarbonyl, C₂-C₆ alkylcarbonyloxy, -NH₂, carbamoyl, urea and -SH,
R² is hydrogen, C₂-C₆ alkyl or substituted C₂-C₆ alkyl, where a substituent of the substituted C₂-C₆ alkyl is selected from the group consisting of C₁-C₆ alkyl, C₆-C₁₀ aryl, halogen, C₁-C₆ haloalkyl, C₁-C₆ alkoxy, C₁-C₆ alkylthio, hydroxy, -COOH, C₁-C₆ alkylcarbonyl, C₂-C₆ alkyloxycarbonyl, C₂-C₆ alkylcarbonyloxy, -NH₂, carbamoyl, urea and -SH,
R³ is hydrogen, halogen, C₂-C₆ alkyl or substituted C₁-C₆ alkyl, where a substituent of the substituted C₂-C₆ alkyl is selected from the group consisting of C₂-C₆ alkyl, C₆-C₁₀ aryl, halogen, C₁-C₆ haloalkyl, C₁-C₆ alkoxy, C₁-C₆ alkylthio, hydroxy, -COOH, C₂-C₆ alkylcarbonyl, C₂-C₆ alkyloxycarbonyl, C₂-C₆ alkylcarbonyloxy, -NH₂, carbamoyl, urea and -SH,
R⁴ is hydrogen, C₂-C₆ alkyl or substituted C₂-C₆ alkyl, where a substituent of the substituted C₂-C₆ alkyl is selected from the group consisting of C₁-C₆ alkyl, C₆-C₁₀ aryl, halogen, C₁-C₆ haloalkyl, C₁-C₆ alkoxy, C₁-C₆ alkylthio, hydroxy, -COOH, C₂-C₆ alkylcarbonyl, C₂-C₆ alkyloxycarbonyl, C₂-C₆ alkylcarbonyloxy, -NH₂, carbamoyl, urea and -SH,
X is hydrogen, C₁-C₆ alkyl, substituted C₁-C₆ alkyl, or halogen, where a substituent of the substituted C₁-C₆ alkyl is selected from the group consisting of C₁-C₆ alkyl, C₆-C₁₀ aryl, halogen, C₁-C₆ haloalkyl, C₁-C₆ alkoxy, C₁-C₆ alkylthio, hydroxy, -COOH, C₁-C₆ alkylcarbonyl, C₁-C₆ alkyloxycarbonyl, C₁-C₆ alkylcarbonyloxy, -NH₂, carbamoyl, urea and -SH,
A is 5-member to 7-member, substituted or unsubstituted heteroaryl containing 1 to 3 heterocycle-forming atoms selected from the group consisting of nitrogen, sulfur and oxygen, or 3-member to 7-member, substituted or unsubstituted cycloalkyl, where a substituent of the substituted heteroaryl and the substituted cyclolakyl is hydrogen, halogen, C₁-C₆ alkyl, C₁-C₆ alkyl, substituted C₁-C₆ alkyl, C₃-C₆ cycloalkyl, C₁-C₅ alkoxy, 5-member to 12-member aryl, C₁-C₆ alkyl, substituted 5-member to 12-member aryl, 5-member to 7-member heteroaryl containing 1 to 3 heterocycle-forming atoms selected from the group consisting of nitrogen, sulfur and oxygen, or C₁-C₆ alkyl, substituted 5-member to 7-member heteroaryl, or oxo,
B is C₁-C₆ alkylcarbonyl, C₁-C₆ alkylimine, N-hydroxysubstituted C₁-C₆ alkylimine, halogen, 5-member to 7-member monoaryl, substituted 5-member to 7-member monoaryl, 5-member to 7-member heteromonoaryl containing 1 to 3 heterocycle-forming atoms selected from the group consisting of nitrogen, sulfur and oxygen, substituted 5-member to 7-member heteromonoaryl, 9-member to 10-member fused biaryl, substituted 9-member to 10-member fused biaryl, 9-member to 10-member fused heterobiaryl containing 1 to 3 heterocycle-forming atoms selected from the group consisting of nitrogen, sulfur and oxygen, or substituted 9-member to 10-member fused heterobiaryl, where substituents of the substituted monoaryl, heteromonoaryl, biaryl and heterobiaryl are 1 to 3 substituents independently selected from the group consisting of C₁-C₃ alkyl, halogen, hydroxy, -NH₂ and -SH, and
the halogen is independently selected from the group consisting of F, Cl, Br and I.

In another aspect, the present invention provides a pharmaceutical composition comprising: the compound of Formula 1 or the pharmaceutically acceptable salt thereof; and a pharmaceutically acceptable carrier.

### ADVANTAGEOUS EFFECTS

According to the present invention, a novel compound or a pharmaceutically acceptable salt thereof, which may be usefully used for the treatment of various immune-mediated diseases as well as anticancer drugs by suppressing the activity of protein kinases, for example, protein kinases of a receptor such as c-MET and/or RON, may be provided, however, the effects of the present invention are not limited thereto.

### BEST MODE FOR CARRYING OUT THE INVENTION

According to an aspect, the present invention provides a novel compound of Formula 1 defined above or a pharmaceutically acceptable salt thereof.

In the present invention, the compound includes the compound of Formula 1, stereoisomers such as enantiomers thereof and diasteromers thereof, solvates and prodrugs, unless otherwise specified.

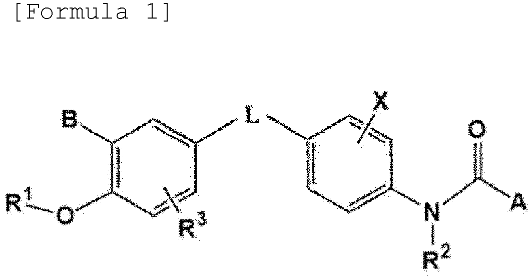

In Formula 1,
L is a direct linkage, O or NR⁴,
R¹ is hydrogen, C₁-C₆ alkyl, substituted C₁-C₆ alkyl, C₅-C₇ aryl, or substituted C₅-C₇ aryl, where a substituent of the substituted C₁-C₆ alkyl and the substituted C₅-C₇ aryl is selected from the group consisting of C₁-C₆ alkyl, C₆-C₁₀ aryl, halogen, C₁-C₆ haloalkyl, C₁-C₆ alkoxy, C₁-C₆ alkylthio, hydroxy, -COOH, C₁-C₆ alkylcarbonyl, C₁-C₆ alkyloxycarbonyl, C₁-C₆ alkylcarbonyloxy, -NH₂, carbamoyl, urea and -SH,
R² is hydrogen, C₁-C₆ alkyl or substituted C₁-C₆ alkyl, where a substituent of the substituted C₁-C₆ alkyl is selected from the group consisting of C₁-C₆ alkyl, C₆-C₁₀ aryl, halogen, C₁-C₆ haloalkyl, C₁-C₆ alkoxy, C₁-C₆ alkylthio, hydroxy, -COOH, C₁-C₆ alkylcarbonyl, C₂-C₆ alkyloxycarbonyl, C₂-C₆ alkylcarbonyloxy, -NH₂, carbamoyl, urea and -SH,
R³ is hydrogen, halogen, C₂-C₆ alkyl or substituted C₁-C₆ alkyl, where a substituent of the substituted C₂-C₆ alkyl is selected from the group consisting of C₂-C₆ alkyl, C₆-C₁₀ aryl, halogen, C₁-C₆ haloalkyl, C₁-C₆ alkoxy, C₁-C₆ alkylthio, hydroxy, -COOH, C₁-C₆ alkylcarbonyl, C₂-C₆ alkyloxycarbonyl, C₂-C₆ alkylcarbonyloxy, -NH₂, carbamoyl, urea and -SH,
R⁴ is hydrogen, C₂-C₆ alkyl or substituted C₂-C₆ alkyl, where a substituent of the substituted C₂-C₆ alkyl is selected from the group consisting of C₁-C₆ alkyl, C₆-C₁₀ aryl, halogen, C₁-C₆ haloalkyl, C₁-C₆ alkoxy, C₁-C₆ alkylthio, hydroxy, -COOH, C₂-C₆ alkylcarbonyl, C₂-C₆ alkyloxycarbonyl, C₂-C₆ alkylcarbonyloxy, -NH₂, carbamoyl, urea and -SH,
X is hydrogen, C₂-C₆ alkyl, substituted C₁-C₆ alkyl, or halogen, where a substituent of the substituted C₂-C₆ alkyl is selected from the group consisting of C₂-C₆ alkyl, C₆-C₁₀ aryl, halogen, C₁-C₆ haloalkyl, C₁-C₆ alkoxy, C₁-C₆ alkylthio, hydroxy, -COOH, C₂-C₆ alkylcarbonyl, C₂-C₆ alkyloxycarbonyl, C₂-C₆ alkylcarbonyloxy, -NH₂, carbamoyl, urea and -SH,
A is 5-member to 7-member, substituted or unsubstituted heteroaryl containing 1 to 3 heterocycle-forming atoms selected from the group consisting of nitrogen, sulfur and oxygen, or 3-member to 7-member, substituted or unsubstituted cycloalkyl, where a substituent of the substituted heteroaryl and the substituted cyclolakyl is hydrogen, halogen, C₁-C₆ alkyl, C₁-C₆ alkyl, substituted C₁-C₆ alkyl, C₃-C₆ cycloalkyl, C₁-C₅ alkoxy, 5-member to 12-member aryl, C₁-C₆ alkyl, substituted 5-member to 12-member aryl, 5-member to 7-member heteroaryl containing 1 to 3 heterocycle-forming atoms selected from the group consisting of nitrogen, sulfur and oxygen, or C₁-C₆ alkyl, substituted 5-member to 7-member heteroaryl, or oxo,
B is C₁-C₆ alkylcarbonyl, C₁-C₆ alkylimine, N-hydroxy-substituted C₁-C₆ alkylimine, halogen, 5-member to 7-member monoaryl, substituted 5-member to 7-member monoaryl, 5-member to 7-member heteromonoaryl containing 1 to 3 heterocycle-forming atoms selected from the group consisting of nitrogen, sulfur and oxygen, substituted 5-member to 7-member heteromonoaryl, 9-member to 10-member fused biaryl, substituted 9-member to 10-member fused biaryl, 9-member to 10-member fused heterobiaryl containing 1 to 3 heterocycle-forming atoms selected from the group consisting of nitrogen, sulfur and oxygen, or substituted 9-member to 10-member fused heterobiaryl, where substituents of the substituted monoaryl, heteromonoaryl, biaryl and heterobiaryl are 1 to 3 substituents independently selected from the group consisting of C₁-C₃ alkyl, halogen, hydroxy, -NH₂ and -SH, and
the halogen is independently selected from the group consisting of F, Cl, Br and I.

In the present disclosure, the term "substitution" means that a hydrogen atom bonded to a carbon atom in a structure is replaced with another substituent, and a substitution position is a position where the hydrogen atom is substituted. That is, the substitution position is not limited as long as substitution may arise, and if the substitution arises at two or more positions, two or more substituents may be the same or different.

In the present disclosure, unless otherwise defined, the "substituent" may be one or more selected from the group consisting of deuterium, halogen, hydroxy, C₁-C₁₀ alkyl, C₃-C₁₂ cycloalkyl, C₁-C₁₀ alkoxy, C₅-C₁₂ aryloxy, C₁-C₁₀ alkylthioxy, C₅-C₁₂ arylthioxy, C₁-C₁₀ alkylsulfoxy, C₅-C₁₂ arylsulfoxy, C₁-C₁₀ haloalkyl, C₂-C₂₀ alkenyl, C₀-C₁₀ amine, nitrile, nitro, imide, amide, oxo, carbonyl, carboxylic acid, carbamoyl, ester, C₅-C₁₂ aryl, and C₅-C₁₂ heteroaryl.

In the present disclosure, the "alkyl" may be a linear chain or a branched chain and may preferably have 1 to 20 carbon atoms, unless otherwise defined. For example, methyl, ethyl, propyl, n-propyl, isopropyl, butyl, n-butyl, isobutyl, tert-butyl, sec-butyl, 1-methylbutyl, ethylbutyl, pentyl, n-pentyl, isopentyl, neopentyl, tert-pentyl, hexyl, n-hexyl, methylpentyl, 2-methylpentyl, 4-methyl-2-pentyl, 3,3-dimethylbutyl, 2-ethylbutyl, heptyl, n-heptyl, 1-methylhexyl, cyclopentylmethyl, cyclohexylmethyl, octyl, n-octyl, tertoctyl, 1-methylheptyl, 2-ethylhexyl, 2-propylpentyl, n-nonyl, 2,2-dimethylheptyl, 1-ethylpropyl, dimethylpropyl, isohexyl, 2-methylpentyl, 4-methylhexyl, 5-methylhexyl, or the like may be included, without limitation.

In the present disclosure, the "cycloalkyl" may mean cyclic saturated hydrocarbon and, unless otherwise defined, may preferably have 3 to 20 carbon number. For example, cyclopropyl, cyclobutyl, cyclopentyl, 3-methylcyclopentyl, 2,3-dimethylcyclopentyl, cyclohexyl, 3-methylcyclohexyl, 4-methylcyclohexyl, 2,3-dimethylcyclohexyl, 3,4,5-trimethylcyclohexyl, 4-tert-butylcyclohexyl, cycloheptyl, cyclooctyl, or the like may be included, without limitation.

In the present disclosure, the "alkoxy" be a linear chain, a branched chain or a cyclic type and, unless otherwise defined, may preferably have 1 to 20 carbon number. For example, methoxy, ethoxy, n-propoxy, isopropoxy, n-butoxy, isobutoxy, tert-butoxy, sec-butoxy, n-pentyloxy, neopentyloxy, isopentyloxy, n-hexyloxy, 3,3-dimethylbutyloxy, 2-ethylbutyloxy, n-octyloxy, n-nonyloxy, n-decyloxy, benzyloxy, p-methylbenzyloxy, or the like, may be included, without limitation.

In the present disclosure, the "alkenyl" may be a linear chain or a branched chain and, unless otherwise defined, may preferably have 2 to 20 carbon number. For example, vinyl, 1-propenyl, isopropenyl, 1-butenyl, 2-butenyl, 3-butenyl, 1-pentenyl, 2-pentenyl, 3-pentenyl, 3-methyl-1-butenyl, 1,3-butadienyl, allyl, 1-phenylvinyl-1-yl, 2-phenylvinyl-1-yl, 2,2-diphenylvinyl-1-yl, 2-phenyl-2-(naphthyl-1-yl)vinyl-1-yl, 2,2-bis(diphenyl-1-yl)vinyl-1-yl, stilbenyl, styrenyl, or the like, may be included, without limitation.

In the present disclosure, the "aryl" may be monoaryl, biaryl, or polycyclic aryl of three or more rings, if two or more cyclic structures are included, each ring may be fused or included as a spiro type, and unless otherwise defined, the aryl may preferably have 5 to 12 carbon number. For example, phenyl, biphenyl, terphenyl, naphthyl, anthracenyl, phenanthryl, pyrenyl, perylenyl, or the like, may be included, without limitation.

In the present disclosure, the "heteroaryl" includes one or more heteroatoms other than carbon, and particularly, the heteroatom may include one or more atoms selected from the group consisting of O, N, Se and S. The heteroaryl may be monocyclic or polycyclic, and if two or more cyclic structures are included, each ring may be fused or included as a spiro type, and unless otherwise defined, the heteroaryl may preferably have 5 to 12 carbon number. For example, thiophene, furanyl, pyrrole, imidazolyl, thiazolyl, oxazolyl, oxadiazolyl, pyridyl, bipyridyl, pyrimidyl, triazinyl, triazolyl, acridyl, pyridazinyl, pyrazinyl, quinolinyl, quinazolinyl, quinoxalinyl, phthalazinyl, pyrido pyrimidyl, pyrido pyrazinyl, pyrazino pyrazinyl, isoquinolinyl, indolyl, carbazolyl, benzoxazolyl, benzimidazolyl, benzothiazolyl, or the like, may be included, without limitation.

According to an embodiment, in Formula 1, A may have a structure of Formula (IIa), Formula (IIb) or Formula (IIc) below.

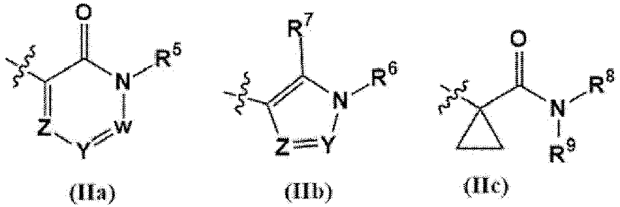

In Formula (IIa), Formula (IIb) and Formula (IIc),
Y and Z are each independently CR¹⁰, COR¹⁰ or NR¹⁰,
W is a direct linkage, CR¹⁰ or COR¹⁰,
R⁵, R⁶ and R⁸ are each independently 5-member to 7-member aryl, substituted 5-member to 7-member aryl, 5-member to 7-member heteroaryl containing 1 to 3 heterocycle-forming atoms selected from the group consisting of nitrogen, sulfur and oxygen, or substituted 5-member to 7-member heteroaryl, where substituents in the substituted 5-member to 7-member aryl or the 5-member to 7-member heteroaryl are each independently C₁-C₆ alkyl, halogen or hydroxy,
R⁷ and R⁹ are each independently hydrogen, C₁-C₆ alkyl or halogen-substituted C₁-C₆ alkyl, and
R¹⁰ is hydrogen, halogen, C₁-C₆ alkyl, substituted C₁-C₆ alkyl, C₃-C₆ cycloalkyl, substituted C₃-C₆ cycloalkyl, 5-member to 7-member aryl, substituted 5-member to 7-member aryl, 5-member to 7-member heteroaryl containing 1 to 3 heterocycle-forming atoms selected from the group consisting of nitrogen, sulfur and oxygen, or substituted 5-member to 7-member heteroaryl, where substituents in the substituted C₁-C₆ alkyl, C₃-C₆ cycloalkyl, 5-member to 7-member aryl, or 5-member to 7-member heteroaryl are each independently C₁-C₆ alkyl, hydroxy or halogen.

According to another embodiment, in Formula (IIa), Formula (IIb) and Formula (IIc),
Y and Z are each independently CR¹⁰, COR¹⁰ or NR¹⁰,
W is a direct linkage or CR¹⁰,
R⁵, R⁶ and R⁸ are each independently phenyl, fluorine-substituted phenyl, or 6-member heteroaryl containing 1 to 3 heterocycle-forming atoms selected from the group consisting of nitrogen and oxygen,
R⁷ and R⁹ are each independently hydrogen or trifluoromethyl, and
R¹⁰ is hydrogen, halogen, C₁-C₄ alkyl, substituted C₁-C₄ alkyl, C₃ cycloalkyl, or 5-member to 6-member heteroaryl, substituted with C₁-C₃ alkyl and containing 1 to 3 heterocycle-forming atoms selected from the group consisting of nitrogen and oxygen.

According to an embodiment, in Formula 1, B may have the structures below.

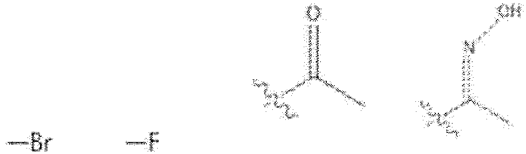

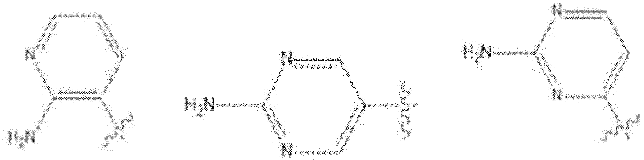

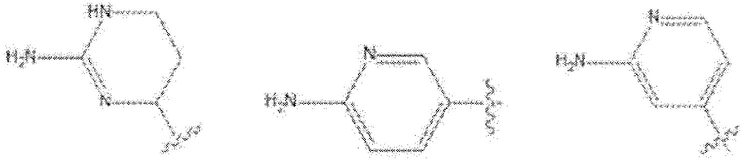

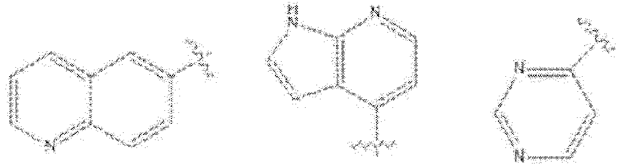

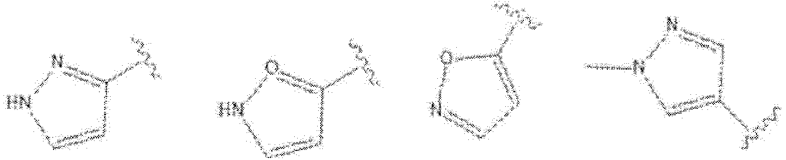

According to an embodiment, in Formula 1, L is a direct linkage, O or NH.

According to another embodiment, in Formula 1, R¹ is hydrogen, methyl, ethyl or benzyl.

According to another embodiment, in Formula 1, R² is hydrogen or methyl.

According to another embodiment, in Formula 1, R³ is hydrogen.

According to another embodiment, in Formula 1, X is hydrogen, methyl or fluorine (F).

According to an embodiment, the compound of Formula 1 may be selected from the group consisting of N-(3'-(2-aminopyrimidin-4-yl)-2-fluoro-4'-hydroxy-[1,1'-biphenyl]-4-yl)-4-ethoxy-1-(4-fluorophenyl)-2-oxo-1,2-dihydropyridin-3-carboxamide, N-(3'-(2-aminopyrimidin-4-yl)-2-fluoro-4'-hydroxy-[1,1'-biphenyl]-4-yl)-1-(4-fluorophenyl)-4-methoxy-2-oxo-1,2-dihydropyridin-3-carboxamide, N-(3'-(2-aminopyrimidin-4-yl)-2-fluoro-4'-hydroxy-[1,1'-biphenyl]-4-yl)-1-(4-fluorophenyl)-6-methyl-2-oxo-1,2-dihydropyridin-3-carboxamide, N-(3'-(2-aminopyrimidin-4-yl)-2-fluoro-4'-hydroxy-[1,1'-biphenyl]-4-yl)-1,5-dimethyl-3-oxo-2-phenyl-2,3-dihydro-1H-pyrazol-4-carboxamide, N-(3'-(2-aminopyrimidin-4-yl)-2-fluoro-4'-hydroxy-[1,1'-biphenyl]-4-yl)-1-phenyl-5-(trifluoromethyl)-1H-imidazol-4-carboxamide, N-(3'-(2-aminopyrimidin-4-yl)-2-fluoro-4'-hydroxy-[1,1'-biphenyl]-4-yl)-5-bromo-1-(4-fluorophenyl)-2-oxo-1,2-dihydropyridin-3-carboxamide, N-(3'-(2-aminopyrimidin-4-yl)-2-fluoro-4'-hydroxy-[1,1'-biphenyl]-4-yl)-1-(4-fluorophenyl)-5-(1-methyl-1H-pyrazol-4-yl)-2-oxo-1,2-dihydropyridin-3-carboxamide, N-3'-(2-aminopyrimidin-4-yl)-2-fluoro-4'-hydroxy-[1,1'-biphenyl]-4-yl)-5-cyclopropyl-1-(4-fluorophenyl)-2-oxo-1,2-dihydropyridin-3-carboxamide, N-(3'-(2-aminopyrimidin-4-yl)-2-fluoro-4'-hydroxy-[1,1'-biphenyl]-4-yl)-1-(4-fluorophenyl)-2-oxo-6-(trifluoromethyl)-1,2-dihydropyridin-3-carboxamide, N-(3'-(2-aminopyrimidin-4-yl)-2-fluoro-4'-hydroxy-[1,1'-biphenyl]-4-yl)-1-(4-fluorophenyl)-6-(hydroxymethyl)-2-oxo-1,2-dihydropyridin-3-carboxamide, N-(3'-(2-aminopyrimidin-4-yl)-4'-(benzyloxy)-2-methyl-[1,1'-biphenyl]-4-yl)-4-ethoxy-1-(4-fluorophenyl)-2-oxo-1,2-dihydropyridin-3-carboxamide, N-(3'-(2-aminopyrimidin-4-yl)-4'-(benzyloxy)-2-methyl-[1,1'-biphenyl]-4-yl)-N-(4-fluorophenyl)cyclopropan-1,1-dicarboxamide, N-(3'-(2-aminopyrimidin-4-yl)-4'-hydroxy-2-methyl-[1,1'-biphenyl]-4-yl)-4-ethoxy-1-(4-fluorophenyl)-2-oxo-1,2-dihydropyridin-3-carboxamide, N-(3'-(2-aminopyrimidin-4-yl)-4'-hydroxy-2-methyl-[1,1'-biphenyl]-4-yl)-1-(4-fluorophenyl)-6-methyl-2-oxo-1,2-dihydropyridin-3-carboxamide, N-(3'-(2-aminopyrimidin-4-yl)-4'-hydroxy-2-methyl-[1,1'-biphenyl]-4-yl)-N-(4-fluorophenyl)cyclopropan-1,1-dicarboxamide, N-(3'-(2-amino-1,4,5,6-tetrahydropyrimidin-4-yl)-4'-hydroxy-2-methyl-[1,1'-biphenyl]-4-yl)-N-(4-fluorophenyl)cyclopropan-1,1-dicarboxamide, N-(3'-(2-aminopyrimidin-5-yl)-2-fluoro-4'-methoxy-[1,1'-biphenyl]-4-yl)-4-ethoxy-1-(4-fluorophenyl)-2-oxo-1,2-dihydropyridin-3-carboxamide, N-(3'-(6-aminopyridin-3-yl)-2-fluoro-4'-methoxy-[1,1'-biphenyl]-4-yl)-4-ethoxy-1-(4-fluorophenyl)-2-oxo-1,2-dihydropyridin-3-carboxamide, N-(3'-(2-aminopyridin-4-yl)-2-fluoro-4'-methoxy-[1,1'-biphenyl]-4-yl)-4-ethoxy-1-(4-fluorophenyl)-2-oxo-1,2-dihydropyridin-3-carboxamide, N-(3'-(2-aminopyridin-3-yl)-2-fluoro-4'-methoxy-[1,1'-biphenyl]-4-yl)-4-ethoxy-1-(4-fluorophenyl)-2-oxo-1,2-dihydropyridin-3-carboxamide, 4-ethoxy-N-(2-fluoro-4'-methoxy-3'-(quinolin-6-yl)-[1,1'-biphenyl]-4-yl)-1-(4-fluorophenyl)-2-oxo-1,2-dihydropyridin-3-carboxamide, 4-ethoxy-N-(2-fluoro-3'-(isoquinolin-6-yl)-4'-methoxy-[1,1'-biphenyl]-4-yl)-1-(4-fluorophenyl)-2-oxo-1,2-dihydropyridin-3-carboxamide, 4-ethoxy-N-(2-fluoro-4'-methoxy-3'-(1H-pyrrolo[2,3-b]pyridin-4-yl)-[1,1'-biphenyl]-4-yl)-1-(4-fluorophenyl)-2-oxo-1,2-dihydropyridin-3-carboxamide, N-(3'-bromo-2-fluoro-4'-hydroxy-[1,1'-biphenyl]-4-yl)-4-ethoxy-1-(4-fluorophenyl)-2-oxo-1,2-dihydropyridin-3-carboxamide, N-(3'-(2-aminopyrimidin-5-yl)-2-fluoro-4'-hydroxy-[1,1'-biphenyl]-4-yl)-4-ethoxy-1-(4-fluorophenyl)-2-oxo-1,2-dihydropyridin-3-carboxamide, N-(3'-(2-aminopyridin-4-yl)-2-fluoro-4'-hydroxy-[1,1'-biphenyl]-4-yl)-4-ethoxy-1-(4-fluorophenyl)-2-oxo-1,2-dihydropyridin-3-carboxamide, 4-ethoxy-N-(2-fluoro-4'-hydroxy-3'-(1-methyl-1H-pyrazol-4-yl)-[1,1'-biphenyl]-4-yl)-1-(4-fluorophenyl)-2-oxo-1,2-dihydropyridin-3-carboxamide, N-(4-((3-(2-aminopyrimidin-4-yl)-4-hydroxyphenyl)amino)-3-fluorophenyl)-4-ethoxy-1-(4-fluorophenyl)-2-oxo-1,2-dihydropyridin-3-carboxamide, N-(4-((3-(2-aminopyrimidin-4-yl)-4-hydroxyphenyl)amino)-3-fluorophenyl)-1-(4-fluorophenyl)-4-methoxy-2-oxo-1,2-dihydropyridin-3-carboxamide, N-(4-((3-(2-aminopyrimidin-4-yl)-4-hydroxyphenyl)amino)-3-fluorophenyl)-1-(4-fluorophenyl)-6-methyl-2-oxo-1,2-dihydropyridin-3-carboxamide, N-(4-((3-(2-aminopyrimidin-4-yl)-4-hydroxyphenyl)amino)-3-fluorophenyl)-N-(4-fluorophenyl)cyclopropan-1,1-dicarboxamide, N-(4-((3-(2-aminopyrimidin-4-yl)-4-hydroxyphenyl)amino)-3-fluorophenyl)-1,5-dimethyl-3-oxo-2-phenyl-2,3-dihydro-1H-pyrazol-4-carboxamide, N-(4-((3-(2-aminopyrimidin-4-yl)-4-hydroxyphenyl)amino)-3-fluorophenyl)-1-phenyl-5-(trifluoromethyl)-1H-imidazol-4-carboxamide, N-(4-((3-(2-aminopyrimidin-4-yl)-4-hydroxyphenyl)amino)-2-fluorophenyl)-4-ethoxy-1-(4-fluorophenyl)-2-oxo-1,2-dihydropyridin-3-carboxamide, N-(4-((3-(2-aminopyrimidin-4-yl)-4-hydroxyphenyl)amino)phenyl)-4-ethoxy-1-(4-fluorophenyl)-2-oxo-1,2-dihydropyridin-3-carboxamide, N-(4-(3-acetyl-4-hydroxyphenoxy)-3-fluorophenyl)-4-ethoxy-1-(4-fluorophenyl)-2-oxo-1,2-dihydropyridin-3-carboxamide, N-(4-(3-acetyl-4-hydroxyphenoxy)-3-fluorophenyl)-1-(4-fluorophenyl)-6-methyl-2-oxo-1,2-dihydropyridin-3-carboxamide, (E)-N-(3-fluoro-4-(4-hydroxy-3-(1-(hydroxyimino)ethyl)phenoxy)phenyl)-1-(4-fluorophenyl)-6-methyl-2-oxo-1,2-dihydropyridin-3-carboxamide, N-(4-(3-(2-aminopyrimidin-4-yl)-4-hydroxyphenoxy)-3-fluorophenyl)-4-ethoxy-1-(4-fluorophenyl)-2-oxo-1,2-dihydropyridin-3-carboxamide, N-(4-(3-(2-aminopyrimidin-4-yl)-4-hydroxyphenoxy)-3-fluorophenyl)-1-(4-fluorophenyl)-6-methyl-2-oxo-1,2-dihydropyridin-3-carboxamide, N-(4-(3-(2-aminopyrimidin-4-yl)-4-hydroxyphenoxy)-3-fluorophenyl)-N-(4-fluorophenyl)cyclopropan-1,1-dicarboxamide, N-(4-(3-(2-aminopyrimidin-4-yl)-4-hydroxyphenoxy)-3-fluorophenyl)-1-(4-fluorophenyl)-4-methoxy-2-oxo-1,2-dihydropyridin-3-carboxamide, N-(4-(3-(2-aminopyrimidin-4-yl)-4-hydroxyphenoxy)-3-fluorophenyl)-1,5-dimethyl-3-oxo-2-phenyl-2,3-dihydro-1H-pyrazol-4-carboxamide, N-(4-(3-(2-aminopyrimidin-4-yl)-4-hydroxyphenoxy)-3-fluorophenyl)-5-bromo-1-(4-fluorophenyl)-2-oxo-1,2-dihydropyridin-3-carboxamide, N-(4-(3-(2-aminopyrimidin-4-yl)-4-hydroxyphenoxy)-3-fluorophenyl)-1-phenyl-5-(trifluoromethyl)-1H-imidazol-4-carboxamide, N-(4-(3-(2-aminopyrimidin-4-yl)-4-hydroxyphenoxy)-3-fluorophenyl)-1-(4-fluorophenyl)-2-oxo-1,2-dihydropyridin-3-carboxamide, N-(4-(3-(2-aminopyrimidin-4-yl)-4-hydroxyphenoxy)-3-fluorophenyl)-1-(4-fluorophenyl)-5-(1-methyl-1H-pyrazol-4-yl)-2-oxo-1,2-dihydropyridin-3-carboxamide, N-(4-(3-(2-aminopyrimidin-4-yl)-4-hydroxyphenoxy)-3-fluorophenyl)-5-cyclopropyl-1-(4-fluorophenyl)-2-oxo-1,2-dihydropyridin-3-carboxamide, N-(4-(3-(2-aminopyrimidin-4-yl)-4-hydroxyphenoxy)-3-fluorophenyl)-1-(4-fluorophenyl)-2-oxo-6-(trifluoromethyl)-1,2-dihydropyridin-3-carboxamide, N-(4-(3-(2-aminopyrimidin-4-yl)-4-hydroxyphenoxy)-3-fluorophenyl)-1-(4-fluorophenyl)-6-(hydroxymethyl)-2-oxo-1,2-dihydropyridin-3-carboxamide, N-(4-(3-(2-aminopyrimidin-4-yl)-4-hydroxyphenoxy)-3-fluorophenyl)-1-phenyl-5-(tri fluoromethyl)-1H-pyrazol-4-carboxamide, N-(4-(3-(2-aminopyrimidin-4-yl)-4-hydroxyphenoxy)-3-fluorophenyl)-1-(2-hydroxy-2-methylpropyl)-5-methyl-3-oxo-2-phenyl-2,3-dihydro-1H-pyrazol-4-carboxamide, N-(4-(3-(2-aminopyrimidin-4-yl)-4-hydroxyphenoxy)-3-fluorophenyl)-5-ethyl-1-(pyrimidin-5-yl)-1H-pyrazol-4-carboxamide, 4-ethoxy-N-(3-fluoro-4-(4-hydroxy-3-(pyrimidin-4-yl)phenoxy)phenyl)-1-(4-fluorophenyl)-2-oxo-1,2-dihydropyridin-3-carboxamide, N-(3-fluoro-4-(4-hydroxy-3-(pyrimidin-4-yl)phenoxy)phenyl)-1-(4-fluorophenyl)-6-methyl-2-oxo-1,2-dihydropyridin-3-carboxamide, N-(3-fluoro-4-(4-hydroxy-3-(pyrimidin-4-yl)phenoxy)phenyl)-N-(4-fluorophenyl)cyclopropan-1,1-dicarboxamide, 4-ethoxy-N-(3-fluoro-4-(4-hydroxy-3-(1H-pyrazol-3-yl)phenoxy)phenyl)-1-(4-fluoro phenyl)-2-oxo-1,2-dihydropyridin-3-carboxamide, N-(3-fluoro-4-(4-hydroxy-3-(1H-pyrazol-3-yl)phenoxy)phenyl)-1-(4-fluorophenyl)-6-methyl-2-oxo-1,2-dihydropyridin-3-carboxamide, N-(3-fluoro-4-(4-hydroxy-3-(isoxazol-5-yl)phenoxy)phenyl)-1-(4-fluorophenyl)-6-methyl-2-oxo-1,2-dihydropyridin-3-carboxamide, N-(4-(3-(2-aminopyrimidin-5-yl)-4-hydroxyphenoxy)-3-fluorophenyl)-4-ethoxy-1-(4-fluorophenyl)-2-oxo-1,2-dihydropyridin-3-carboxamide and N-(4-(3-(2-aminopyridin-4-yl)-4-hydroxyphenoxy)-3-fluorophenyl)-4-ethoxy-1-(4-fluorophenyl)-2-oxo-1,2-dihydropyridin-3-carboxamide, and the structures are shown in Table 1 below.

**[Table 1]**

| | **Structure** | **IUPAC name** |
|---|---|---|
| **Example 1** | 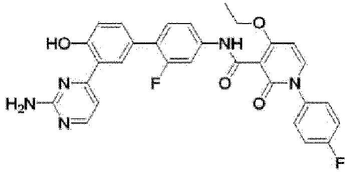 | N-(3'-(2-aminopyrimidin-4-yl)-2-fluoro-4'-hydroxy-[1,1'-biphenyl]-4-yl)-4-ethoxy-1-(4-fluorophenyl)-2-oxo-1,2-dihydropyridine-3-carboxamide |
| **Example 2** | 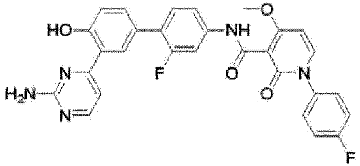 | N-(3'-(2-aminopyrimidin-4-yl)-2-fluoro-4'-hydroxy-[1,1'-biphenyl]-4-yl)-1-(4-fluorophenyl)-4-methoxy-2-oxo-1,2-dihydropyridine-3-carboxamide |
| **Example 3** | 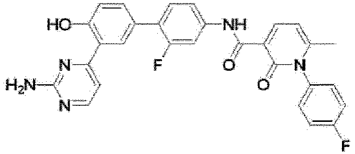 | N-(3'-(2-aminopyrimidin-4-yl)-2-fluoro-4'-hydroxy-[1,1'-biphenyl]-4-yl)-1-(4-fluorophenyl)-6-methyl-2-oxo-1,2-dihydropyridine-3-carboxamide |
| **Example 4** | 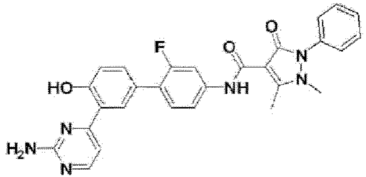 | N-(3'-(2-aminopyrimidin-4-yl)-2-fluoro-4'-hydroxy-[1,1'-biphenyl]-4-yl)-1,5-dimethyl-3-oxo-2-phenyl-2,3-dihydro-1H-pyrazole-4-carboxamide |
| **Example 5** | 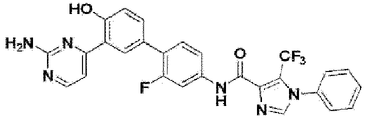 | N-(3'-(2-aminopyrimidin-4-yl)-2-fluoro-4'-hydroxy-[1,1'-biphenyl]-4-yl)-1-phenyl-5-(trifluoromethyl)-1H-imidazole-4-carboxamide |
| **Example 6** | 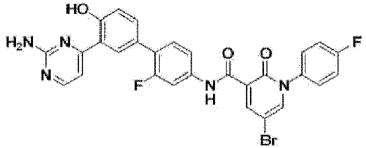 | N-(3'-(2-aminopyrimidin-4-yl)-2-fluoro-4'-hydroxy-[1,1'-biphenyl]-4-yl)-5-bromo-1-(4-fluorophenyl)-2-oxo-1,2-dihydropyridine-3-carboxamide |
| **Example 7** | 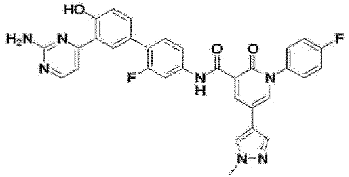 | N-(3'-(2-aminopyrimidin-4-yl)-2-fluoro-4'-hydroxy-[1,1'-biphenyl]-4-yl)-1-(4-fluorophenyl)-5-(1-methyl-1H-pyrazol-4-yl)-2-oxo-1,2-dihydropyridine-3-carboxamide |
| **Example 8** | 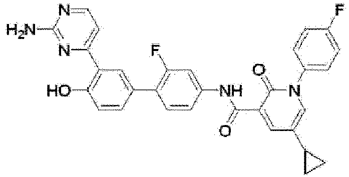 | N-(3'-(2-aminopyrimidin-4-yl)-2-fluoro-4'-hydroxy-[1,1'-biphenyl]-4-yl)-5-cyclopropyl-1-(4-fluorophenyl)-2-oxo-1,2-dihydropyridine-3-carboxamide |
| **Example 9** | 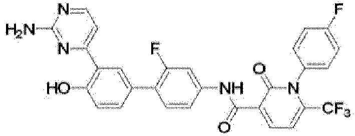 | N-(3'-(2-aminopyrimidin-4-yl)-2-fluoro-4'-hydroxy-[1,1'-biphenyl]-4-yl)-1-(4-fluorophenyl)-2-oxo-6-(trifluoromethyl)-1,2-dihydropyridine-3-carboxamide |
| **Example 10** | 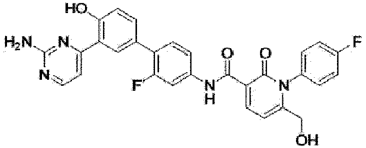 | N-(3'-(2-aminopyrimidin-4-yl)-2-fluoro-4'-hydroxy-[1,1'-biphenyl]-4-yl)-1-(4-fluorophenyl)-6-(hydroxymethyl)-2-oxo-1,2-dihydropyridine-3-carboxamide |
| **Example 11** | 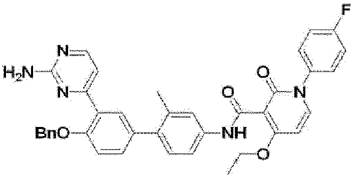 | N-(3'-(2-aminopyrimidin-4-yl)-4'-(benzyloxy)-2-methyl-[1,1'-biphenyl]-4-yl)-4-ethoxy-1-(4-fluorophenyl)-2-oxo-1,2-dihydropyridine-3-carboxamide |
| **Example 12** | 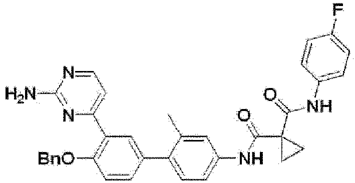 | N-(3'-(2-aminopyrimidin-4-yl)-4'-(benzyloxy)-2-methyl-[1,1'-biphenyl]-4-yl)-N-(4-fluorophenyl) cyclopropane -1,1-dicarboxamide |
| **Example 13** | 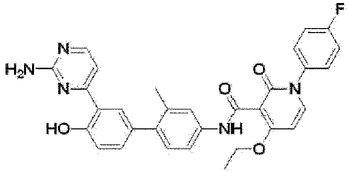 | N-(3'-(2-aminopyrimidin-4-yl)-4'-hydroxy-2-methyl-[1,1'-biphenyl]-4-yl)-4-ethoxy-1-(4-fluorophenyl)-2-oxo-1,2-dihydropyridine-3-carboxamide |
| **Example 14** | 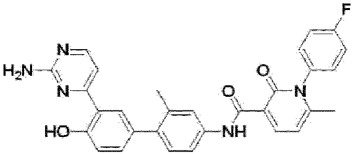 | N-(3'-(2-aminopyrimidin-4-yl)-4'-hydroxy-2-methyl-[1,1'-biphenyl]-4-yl)-1-(4-fluorophenyl)-6-methyl-2-oxo-1,2-dihydropyridine-3-carboxamide |
| **Example 15** | 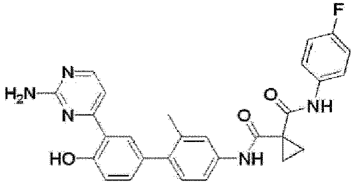 | N-(3'-(2-aminopyrimidin-4-yl)-4'-hydroxy-2-methyl-[1,1'-biphenyl]-4-yl)-N-(4-fluorophenyl) cyclopropane -1,1-dicarboxamide |
| **Example 16** | 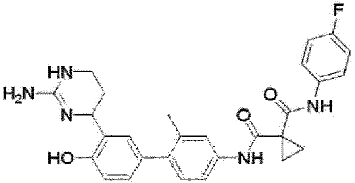 | N-(3'-(2-amino-1,4,5,6-tetrahydropyrimidin-4-yl)-4'-hydroxy-2-methyl-[1,1'-biphenyl]-4-yl)-N-(4-fluorophenyl) cyclopropane -1,1-dicarboxamide |
| **Example 17** | 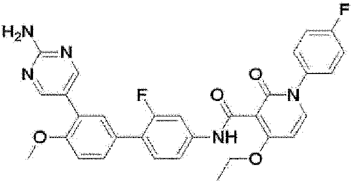 | N-(3'-(2-aminopyrimidin-5-yl)-2-fluoro-4'-methoxy-[1,1'-biphenyl]-4-yl)-4-ethoxy-1-(4-fluorophenyl)-2-oxo-1,2-dihydropyridine-3-carboxamide |
| **Example 18** | 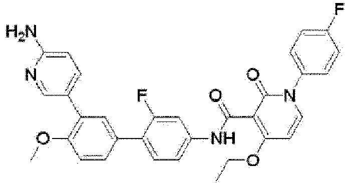 | N-(3'-(6-aminopyridin-3-yl)-2-fluoro-4'-methoxy-[1,1'-biphenyl]-4-yl)-4-ethoxy-1-(4-fluorophenyl)-2-oxo-1,2-dihydropyridine-3-carboxamide |
| **Example 19** | 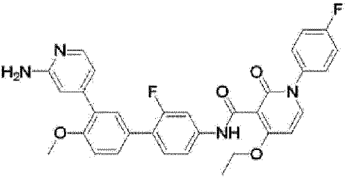 | N-(3'-(2-aminopyridin-4-yl)-2-fluoro-4'-methoxy-[1,1'-biphenyl]-4-yl)-4-ethoxy-1-(4-fluorophenyl)-2-oxo-1,2-dihydropyridine-3-carboxamide |
| **Example 20** | 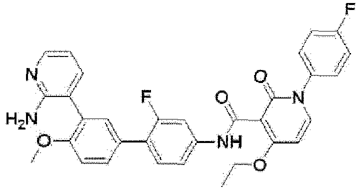 | N-(3'-(2-aminopyridin-3-yl)-2-fluoro-4'-methoxy-[1,1'-biphenyl]-4-yl)-4-ethoxy-1-(4-fluorophenyl)-2-oxo-1,2-dihydropyridine-3-carboxamide |
| **Example 21** | 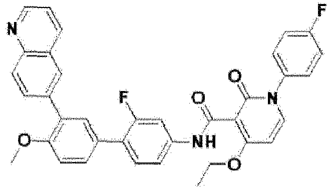 | 4-ethoxy-N-(2-fluoro-4'-methoxy-3'-(quinolin-6-yl)-[1,1'-biphenyl]-4-yl)-1-(4-fluorophenyl)-2-oxo-1,2-dihydropyridine-3-carboxamide |
| **Example 22** | 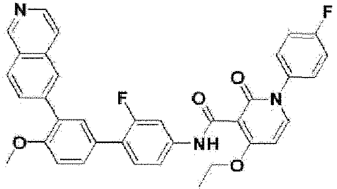 | 4-ethoxy-N-(2-fluoro-3'-(isoquinolin-6-yl)-4'-methoxy-[1,1'-biphenyl]-4-yl)-1-(4-fluorophenyl)-2-oxo-1,2-dihydropyridine-3-carboxamide |
| **Example 23** | 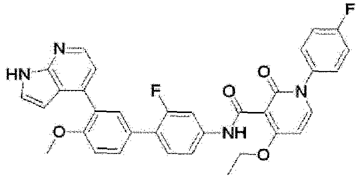 | 4-ethoxy-N-(2-fluoro-4'-methoxy-3'-(1H-pyrrolo[2,3-b]pyridin-4-yl)-[1,1'-biphenyl]-4-yl)-1-(4-fluorophenyl)-2-oxo-1,2-dihydropyridine-3-carboxamide |
| **Example 24** | 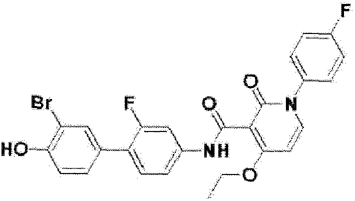 | N-(3'-bromo-2-fluoro-4'-hydroxy-[1,1'-biphenyl]-4-yl)-4-ethoxy-1-(4-fluorophenyl)-2-oxo-1,2-dihydropyridine-3-carboxamide |
| **Example 25** | 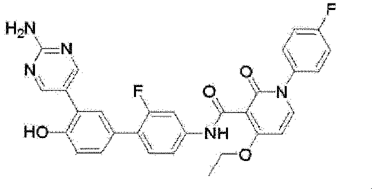 | N-(3'-(2-aminopyrimidin-5-yl)-2-fluoro-4'-hydroxy-[1,1'-biphenyl]-4-yl)-4-ethoxy-1-(4-fluorophenyl)-2-oxo-1,2-dihydropyridine-3-carboxamide |
| **Example 26** | 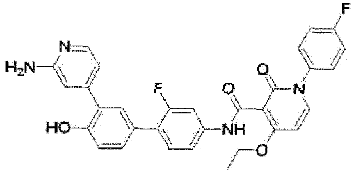 | N-(3'-(2-aminopyridin-4-yl)-2-fluoro-4'-hydroxy-[1,1'-biphenyl]-4-yl)-4-ethoxy-1-(4-fluorophenyl)-2-oxo-1,2-dihydropyridine-3-carboxamide |
| **Example 27** | 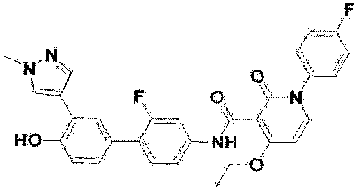 | 4-ethoxy-N-(2-fluoro-4'-hydroxy-3'-(1-methyl-1H-pyrazol-4-yl)-[1,1'-biphenyl]-4-yl)-1-(4-fluorophenyl)-2-oxo-1,2-dihydropyridine-3-carboxamide |
| **Example 28** | 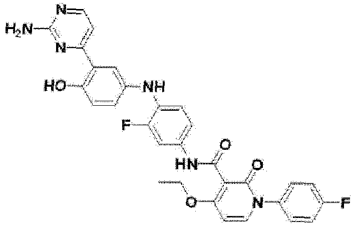 | N-(4-((3-(2-aminopyrimidin-4-yl)-4-hydroxyphenyl)amino)-3-fluorophenyl)-4-ethoxy-1-(4-fluorophenyl)-2-oxo-1,2-dihydropyridine-3-carboxamide |
| **Example 29** | 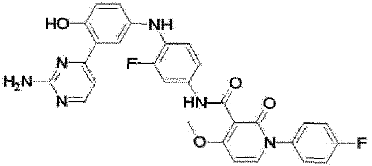 | N-(4-((3-(2-aminopyrimidin-4-yl)-4-hydroxyphenyl)amino)-3-fluorophenyl)-1-(4-fluorophenyl)-4-methoxy-2-oxo-1,2-dihydropyridine-3-carboxamide |
| **Example 30** | 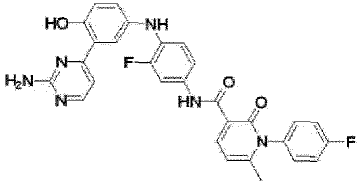 | N-(4-((3-(2-aminopyrimidin-4-yl)-4-hydroxyphenyl)amino)-3-fluorophenyl)-1-(4-fluorophenyl)-6-methyl-2-oxo-1,2-dihydropyridine-3-carboxamide |
| **Example 31** | 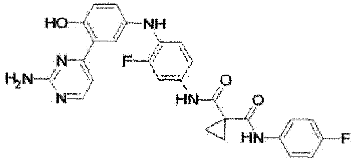 | N-(4-((3-(2-aminopyrimidin-4-yl)-4-hydroxyphenyl)amino)-3-fluorophenyl)-N-(4-fluorophenyl) cyclopropane -1,1-dicarboxamide |
| **Example 32** | 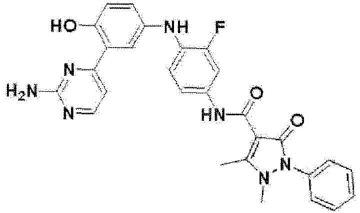 | N-(4-((3-(2-aminopyrimidin-4-yl)-4-hydroxyphenyl)amino)-3-fluorophenyl)-1,5-dimethyl-3-oxo-2-phenyl-2,3-dihydro-1H-pyrazole-4-carboxamide |
| **Example 33** | 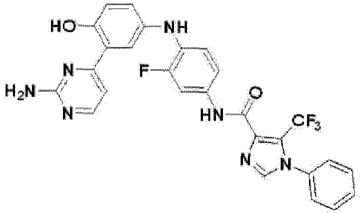 | N-(4-((3-(2-aminopyrimidin-4-yl)-4-hydroxyphenyl)amino)-3-fluorophenyl)-1-phenyl-5-(trifluoromethyl)-1H-imidazole-4-carboxamide |
| **Example 34** | 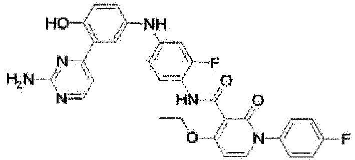 | N-(4-((3-(2-aminopyrimidin-4-yl)-4-hydroxyphenyl)amino)-2-fluorophenyl)-4-ethoxy-1-(4-fluorophenyl)-2-oxo-1,2-dihydropyridine-3-carboxamide |
| **Example 35** | 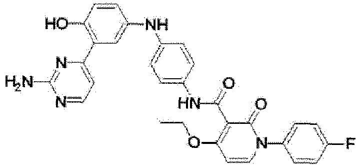 | N-(4-((3-(2-aminopyrimidin-4-yl)-4-hydroxyphenyl)amino)pheny 1)-4-ethoxy-1-(4-fluorophenyl)-2-oxo-1,2-dihydropyridine-3-carboxamide |
| **Example 36** | 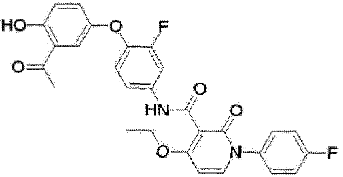 | N-(4-(3-acetyl-4-hydroxyphenoxy)-3-fluorophenyl)-4-ethoxy-1-(4-fluorophenyl)-2-oxo-1,2-dihydropyridine-3-carboxamide |
| **Example 37** | 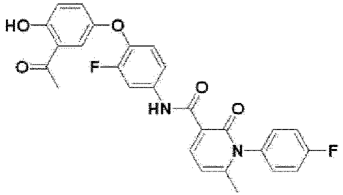 | N-(4-(3-acetyl-4-hydroxyphenoxy)-3-fluorophenyl)-1-(4-fluorophenyl)-6-methyl-2-oxo-1,2-dihydropyridine-3-carboxamide |
| **Example 38** | 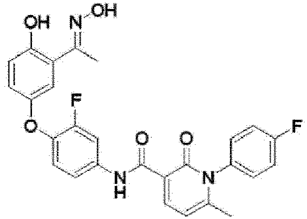 | (E)-N-(3-fluoro-4-(4-hydroxy-3-(1-(hydroxyimino)ethyl)pheno xy)phenyl)-1-(4-fluorophenyl)-6-methyl-2-oxo-1,2-dihydropyridine-3-carboxamide |
| **Example 39** | 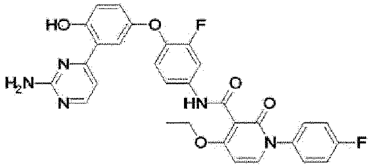 | N-(4-(3-(2-aminopyrimidin-4-yl)-4-hydroxyphenoxy)-3-fluorophenyl)-4-ethoxy-1-(4-fluorophenyl)-2-oxo-1,2-dihydropyridine-3-carboxamide |
| **Example 40** | 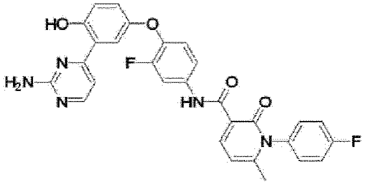 | N-(4-(3-(2-aminopyrimidin-4-yl)-4-hydroxyphenoxy)-3-fluorophenyl)-1-(4-fluorophenyl)-6-methyl-2-oxo-1,2-dihydropyridine-3-carboxamide |
| **Example 41** | 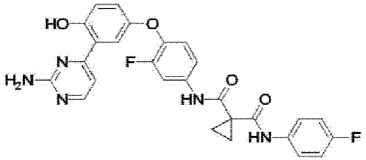 | N-(4-(3-(2-aminopyrimidin-4-yl)-4-hydroxyphenoxy)-3-fluorophenyl)-N-(4-fluorophenyl) cyclopropane -1,1-dicarboxamide |
| **Example 42** | 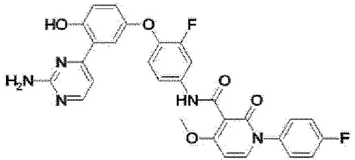 | N-(4-(3-(2-aminopyrimidin-4-yl)-4-hydroxyphenoxy)-3-fluorophenyl)-1-(4-fluorophenyl)-4-methoxy-2-oxo-1,2-dihydropyridine-3-carboxamide |
| **Example 43** | 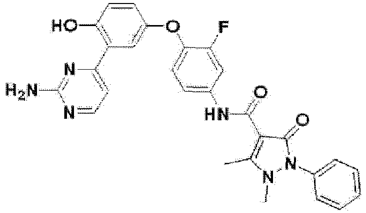 | N-(4-(3-(2-aminopyrimidin-4-yl)-4-hydroxyphenoxy)-3-fluorophenyl)-1,5-dimethyl-3-oxo-2-phenyl-2,3-dihydro-1H-pyrazole-4-carboxamide |
| **Example 44** | 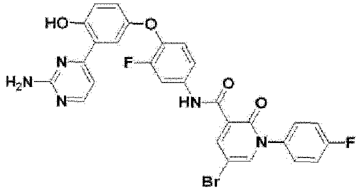 | N-(4-(3-(2-aminopyrimidin-4-yl)-4-hydroxyphenoxy)-3-fluorophenyl)-5-bromo-1-(4-fluorophenyl)-2-oxo-1,2-dihydropyridine-3-carboxamide |
| **Example 45** | 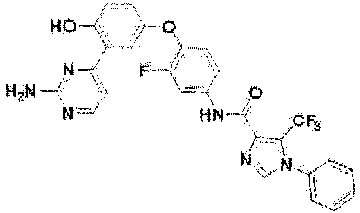 | N-(4-(3-(2-aminopyrimidin-4-yl)-4-hydroxyphenoxy)-3-fluorophenyl)-1-phenyl-5-(trifluoromethyl)-1H-imidazole-4-carboxamide |
| **Example 46** | 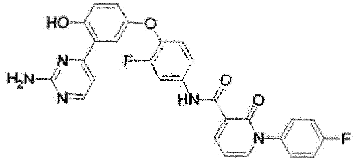 | N-(4-(3-(2-aminopyrimidin-4-yl)-4-hydroxyphenoxy)-3-fluorophenyl)-1-(4-fluorophenyl)-2-oxo-1,2-dihydropyridine-3-carboxamide |
| **Example 47** | 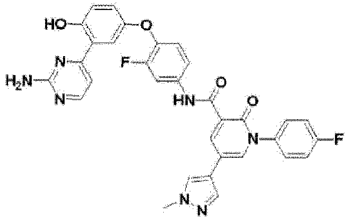 | N-(4-(3-(2-aminopyrimidin-4-yl)-4-hydroxyphenoxy)-3-fluorophenyl)-1-(4-fluorophenyl)-5-(1-methyl-1H-pyrazol-4-yl)-2-oxo-1,2-dihydropyridine-3-carboxamide |
| **Example 48** | 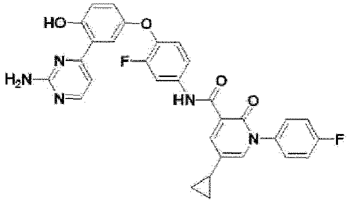 | N-(4-(3-(2-aminopyrimidin-4-yl)-4-hydroxyphenoxy)-3-fluorophenyl)-5-cyclopropyl-1-(4-fluorophenyl)-2-oxo-1,2-dihydropyridine-3-carboxamide |
| **Example 49** | 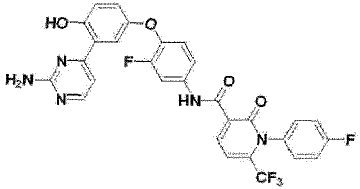 | N-(4-(3-(2-aminopyrimidin-4-yl)-4-hydroxyphenoxy)-3-fluorophenyl)-1-(4-fluorophenyl)-2-oxo-6-(trifluoromethyl)-1,2-dihydropyridine-3-carboxamide |
| **Example 50** | 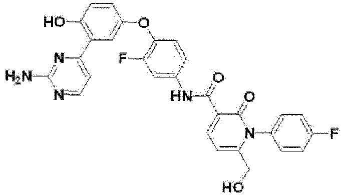 | N-(4-(3-(2-aminopyrimidin-4-yl)-4-hydroxyphenoxy)-3-fluorophenyl)-1-(4-fluorophenyl)-6-(hydroxymethyl)-2-oxo-1,2-dihydropyridine-3-carboxamide |
| **Example 51** | 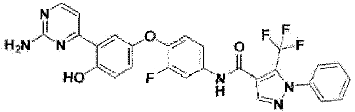 | N-(4-(3-(2-aminopyrimidin-4-yl)-4-hydroxyphenoxy)-3-fluorophenyl)-1-phenyl-5-(trifluoromethyl)-1H-pyrazole-4-carboxamide |
| **Example 52** | 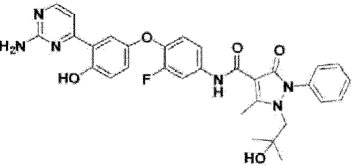 I | N-(4-(3-(2-aminopyrimidin-4-yl)-4-hydroxyphenoxy)-3-fluorophenyl)-1-(2-hydroxy-2-methylpropyl)-5-methyl-3-oxo-2-phenyl-2,3-dihydro-1H-pyrazole-4-carboxamide |
| **Example 53** | 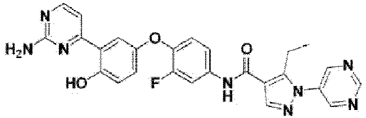 | N-(4-(3-(2-aminopyrimidin-4-yl)-4-hydroxyphenoxy)-3-fluorophenyl)-5-ethyl-1-(pyrimidin-5-yl)-1H-pyrazole-4-carboxamide |
| **Example 54** | 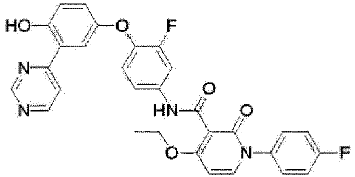 | 4-ethoxy-N-(3-fluoro-4-(4-hydroxy-3-(pyrimidin-4-yl)phenoxy)phenyl)-1-(4-fluorophenyl)-2-oxo-1,2-dihydropyridine-3-carboxamide |
| **Example 55** | 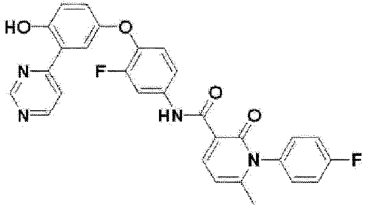 | N-(3-fluoro-4-(4-hydroxy-3-(pyrimidin-4-yl)phenoxy)phenyl)-1-(4-fluorophenyl)-6-methyl-2-oxo-1,2-dihydropyridine-3-carboxamide |
| **Example 56** | 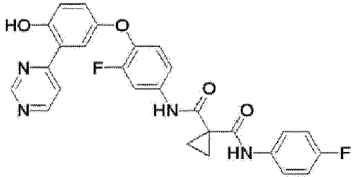 | N-(3-fluoro-4-(4-hydroxy-3-(pyrimidin-4-yl)phenoxy)phenyl)-N-(4-fluorophenyl) cyclopropane -1,1-dicarboxamide |
| **Example 57** | 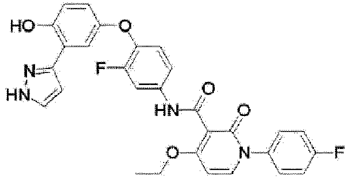 | 4-ethoxy-N-(3-fluoro-4-(4-hydroxy-3-(1H-pyrazol-3-yl)phenoxy)phenyl)-1-(4-fluorophenyl)-2-oxo-1,2-dihydropyridine-3-carboxamide |
| **Example 58** | 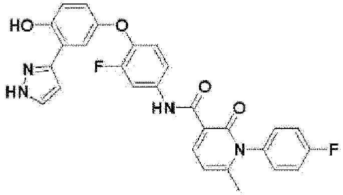 | N-(3-fluoro-4-(4-hydroxy-3-(1H-pyrazol-3-yl)phenoxy)phenyl)-1-(4-fluorophenyl)-6-methyl-2-oxo-1,2-dihydropyridine-3-carboxamide |
| **Example 59** | 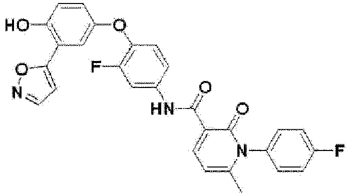 | N-(3-fluoro-4-(4-hydroxy-3-(isoxazol-5-yl)phenoxy)phenyl)-1-(4-fluorophenyl)-6-methyl-2-oxo-1,2-dihydropyridine-3-carboxamide |
| **Example 60** | 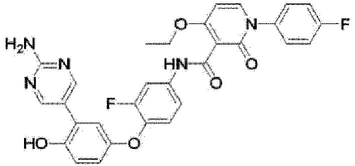 | N-(4-(3-(2-aminopyrimidin-5-yl)-4-hydroxyphenoxy)-3-fluorophenyl)-4-ethoxy-1-(4-fluorophenyl)-2-oxo-1,2-dihydropyridine-3-carboxamide |
| **Example 61** | 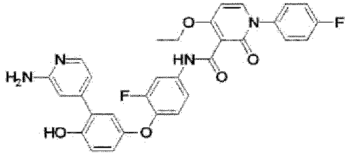 | N-(4-(3-(2-aminopyridin-4-yl)-4-hydroxyphenoxy)-3-fluorophenyl)-4-ethoxy-1-(4-fluorophenyl)-2-oxo-1,2-dihydropyridine-3-carboxamide |

In the present invention, the "pharmaceutically acceptable salt" includes salts commonly used for forming alkali metal salts and free acid- or free base-added salts. The properties of the salts are not important but are required to be pharmaceutically acceptable. Appropriate pharmaceutically acceptable acid-added salts for the compound of Formula 1 may be prepared from inorganic acids or organic acids. Examples of such an inorganic acid include hydrochloric acid, hydrobromic acid, hydroiodic acid, nitric acid, carbonic acid, sulfuric acid and phosphoric acid. Appropriate organic acid may be selected from aliphatic, alicyclic, aromatic, arylaliphatic, heterocyclic, carboxylic and sulfonic organic acids, and examples thereof include formic acid, acetic acid, adipic acid, butyric acid, propionic acid, succinic acid, glycolic acid, gluconic acid, lactic acid, malic acid, tartaric acid, citric acid, ascorbic acid, glucuronic acid, maleic acid, fumaric acid, pyruvic acid, aspartic acid, glutamic acid, benzoic acid, anthranilic acid, mesilic acid, 4-hydroxybenzoic acid, phenylacetic acid, mandelic acid, embonic acid (pamoic acid), methanesulfonic acid, ethanesulfonic acid, ethanedisulfonic acid, benzenesulfonic acid, panthothenic acid, 2-hydroxyethanesulfonic acid, toluenesulfonic acid, sulfanic acid, cyclohexylaminosulfonic acid, camphoric acid, camphorsulfonic acid, digluconic acid, cyclopentanepropionic acid, dodecylsulfonic acid, glucoheptanoic acid, glycerophosphonic acid, heptanoic acid, hexanoic acid, 2-hydroxy-ethanesulfonic acid, nicotinic acid, 2-naphthalenesulfonic acid, oxalic acid, palmitic acid, pectinic acid, persulfuric acid, 2-phenylpropionic acid, picric acid, pivalic acid, propionic acid, succinic acid, tartaric acid, thiocyanic acid, mesilic acid, undecanoic acid, stearic acid, alginic acid, β-hydroxybutyric acid, salicylic acid, galactaric acid and galacturonic acid. Appropriate pharmaceutically acceptable based-added salts for the compound of Formula 1 include metal salts, for example, salts prepared from aluminum, calcium, lithium, magnesium, potassium, sodium and zinc, or salts prepared from organic bases including primary, secondary or tertiary amine, cyclic amine and substituted amine, for example, caffeine, arginine, diethylamine, N-ethyl piperidine, histidine, glucamine, isopropylamine, lysine, morpholine, N-ethyl morpholine, piperazine, piperidine, triethylamine, trimethylamine. These salts may be prepared from the corresponding compounds of the present invention by a common method such as reacting appropriate acids or bases with the compound of Formula 1. If a basic group and an acidic group are present in the same molecule, the compound of Formula 1 may also form an inner salt.

In the present invention, the "solvate" may include hydrates; and solvates with organic solvents such as methanol, ethanol, 2-propanol, 1,2-propandiol, 1,3-propanediol, n-butanol, 1,4-butanediol, tert-butanol, acetic acid, acetone, butyl acetate, methyl acetate, ethyl acetate, propyl acetate, t-butyl acetate, isobutyl acetate, methyl ethyl ketone, 2-pentanone, tetrahydrofuran, acetonitrile, chloroform, toluene and mixtures thereof.

According to another aspect, the present invention provides a pharmaceutical composition comprising: the novel compound of Formula 1 defined above, or the pharmaceutically acceptable salt thereof; and a pharmaceutically acceptable carrier.

The pharmaceutical composition may be useful for the prevention or treatment of protein kinase-mediated diseases, but the use of the present invention is not limited for these diseases.

In an embodiment, the pharmaceutical composition may be useful for the prevention or treatment of c-MET or RON-mediated diseases.

In an embodiment, the pharmaceutical composition may be useful for the prevention or treatment of c-MET or RON-double mediated diseases.

The pharmaceutical composition according to the present invention may include a therapeutically effective amount of the compound of Formula 1 or the pharmaceutically acceptable salt thereof.

In an embodiment, the disease may be cancer selected from the group consisting of lung cancer, breast cancer, colorectal cancer, kidney cancer, pancreatic cancer, head cancer, cervical cancer, hereditary papillary renal cell carcinoma, juvenile hepatocellular carcinoma, and stomach cancer, without limitation.

In another embodiment, the disease may be immune disorder selected from the group consisting of inflammatory disorders, cardiovascular disease, virus induced disease, circulatory system disease, fibroproliferative disease and pain sensation, without limitation.

In order to treat such diseases, the pharmaceutical composition may be administered into a subject requiring the prevention or treatment of the diseases.

In the present disclosure, the term "prevention" means the reduction of the risk of getting a disease or disorder, and means all acts suppressing or delaying the onset of a disease by preventing the progress of one or more clinical symptoms of the disease for a subject who is prone to expose to a disease or prone to a disease but does not get sick yet or show the symptoms of the disease.

In the present disclosure, the term "treatment" means the alleviation of a disease or a disorder, and means all acts improving or changing beneficially of the symptoms of a disease by stopping or reducing the progression of the disease or one or more clinical symptoms.

In the present disclosure, the term "carrier" means a compound facilitating the compound injection into cells or tissues. The pharmaceutical composition may be prepared into a unit dosage type or prepared by injecting into a multi-dose container by formulating using a pharmaceutically acceptable carrier. In this case, the formulation may be a solution in oil or aqueous medium, suspension or emulsion type, an extract, powder, granule, tablet, capsule or gel (for example, hydrogel) type, or may additionally include a dispersant or a stabilizer.

In addition, the compound represented by Formula 1 or the pharmaceutically acceptable salt thereof, included in the pharmaceutical composition may be carried in a pharmaceutically acceptable carrier such as a colloidal suspension, a powder, a saline solution, a lipid, a liposome, microspheres, and nano spherical particles. These may form a composite with a carrying means or be related therewith, and may be carried in vivo using carrying systems well known in the art, such as lipids, liposomes, minute particles, gold, nano particles, polymers, condensation reactants, polysaccharides, polyamino acids, dendrimers, saponins, adsorption enhancing substances or fatty acids.

Besides, the pharmaceutically acceptable carrier may include lactose, dextrose, sucrose, sorbitol, mannitol, starch, acacia, rubber, calcium phosphate, alginate, gelatin, calcium silicate, microcrystalline cellulose, polyvinyl pyrrolidone, cellulose, water, syrup, methyl cellulose, methyl hydroxybenzoate, propyl hydroxybenzoate, talc, magnesium stearate and mineral oil, which are commonly used for formulation, without limitation. In addition, a lubricant, a wetting agent, a sweetener, a flavoring agent, an emulsifier, a suspending agent, a preservative, or the like may be additionally included in addition to the above-described components.

The pharmaceutical composition according to the present invention may be oral or parenteral administered in clinical administration, and may be used as common pharmaceutical formulations. That is, the pharmaceutical composition of the present invention may be administered in various oral or parenteral formulations in clinical administration, and if formulated, may be formulated using commonly used diluents or excipients such as a filler, an extender, a binder, a wetting agent, a disintegrant, and a surfactant. A solid formulation for oral administration includes tablets, pills, powders, granules, capsules, or the like, and such solid formulations are formulated by mixing herbal extracts or herbal fermented products with at least one or more excipients, for example, starch, calcium carbonate, sucrose or lactose, gelatin, or the like. In addition, a lubricant such as magnesium stearate and talc is used in addition to a simple excipient. As a liquid formulation for oral administration, a suspension, an oral liquid solution, an emulsion, a syrup, or the like may be used, and various excipients, for example, a wetting agent, a sweetener, a flavoring agent, a preservative, or the like may be included in addition to water and liquid paraffin, which are frequently used simple diluents. The formulation for parenteral administration includes a sterile aqueous solution, a non-aqueous solvent, a suspension, an emulsion, a freeze-dried formulation, and a suppository. As the non-aqueous solvent and a suspension solvent, propylene glycol, polyethylene glycol, vegetable oil such as olive oil, injectable ester such as ethyl oleate, or the like may be used. As the basis of the suppository, witepsol, Macrogol, tween 61, cacao butter, laurinum, glycerol, gelatin, or the like may be used.

When administering the pharmaceutical composition for the clinical purposes, the effective dosage of Formula 1 or the pharmaceutically acceptable salt thereof may be diversified by factors such as formulation method, administration mode, patient's age, weight, gender, morbidity, diet, dosing time, dosing route, excretion rate, pharmaceutical mix and reaction sensitivity, but generally, may be administered in 0.01 to 20 mg/day, preferably, 1 to 10 mg/day per 1 kg of the weight of an adult patient, and according to the discretion of the doctor or pharmacist, may be administered in several times a day, preferably, two to three times a day with a constant time interval.

In another aspect, the present invention provides a method of treating protein kinase-mediated diseases, comprising administering a therapeutically effective amount of the compound of Formula 1 or the pharmaceutically acceptable salt thereof to a subject.

In another embodiment, the present invention provides a method of inhibiting the activity of c-MET and/or RON receptors, comprising administering a therapeutically effective amount of the compound of Formula 1 or the pharmaceutically acceptable salt thereof to a subject.

In the present disclosure, the term "therapeutically effective amount" means an amount of each formulation for accomplishing the purpose for improving disease severity and incidence frequency during the treatment of each formulation itself, while avoiding harmful side effects associated with common other treatments. For example, an effective oncology treatment prolongs the patient's survival, inhibits the growth of rapidly growing cells associated with tumors, or has an effect on tumor regression.

Hereinafter, the Example Compounds of the present invention may be prepared by the methods below, but the preparation method of the compound of the present invention is not limited thereto.

### Preparation Example 1. 4-ethoxy-1-(4-fluorophenyl)-2-oxo-1,2-dihydropyridin-3-carboxylic acid

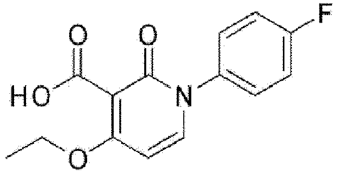

Hereinafter, the compound of Preparation Example 1 was prepared by Reaction 1 below.

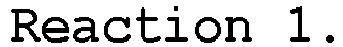

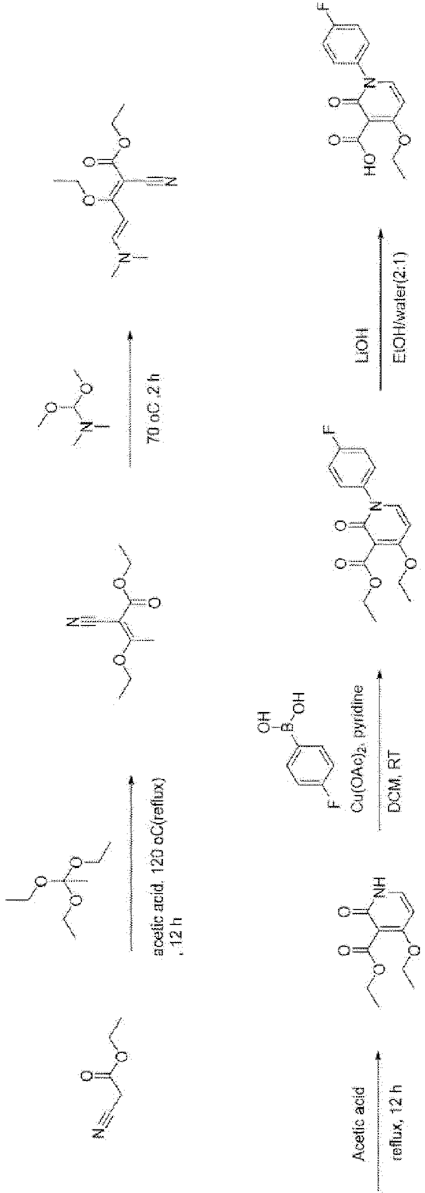

### Step 1) Ethyl 4-ethoxy-2-oxo-1,2-dihydropyridin-3-carboxylate

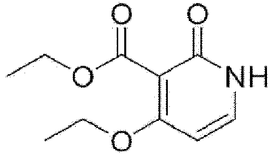

To ethyl cyanoacetate (6 g, 53 mmol), triethyl orthoacetate (17.21 g, 106 mmol), and 50 ml of acetic acid were added, followed by stirring at 120°C for 12 hours. The solvent of the reaction mixture was concentrated, and N,N-dimethylformamide diethyl acetal (DMF-DEA, 7.1 ml, 53 mmol) was added thereto. Stirring was performed at 70°C for 2 hours. To the reaction mixture, 50 ml of acetic acid and 6 ml of distilled water were added, followed by refluxing and stirring for 12 hours. The solvent of the reaction mixture was concentrated and then, the reaction mixture was diluted with water. With a sodium bicarbonate saturated aqueous solution, pH was adjusted to 9-10, and extraction was performed with DCM three times. An organic solution was washed with brine and then, concentrated. The residue was purified by column chromatography to obtain the title compound (3.6 g, yield: 32%) .
¹H NMR (400 MHz, CDCl3) δ 13.42 (brs, 1H), 7.47 (d, 1H), 6.18 (d, 1H), 4.34 (q, 2H), 4.20 (q, 2H), 1.36(m, 6H); MS m/z: 212[M+H]

### Step 2) Ethyl 4-ethoxy-1-(4-fluorophenyl)-2-oxo-1,2-dihydropyridin-3-carboxylate

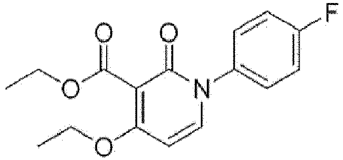

The compound obtained in Step 1 above of ethyl 4-ethoxy-2-oxo-1,2-dihydropyridin-3-carboxylate (1.2 g, 5.68 mmol) was dissolved in 50 ml of DCM, and (4-fluorophenyl)boronic acid (1.59 g, 11.4 mmol), Cu(OAc)2 (1.75 g, 9.66 mmol), and pyridine (1.35 g, 17.04 mmol) were put therein, followed by stirring at room temperature for 12 hours. The reaction mixture was filtered using celite, and solids were washed with water. The filtrate was extracted with DCM twice and concentrated to use in Step 3 below, without performing a purification process.
MS m/z: 306 [M+H]

### Step 3) 4-Ethoxy-1-(4-fluorophenyl)-2-oxo-1,2-dihydropyridin-3-carboxylic acid

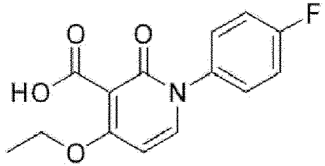

The compound obtained in Step 2 above of ethyl 4-ethoxy-1-(4-fluorophenyl)-2-oxo-1,2-dihydropyridin-3-carboxylate was dissolved in a mixture solution of ethanol/water (2:1), and LiOH (0.41 g, 17.04 mmol) was added thereto, followed by stirring at room temperature for 12 hours. After concentrating, the reaction mixture was diluted with water and washed with EA. The pH of an aqueous solution was adjusted to 2 using a 3 N hydrogen chloride aqueous solution, and extraction with DCM was performed three times. An organic solution was washed with brine and concentrated. Solids produced by adding ethyl-ether to the residue were filtered and dried to obtain the title compound (1.3 g, 83%).
MS *m*/*z:* 278 [M+H]

### Preparation Example 2. 4-Methoxy-1-(4-fluorophenyl)-2-oxo-1,2-dihydropyridin-3-carboxylic acid

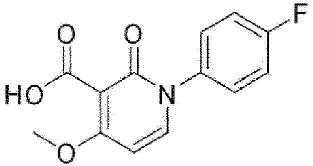

Hereinafter, the compound of Preparation Example 2 was prepared by Reaction 2 below.

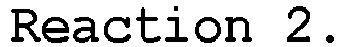

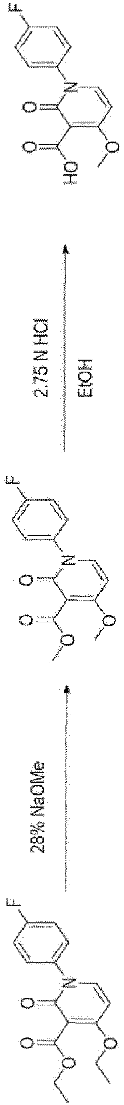

### Step 1) Methyl 1-(4-fluorophenyl)-4-methoxy-2-oxo-1,2-dihydropyridin-3-carboxylate

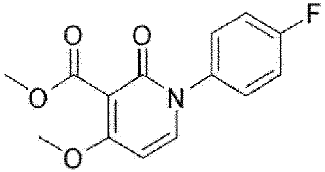

The compound obtained in Step 2 of Preparation Example 1 of ethyl 4-ethoxy-1-(4-fluorophenyl)-2-oxo-1,2-dihydropyridin-3-carboxylate (0.5 g, 1.64 mmol) was treated with a 28% NaOMe solution and stirred at room temperature for 10 minutes. The reaction mixture was concentrated to obtain the title compound (0.36 g, 79%).
MS m/z: 278 [M+H]

### Step 2) 4-Methoxy-1-(4-fluorophenyl)-2-oxo-1,2-dihydropyridin-3-carboxylic acid

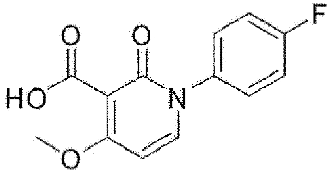

The compound obtained in Step 1 above of methyl 1-(4-fluorophenyl)-4-methoxy-2-oxo-1,2-dihydropyridin-3-carboxylate (0.36 g, 1.29 mmol) was dissolved in 5 ml of ethanol, and 5 ml of a 3 N hydrogen chloride solution was added thereto at room temperature. After stirring at 60°C for 4 hours, the solids thus produced were filtered to obtain the title compound (0.23 g, 68%).
MS *m*/*z:* 264 [M+H]

### Preparation Example 3. 4'-Fluoro-5-(1-methyl-1H-pyrazol-4-yl)-2-oxo-1,2-dihydro-[1,1'-biphenyl]-3-carboxylic acid

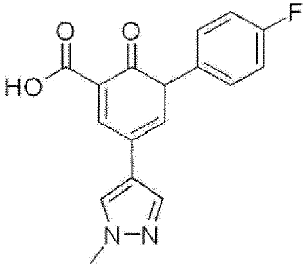

Hereinafter, the compound of Preparation Example 3 was prepared according to Reaction 3 below.

### Reaction 3.

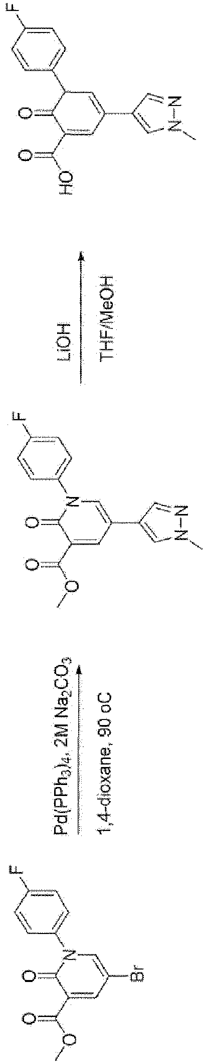

### Step 1) Methyl 5-bromo-1-(4-fluorophenyl)-2-oxo-1,2-dihydropyridin-3-carboxylate

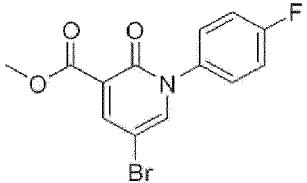

According to Step 2 of Preparation Example 1, by a similar method using methyl 5-bromo-2-oxo-1,2-dihydropyridin-3-carboxylate (5 g, 21.55 mmol) and (4-fluorophenyl)boronic acid (6.03 g, 43.1 mmol), the title compound (4.8 g, 68%) was obtained.
MS *m*/*z:* 326[M], 328[M+2]

### Step 2) Methyl 1-(4-fluorophenyl)-5-(1-methyl-1H-pyrazol-4-yl)-2-oxo-1,2-dihydropyridin-3-carboxylate

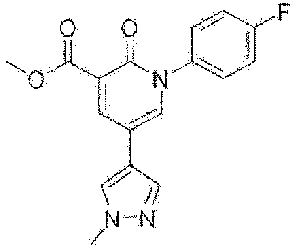

The compound obtained in Step 1 above of methyl 5-bromo-1-(4-fluorophenyl)-2-oxo-1,2-dihydropyridin-3-carboxylate (100 mg, 0.28 mmol) was dissolved in 1,4-dioxane, and 1-methyl-4-(4,4,5,5-tetramethyl-1,3,2-dioxaborolan-2-yl)-1H-pyrazole (64 mg, 0.31 mmol), a 2 N Na₂CO₃ aqueous solution (0.4 ml, 0.7 mmol), and Pd(PPh₃)₄ (5 mg, 0.05 mmol) were added thereto, followed by stirring at 95°C for 12 hours. A solution was cooled and then, diluted with water, extracted with DCM three times, and concentrated. Through purifying by silica gel chromatography, the title compound (80 mg, yield: 87%) was obtained.
MS *m*/*z:* 328[M+H]

### Step 3) 4'-Fluoro-5-(1-methyl-1H-pyrazol-4-yl)-2-oxo-1,2-dihydro-[1,1'-biphenyl]-3-carboxylic acid

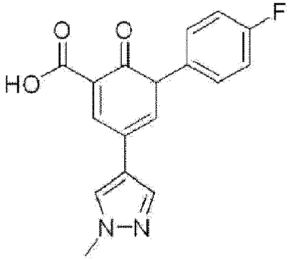

According to Step 3 of Preparation Example 1, by a similar method using the compound obtained in Step 2 above of methyl 1-(4-fluorophenyl)-5-(1-methyl-1H-pyrazol-4-yl)-2-oxo-1,2-dihydropyridin-3-carboxylate (80 mg, 0.24 mmol) and LiOH (17.2 mg, 0.72 mmol), the title compound (50 mg, 67%) was obtained.
MS *m*/*z:* 313[M+H]

### Preparation Example 4. 1-(4-Fluorophenyl)-2-oxo-6-(trifluoromethyl)-1,2-dihydropyridin-3-carboxylic acid

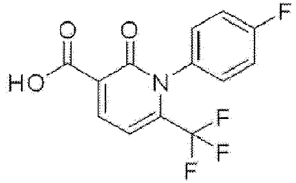

Hereinafter, the compound of Preparation Example 4 was prepared according to Reaction 4 below.

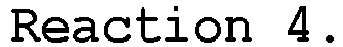

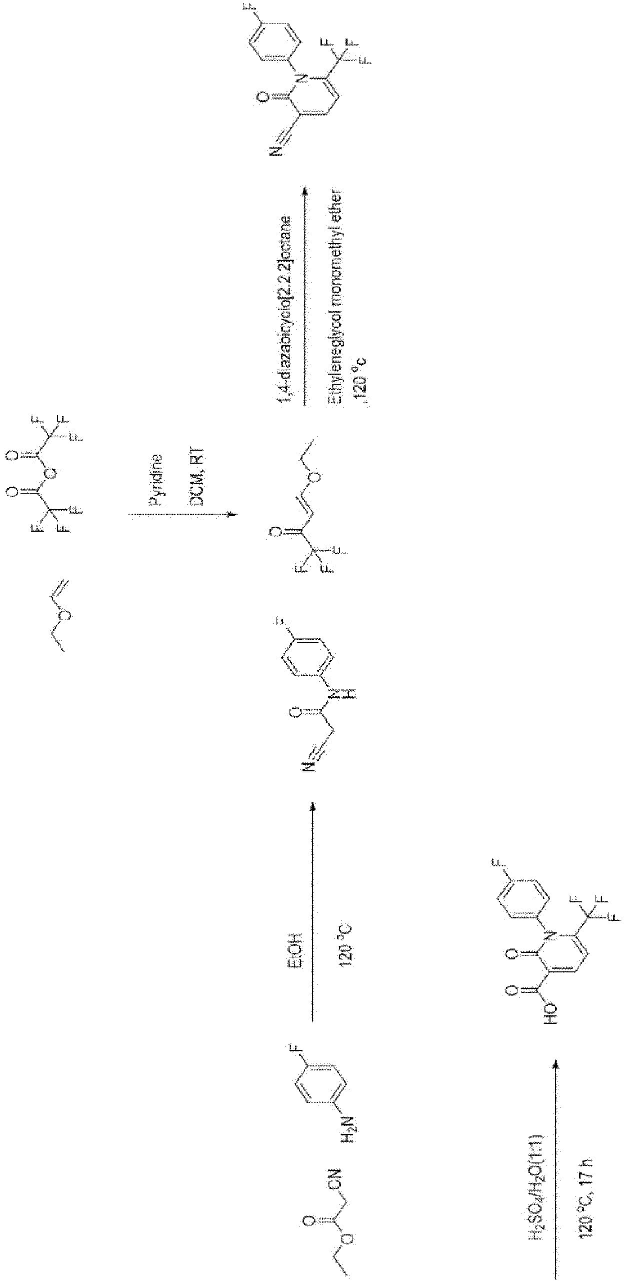

### Step 1) 2-Cyano-N-(4-fluorophenyl)acetamide

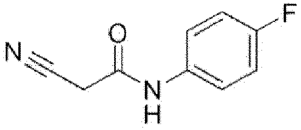

4-Fluoroaniline (820 mg, 7.36 mmol) was dissolved in 10 ml of ethanol, and ethyl 2-cyanoacetate (1 g, 8.84 mmol) was added thereto, followed by stirring at 120°C for 5 hours. The reaction mixture was cooled to 0°C, and the solids thus produced were filtered to obtain the title compound (1.1 g, 84%) .
MS *m*/*z:* 179[M+H]

### Step 2) (E)-4-ethoxy-1,1,1-trifluorobut-3-en-2-one

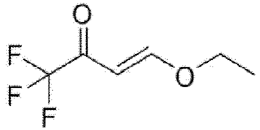

Ethyl vinyl ether (2 g, 27.74 mmol) was dissolved in 70 ml of DCM, and pyridine (2.6 g, 33.29 mmol) and trifluoro acetic acid anhydride (3.48 g, 30.51 mmol) were added thereto at 0°C, followed by stirring at room temperature for 1 hour. The resultant was diluted with water, extracted with DCM three times, and concentrated to obtain the title compound (3.7 g, 80%) .
MS *m*/*z:* 169[M+H]

### Step 3) 1-(4-Fluorophenyl)-2-oxo-6-(trifluoromethyl)-1,2-dihydro pyridin-3-carbonitrile

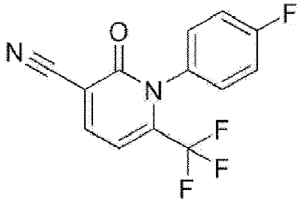

The compound obtained in Step 1 above of 2-cyano-N-(4-fluorophenyl)acetamide (1.1 g, 6.17 mmol) and the compound obtained in Step 2 above of (E)-4-ethoxy-1,1,1-trifluorobut-3-en-2-one (1.2 g, 7.40 mmol) were dissolved in 15 ml of ethylene glycol monomethyl ether, and 1,4-diazabicyclo[2.2.2]octane (0.69 g, 6.17 mmol) was added thereto, followed by stirring at 120°C for 6 hours. The reaction mixture was diluted using 1N HCl, extracted with EA three times, and concentrated. Through purifying by silica gel chromatography, the title compound (500 mg, yield: 29%) was obtained.
MS *m*/*z:* 283[M+H]

### Step 4) 1-(4-Fluorophenyl)-2-oxo-6-(trifluoromethyl)-1,2-dihydropyridin-3-carboxylic acid

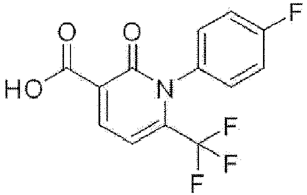

The compound obtained in Step 3 above of 1-(4-fluorophenyl)-2-oxo-6-(trifluoromethyl)-1,2-dihydropyridin-3-carbonitrile (500 mg, 1.77 mmol) was dissolved in a mixture solution of conc sulfuric acid (2.5 ml)/water (2.5 ml), followed by stirring at 120°C for 12 hours. The reaction mixture was cooled to room temperature, diluted with a 6 N NaOH aqueous solution, and washed with EA. Solids produced by adding 1 N HCl to an aqueous layer were filtered and washed with water. The solids thus filtered were dried to obtain the title compound (400 mg, yield: 75%).
MS *m*/*z:* 302[M+H]

### Example 1. N-(3'-(2-aminopyrimidin-4-yl)-2-fluoro-4'-hydroxy-[1,1'-biphenyl]-4-yl)-4-ethoxy-1- (4-fluorophenyl)-2-oxo-1,2-dihydropyridin-3-carboxamide

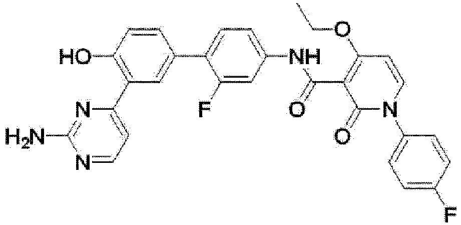

Hereinafter, the compound of Example 1 was prepared according to Reaction 5 below.

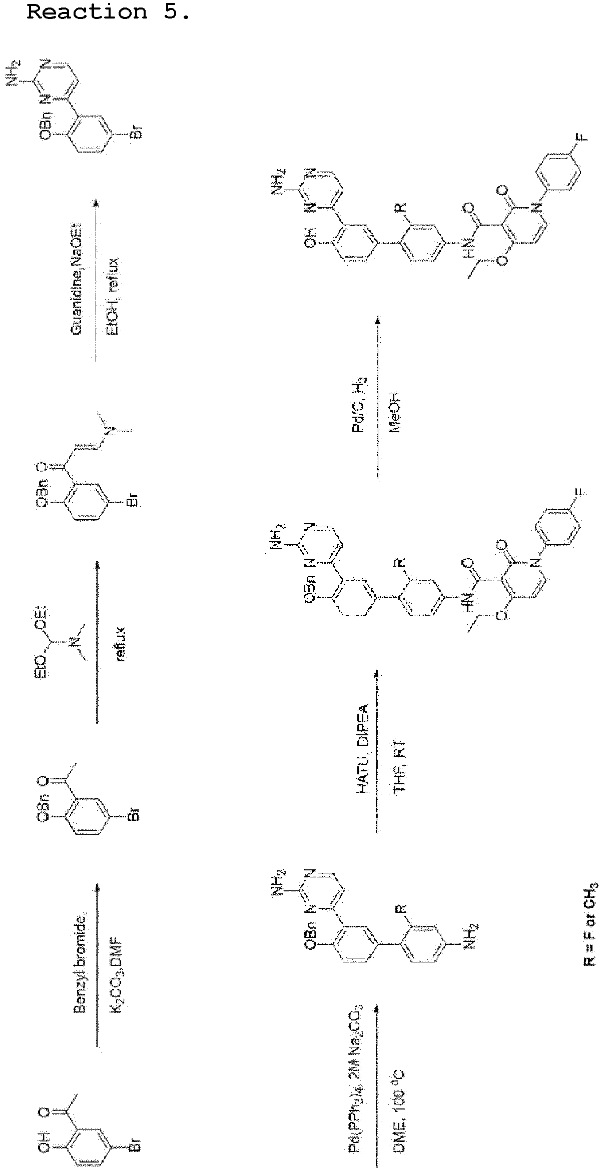

### Step 1. 1-(2-(Benzyloxy)-5-bromophenyl)ethan-1-one

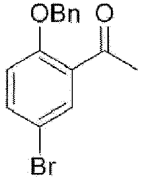

5'-Bromo-2' hydroxyacetophenone (3 g, 13.9 mmol) was dissolved in 50 ml of N,N-dimethylformamide, K₂CO₃ (2.9 g, 20.9 mmol) was added thereto, and benzyl bromide (2 ml, 16.8 mmol) was added thereto, followed by stirring at room temperature for 12 hours. After completing the reaction, the resultant was diluted with 100 ml of EA, washed with 500 ml of water and 300 ml of brine, and concentrated. Through purifying by silica gel chromatography, the title compound (4.2 g, yield: 98%) was obtained.
MS *m*/*z:* 305[M], 307[M+2]

### Step 2. (E)-1-(2-(benzyloxy)-5-bromophenyl)-3-(dimethylamino)prop-2-en-1-one

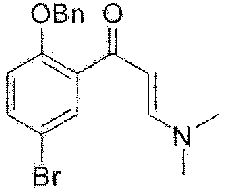

The compound obtained in Step 1 above of 1-(2-(benzyloxy) -5-bromophenyl) ethan-1-one (4.2 g, 12.0 mmol) and N,N'-dimethylformamide diethyl acetal (23 ml, 140 mmol) were mixed and then, refluxed and stirred at 120°C for 12 hours. After completing the reaction, the resultant was concentrated to obtain the title compound (4 g, yield: 93%).
MS *m*/*z:* 360[M], 362[M+2]

### Step 3. 4-(2-(Benzyloxy)-5-bromophenyl)pyrimidin-2-amine

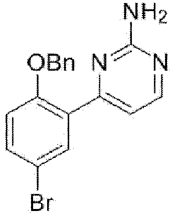

The compound obtained in Step 2 above of (E)-1-(2-(benzyloxy)-5-bromophenyl)-3-(dimethylamino)prop-2-en-1-one (4 g, 11.1 mmol) was dissolved in 70 ml of ethanol, and guanidine hydrochloride (1.3 g, 13.6 mmol) and sodium ethoxide (2.3 g, 33.3 mmol) were added thereto, followed by refluxing and stirring for 12 hours. After completing the reaction, the resultant was diluted with water, extracted with DCM three times, and concentrated. Through purifying by silica gel chromatography, the title compound (2.7 g, yield: 68%) was obtained.
MS *m*/*z:* 356[M], 358[M+2]

### Step 4. 4-(4'-Amino-4-(benzyloxy)-2'-fluoro-[1,1'-biphenyl]-3-yl)pyrimidin-2-amine

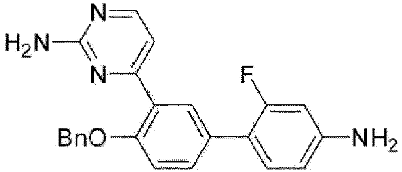

The compound obtained in Step 3 above of 4-(2-(benzyloxy)-5-bromophenyl)pyrimidin-2-amine (300 mg, 0.84 mmol) was dissolved in 7 ml of DME, and 3-fluoro-4-(4,4,5,5-tetramethyl-1,3,2-dioxaborolan-2-yl)aniline (240 mg, 1.1 mmol), a 2 N Na2CO3 aqueous solution (1.1 ml, 2.1 mmol), and Pd(PPh3)4 (20 mg, 0.2 mmol) were added thereto, followed by stirring at 95°C for 12 hours. The solution was cooled, diluted with water, extracted with DCM three times, and concentrated. Through purifying by silica gel chromatography, the title compound (220 mg, yield: 67%) was obtained.
MS *m*/*z:* 387[M+H]

### Step 5. N-(3'-(2-aminopyrimidin-4-yl)-4'-(benzyloxy)-2-fluoro-[1,1'-biphenyl]-4-yl)-4-ethoxy-1-(4-fluorophenyl)-2-oxo-1,2-dihydropyridin-3-carboxamide

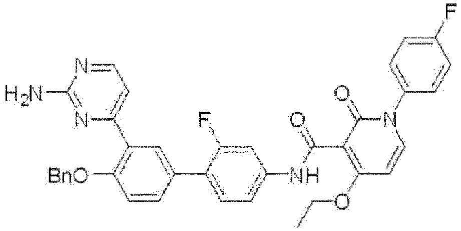

The compound obtained in Step 4 above of 4-(4'-amino-4-(benzyloxy)-2'-fluoro-[1,1'-biphenyl]-3-yl)pyrimidin-2-amine (50 mg, 0.13 mmol) was dissolved in 3 ml of THF, and 4-ethoxy-1-(4-fluorophenyl)-2-oxo-1,2-dihydropyridin-3-carboxylic acid (43 mg, 0.16 mmol), HATU (74 mg, 0.19 mmol), and DIPEA (45 µl, 0.26 mmol) were added thereto, followed by stirring at room temperature for 12 hours. The reaction solution was diluted with water, extracted with EA three times, and concentrated. The residue was purified by silica gel chromatography to obtain the title compound (55 mg, 66%).
MS *m*/*z:* 646[M+H]

### Step 6. N-(3'-(2-aminopyrimidin-4-yl)-2-fluoro-4'-hydroxy-[1,1'-biphenyl]-4-yl)-4-ethoxy-1-(4-fluorophenyl)-2-oxo-1,2-dihydropyridin-3-carboxamide

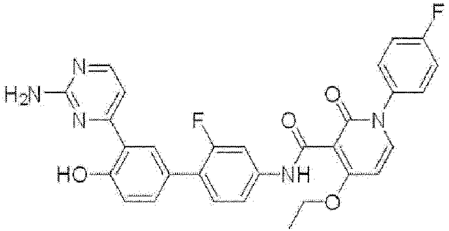

The compound obtained in Step 5 above of N-(3'-(2-aminopyrimidin-4-yl)-4'-(benzyloxy)-2-fluoro-[1,1'-biphenyl]-4-yl)-4-ethoxy-1-(4-fluorophenyl)-2-oxo-1,2-dihydropyridin-3-carboxamide (55 mg, 0.085 mmol) was dissolved in 5 ml of methanol, and 10% Pd/C was added thereto, followed by stirring under a hydrogen atmosphere. A catalyst was filtered through celite, and the filtrate was dried in vacuum. The residue was purified by prep-HPLC (0.1% formic acid in water/acetonitrile) to obtain the title compound (34.7 mg, yield: 73%).
¹H NMR (400 MHz, DMSO-d₆) δ 13.98 (s, 1H), 10.48 (s, 1H), 8.37 (d, 1H), 8.18 (d, 1H), 7.86 (d, 1H), 7.76 (d, 1H), 7.52 (m, 2H), 7.48 (m, 3H), 7.37 (m, 3H), 7.16 (brs, 2H), 6.98 (d, 1H), 6.47 (d, 1H), 4.21 (q, 2H), 1.23(t, 3H);
MS *m*/*z:* 556[M+H]

### Example 2. N-(3'-(2-aminopyrimidin-4-yl)-2-fluoro-4'-hydroxy-[1,1'-biphenyl]-4-yl)-1-(4-fluorophenyl)-4-methoxy-2-oxo-1,2-dihydropyridin-3-carboxamide

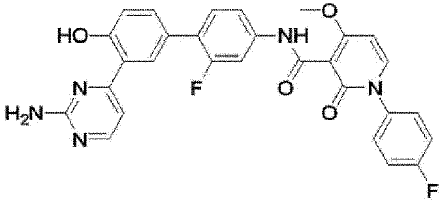

According to Step 5 of Example 1, by using 1-(4-fluorophenyl)-4-methoxy-2-oxo-1,2-dihydropyridin-3-carboxylic acid instead of 4-ethoxy-1-(4-fluorophenyl)-2-oxo-1,2-dihydropyridin-3-carboxylic acid and by a similar method as Example 1, the title compound (33.5 mg, yield: 71%) was obtained.
¹H NMR (400 MHz, DMSO-d₆) δ 13.88 (s, 1H), 10.56 (s, 1H), 8.39 (d, 1H), 8.05 (s, 1H), 7.91 (d, 1H), 7.78 (d, 1H), 7.54 (m, 2H), 7.49 (m, 3H), 7.36 (m, 3H), 7.17 (brs, 2H), 7.01 (d, 1H), 6.55 (d, 1H), 3.93(s, 3H);
MS *m*/*z:* 542[M+H]

### Example 3. N-(3'-(2-aminopyrimidin-4-yl)-2-fluoro-4'-hydroxy-[1,1'-biphenyl]-4-yl)-1-(4-fluorophenyl)-6-methyl-2-oxo-1,2-dihydropyridin-3-carboxamide

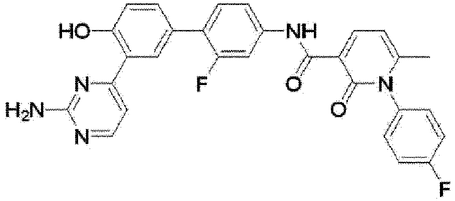

According to Step 5 of Example 1, by using 1-(4-fluorophenyl)-6-methyl-2-oxo-1,2-dihydropyridin-3-carboxylic acid instead of 4-ethoxy-1-(4-fluorophenyl)-2-oxo-1,2-dihydropyridin-3-carboxylic acid and by a similar method as Example 1, the title compound (8 mg, yield: 35%) was obtained.
¹H NMR (400 MHz, DMSO-d₆) δ 13.97 (s, 1H), 12.08 (s, 1H), 8.51 (d, 1H), 8.38 (d, 1H), 8.18 (s, 1H), 7.96 (d, 1H), 7.52-7.35 (m, 7H), 7.25 (m, 1H), 7.17 (brs, 2H), 6.99 (d, 1H), 6.65 (d, 1H), 2.15 (s, 3H);
MS *m*/*z:* 526[M+H]

### Example 4. N-(3'-(2-aminopyrimidin-4-yl)-2-fluoro-4'-hydroxy-[1,1'-biphenyl]-4-yl)-1,5-dimethyl-3-oxo-2-phenyl-2,3-dihydro-1H-pyrazol-4-carboxamide

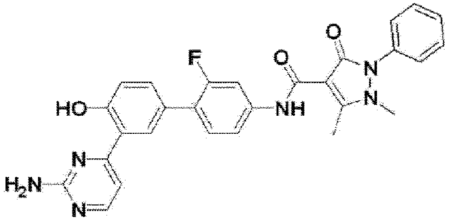

According to Step 5 of Example 1, by using 1,5-dimethyl-3-oxo-2-phenyl-2,3-dihydro-1H-pyrazol-4-carboxylic acid instead of 4-ethoxy-1-(4-fluorophenyl)-2-oxo-1,2-dihydropyridin-3-carboxylic acid and by a similar method as Example 1, the title compound (51 mg, yield: 59%) was obtained.
¹H NMR (400 MHz, DMSO-d₆) δ 13.94 (s, 1H), 10.96 (s, 1H), 8.39 (d, 1H), 8.05 (d, 1H), 7.84 (d, 1H), 7.62-7.44 (m, 7H), 7.36 (m, 2H), 7.18 (brs, 2H), 7.00 (d, 1H), 3.37 (s, 3H), 2.71 (s, 3H);
MS *m*/*z:* 511[M+H]

### Example 5. N-(3'-(2-aminopyrimidin-4-yl)-2-fluoro-4'-hydroxy-[1,1'-biphenyl]-4-yl)-1-phenyl-5-(trifluoromethyl)-1H-imidazol-4-carboxamide

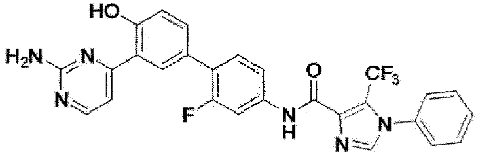

According to Step 5 of Example 1, by using 1-phenyl-5-(trifluoromethyl)-1H-imidazol-4-carboxylic acid instead of 4-ethoxy-1-(4-fluorophenyl)-2-oxo-1,2-dihydropyridin-3-carboxylic acid and by a similar method as Example 1, the title compound (20 mg, yield: 22%) was obtained.
¹H NMR (400 MHz, DMSO-d₆) δ 13.96 (s, 1H), 10.72 (s, 1H), 8.40 (m, 1H), 8.36 (s, 1H), 8.08 (m, 1H), 7.92 (d, 1H), 7.76 (m, 1H), 7.67-7.55 (m, 7H), 7.36 (d, 1H), 7.20 (brs, 2H), 7.03 (d, 1H);
MS *m*/*z:* 511[M+H]

### Example 6. N-(3'-(2-aminopyrimidin-4-yl)-2-fluoro-4'-hydroxy-[1,1'-biphenyl]-4-yl)-5-bromo-1-(4-fluorophenyl)-2-oxo-1,2-dihydropyridin-3-carboxamide

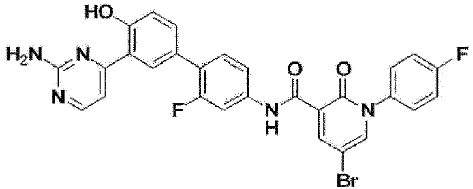

According to Step 5 of Example 1, by using 5-bromo-1-(4-fluorophenyl)-2-oxo-1,2-dihydropyridin-3-carboxylic acid instead of 4-ethoxy-1-(4-fluorophenyl)-2-oxo-1,2-dihydropyridin-3-carboxylic acid and by a similar method as Example 1, the title compound (80 mg, yield: 40%) was obtained.
¹H NMR (400 MHz, DMSO-d₆) δ 13.96 (s, 1H), 11.95 (s, 1H), 8.54 (m, 2H), 8.39 (s, 1H), 8.07 (m, 1H), 7.89 (d, 1H), 7.66 (m, 2H), 7.64-7.45 (m, 5H), 7.37 (d, 1H), 7.19 (brs, 2H), 7.01 (d, 1H);
MS *m*/*z:* 591[M+H]

### Example 7. N-(3'-(2-aminopyrimidin-4-yl)-2-fluoro-4'-hydroxy-[1,1'-biphenyl]-4-yl)-1-(4-fluorophenyl)-5-(1-methyl-1H-pyrazol-4-yl)-2-oxo-1,2-dihydropyridin-3-carboxamide

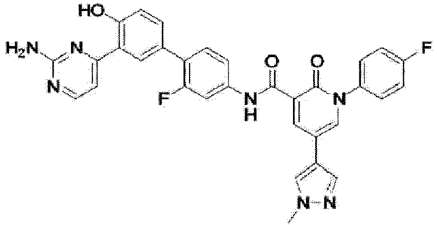

According to Step 5 of Example 1, by using 1-(4-fluorophenyl)-5-(1-methyl-1H-pyrazol-4-yl)-2-oxo-1,2-dihydropyridin-3-carboxylic acid instead of 4-ethoxy-1-(4-fluorophenyl)-2-oxo-1,2-dihydropyridin-3-carboxylic acid and by a similar method as Example 1, the title compound (9 mg, yield: 23%) was obtained.
¹H NMR (400 MHz, DMSO-d₆) δ 13.98 (s, 1H), 12.23 (s, 1H), 8.78 (s, 1H), 8.43 (d, 2H), 8.27 (d, 1H), 8.09 (m, 1H), 7.94 (m, 2H), 7.70 (m, 2H), 7.60 (m, 2H), 7.49 (m, 3H), 7.39 (m, 1H), 7.20 (brs, 2H), 7.02 (d, 1H), 3.88 (s, 3H);
MS *m*/*z:* 592[M+H]

### Example 8. N-3'-(2-aminopyrimidin-4-yl)-2-fluoro-4'-hydroxy-[1,1'-biphenyl]-4-yl)-5-cyclopropyl-1-(4-fluorophenyl)-2-oxo-1,2-dihydropyridin-3-carboxamide

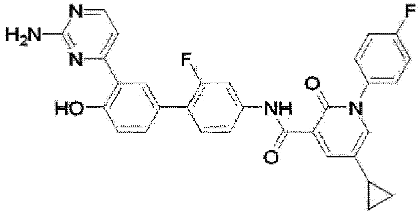

To a toluene/water (10:1) solution of the compound obtained in Example 6 of N-(3'-(2-aminopyrimidin-4-yl)-2-fluoro-4'-hydroxy-[1,1'-biphenyl]-4-yl)-5-bromo-1-(4-fluorophenyl)-2-oxo-1,2-dihydropyridin-3-carboxamide (25 mg, 0.04 mmol), cyclopropylboronic acid (5.5 mg, 0.064 mmol), tricyclohexylphosphine (1.3 mg, 0.005 mmol), K₃PO₄ (22.5 mg, 0.11 mmol), and Pd(OAc)₂ (1 mg, 0.005 mmol) were added, followed by stirring at 140°C for 30 minutes. The reaction solution was diluted with water, extracted with ethyl acetate three times, and concentrated. The residue was purified by prep-HPLC (0.1% formic acid in water/acetonitrile) to obtain the title compound (4.8 mg, yield: 21%).
¹H NMR (400 MHz, DMSO-d₆) δ 13.95 (s, 1H), 12.23 (s, 1H), 8.39 (d, 1H), 8.07 (m, 1H), 7.96 (m, 1H), 7.90 (d, 1H), 7.87 (m, 1H), 7.64-7.54 (m, 4H), 7.44 (m, 3H), 7.36 (m, 1H), 7.18 (brs, 2H), 6.99 (d, 1H), 1.97 (m, 1H), 0.93 (m, 2H), 0.78 (m, 2H);
MS *m*/*z:* 552[M+H]

### Example 9. N-(3'-(2-aminopyrimidin-4-yl)-2-fluoro-4'-hydroxy-[1,1'-biphenyl]-4-yl)-1-(4-fluorophenyl)-2-oxo-6-(trifluoromethyl)-1,2-dihydropyridin-3-carboxamide

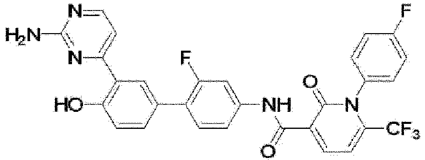

According to Step 5 of Example 1, by using 1-(4-fluorophenyl)-2-oxo-6-(trifluoromethyl)-1,2-dihydropyridin-3-carboxylic acid instead of 4-ethoxy-1-(4-fluorophenyl)-2-oxo-1,2-dihydropyridin-3-carboxylic acid and by a similar method as Example 1, the title compound (23 mg, yield: 39%) was obtained.
¹H NMR (400 MHz, DMSO-d₆) δ 13.95 (s, 1H), 11.75 (s, 1H), 8.66 (d, 1H), 8.38 (d, 1H), 8.06 (s, 1H), 7.89 (d, 1H), 7.61-7.55 (m, 5H), 7.49 (m, 2H), 7.35 (m, 2H), 7.18 (brs, 2H), 7.01 (d, 1H);
MS *m*/*z:* 580[M+H]

### Example 10. N-(3'-(2-aminopyrimidin-4-yl)-2-fluoro-4'-hydroxy-[1,1'-biphenyl]-4-yl)-1-(4-fluorophenyl)-6-(hydroxymethyl)-2-oxo-1,2-dihydropyridin-3-carboxamide

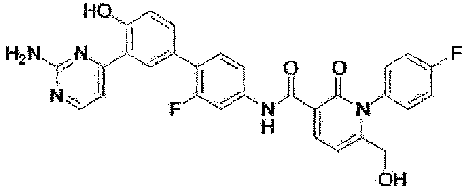

According to Step 5 of Example 1, by using 1-(4-fluorophenyl)-6-(hydroxymethyl)-2-oxo-1,2-dihydropyridin-3-carboxylic acid instead of 4-ethoxy-1-(4-fluorophenyl)-2-oxo-1,2-dihydropyridin-3-carboxylic acid and by a similar method as Example 1, the title compound (9.8 mg, yield: 18%) was obtained.
¹H NMR (400 MHz, DMSO-d₆) δ 13.94 (s, 1H), 12.06 (s, 1H), 8.64 (d, 1H), 8.38 (s, 1H), 8.15 (s, 1H), 8.06 (s, 1H), 7.90 (d, 1H), 7.58-7.46 (m, 4H), 7.42 (m, 2H), 7.35 (m, 1H), 7.17 (brs, 2H), 7.01 (s, 1H), 6.88 (d, 1H), 5.80 (brs, 1H), 4.02 (s, 2H);
MS *m*/*z:* 542[M+H]

### Example 11. N-(3'-(2-aminopyrimidin-4-yl)-4'-(benzyloxy)-2-methyl-[1,1'-biphenyl]-4-yl)-4-ethoxy-1-(4-fluorophenyl)-2-oxo-1,2-dihydropyridin-3-carboxamide

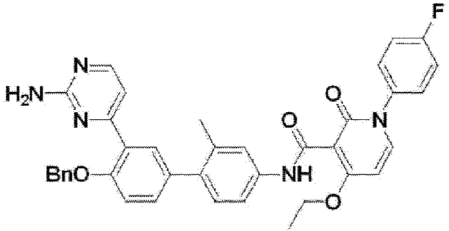

According to Step 4 of Example 1, by using 3-methyl-4-(4,4,5,5-tetramethyl-1,3,2-dioxaborolan-2-yl)aniline instead of 3-fluoro-4-(4,4,5,5-tetramethyl-1,3,2-dioxaborolan-2-yl)aniline and by a similar method as Example 1, the title compound (25 mg, yield: 37%) was obtained.
¹H NMR (400 MHz, CDCl₃) δ 11.28 (s, 1H), 8.27 (m, 1H), 7.89 (s, 1H), 7.69 (m, 2H), 7.56-7.38 (m, 9H), 7.31 (m, 2H), 7.28 (d, 1H), 7.12 (d, 1H), 6.37 (d, 1H), 5.26 (s, 2H), 5.06 (brs, 2H), 4.35 (q, 2H), 2.27 (s, 3H), 1.58(t, 3H);
MS *m*/*z:* 556[M+H]

### Example 12. N-(3'-(2-aminopyrimidin-4-yl)-4'-(benzyloxy)-2-methyl-[1,1'-biphenyl]-4-yl)-N-(4-fluoro phenyl)cyclopropan-1,1-dicarboxamide

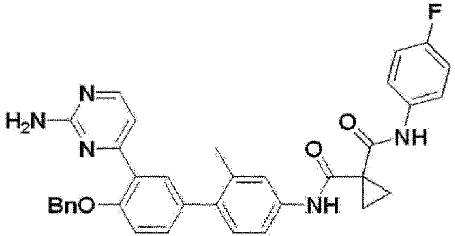

By a similar method as Example 11 and using 1-((4-fluorophenyl)carbamoyl)cyclopropan-1-carboxylic acid instead of 4-ethoxy-1-(4-fluorophenyl)-2-oxo-1,2-dihydropyridin-3-carboxylic acid of Example 11, the title compound (30 mg, yield: 49%) was obtained.
¹H NMR (400 MHz, CDCl₃) δ 9.36 (s, 1H), 8.72 (s, 1H), 8.26 (s, 1H), 7.88 (s, 1H), 7.58 (m, 2H), 7.50-7.38 (m, 9H), 7.30 (m, 1H), 7.18 (m, 1H), 7.08 (m, 2H), 5.06 (brs, 2H), 2.28 (s, 3H), 1.68(m, 2H), 1.56 (m, 2H);
MS *m*/*z:* 588[M+H]

### Example 13. N-(3'-(2-aminopyrimidin-4-yl)-4'-hydroxy-2-methyl-[1,1'-biphenyl]-4-yl)-4-ethoxy-1-(4-fluorophenyl)-2-oxo-1,2-dihydropyridin-3-carboxamide

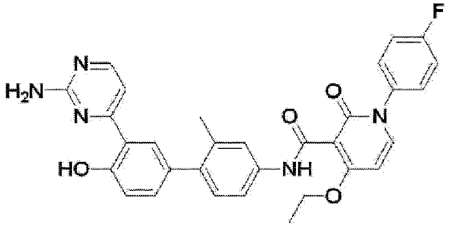

According to Step 4 of Example 1, by using 3-methyl-4-(4,4,5,5-tetramethyl-1,3,2-dioxaborolan-2-yl)aniline instead of 3-fluoro-4-(4,4,5,5-tetramethyl-1,3,2-dioxaborolan-2-yl)aniline and by a similar method as Example 1, the title compound (5.6 mg, yield: 36%) was obtained.
¹H NMR (400 MHz, MeOD-d₄) δ 8.32 (m, 1H), 7.82 (s, 1H), 7.76 (d, 1H), 7.59 (s, 1H), 7.48 (d, 1H), 7.35 (m, 2H), 7.29-7.20 (m, 5H), 6.99 (m, 1H), 6.61 (d, 1H), 4.35 (q, 2H), 2.30 (s, 3H), 1.48(t, 3H);
MS *m*/*z:* 552 [M+H]

### Example 14. N-(3'-(2-aminopyrimidin-4-yl)-4'-hydroxy-2-methyl-[1,1'-biphenyl]-4-yl)-1-(4-fluoro phenyl)-6-methyl-2-oxo-1,2-dihydropyridin-3-carboxamide

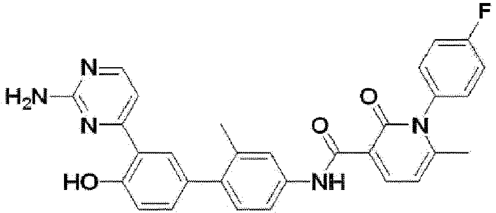

By a similar method as Example 13 and using 1-(4-fluorophenyl)-6-methyl-2-oxo-1,2-dihydropyridin-3-carboxylic acid instead of 4-ethoxy-1-(4-fluorophenyl)-2-oxo-1,2-dihydropyridin-3-carboxylic acid of Example 13, the title compound (7.6 mg, yield: 45%) was obtained.
¹H NMR (400 MHz, DMSO-d₆) δ 13.86 (s, 1H), 11.98 (s, 1H), 8.53 (d, 1H), 8.38 (m, 1H), 7.88 (s, 1H), 7.66 (d, 1H), 7.52 (s, 1H), 7.50-7.38 (m, 4H), 7.30 (m, 2H), 7.20 (d, 1H), 7.16 (brs, 2H), 6.90 (d, 1H), 6.68 (d, 1H), 2.30 (s, 3H), 2.15 (s, 3H);
MS *m*/*z:* 522[M+H]

### Example 15. N-(3'-(2-aminopyrimidin-4-yl)-4'-hydroxy-2-methyl-[1,1'-biphenyl]-4-yl)-N-(4-fluorophenyl)cyclopropan-1,1-dicarboxamide

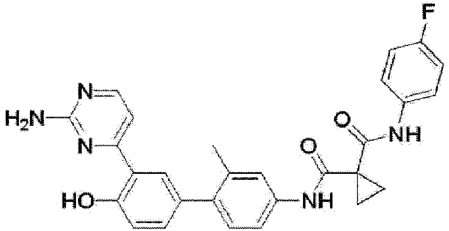

By a similar method as Example 13 and using 1-((4-fluorophenyl)carbamoyl)cyclopropan-1-carboxylic acid instead of 4-ethoxy-1-(4-fluorophenyl)-2-oxo-1,2-dihydropyridin-3-carboxylic acid of Example 13, the title compound (9.9 mg, yield: 39.0%) was obtained.
¹H NMR (400 MHz, MeOD-d₄) δ 8.36 (m, 1H), 7.79 (s, 1H), 7.59 (m, 2H), 7.48 (m, 2H), 7.32 (d, 1H), 7.28 (m, 2H), 710 (m, 2H), 6.98 (d, 1H), 2.28 (s, 3H), 1.68(m, 4H);
MS *m*/*z:* 498[M+H]

### Example 16. N-(3'-(2-amino-1,4,5,6-tetrahydropyrimidin-4-yl)-4'-hydroxy-2-methyl-[1,1'-biphenyl]-4-yl)-N-(4-fluorophenyl)cyclopropan-1,1-dicarboxamide

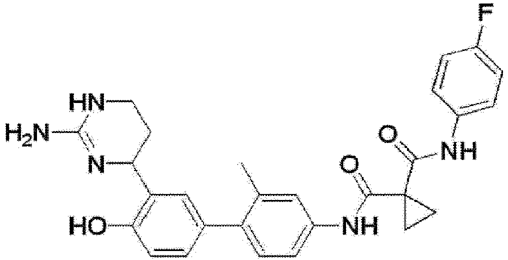

During hydrogenolysis of the benzyl group of the compound obtained in Example 12 of N-(3'-(2-aminopyrimidin-4-yl)-4'-(benzyloxy)-2-methyl-[1,1'-biphenyl]-4-yl)-N-(4-fluoro phenyl)cyclopropan-1,1-dicarboxamide using a Pd/C catalyst, the title compound (4.2 mg, yield: 16.4%) was obtained as the by-product of Example 15.
¹H NMR (400 MHz, MeOD-d₄) δ 8.36 (m, 1H), 7.79 (s, 1H), 7.59 (m, 2H), 7.48 (m, 2H), 7.32 (d, 1H), 7.28 (m, 2H), 710 (m, 2H), 6.98 (d, 1H), 2.28 (s, 3H), 1.68(m, 4H);
MS *m*/*z:* 502[M+H]

### Example 17. N-(3'-(2-aminopyrimidin-5-yl)-2-fluoro-4'-methoxy-[1,1'-biphenyl]-4-yl)-4-ethoxy-1-(4-fluorophenyl)-2-oxo-1,2-dihydropyridin-3-carboxamide

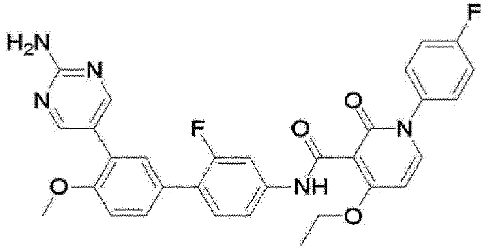

Hereinafter, the compound of Example 17 was prepared according to Reaction 6 below.

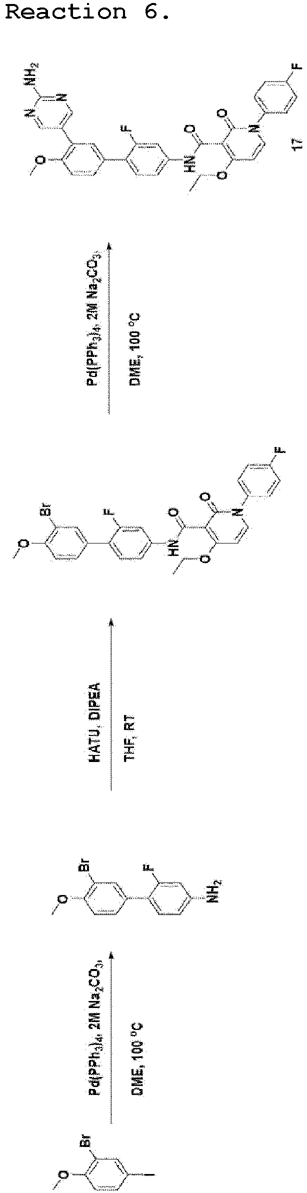

### Step 1. 3'-Bromo-2-fluoro-4'-methoxy(1,1'-biphenyl)-4-amine

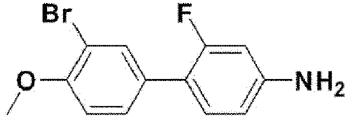

2-Bromo-4-iodo-1-methoxybenzene (792 mg, 2.53 mmol) was dissolved in 15 ml of toluene, and 3-fluoro-4-(4,4,5,5-tetramethyl-1,3,2-dioxaborolan-2-yl)aniline (0.5 g, 2.11 mmol), Cs₂CO₃ (1.03 g, 3.16 mmol), Pd(OAc)₂ (14 mg, 0.06 mmol) and triphenylphosphine (55 mg, 0.21 mmol) were added thereto, followed by refluxing and stirring at 115°C for 12 hours. The solution was cooled to room temperature, a catalyst was filtered through celite, and the filtrate was dried in vacuum. The residue was purified by silica gel chromatography to obtain the title compound (416 mg, yield: 67%).
MS *m*/*z:* 296 [M], 298[M+2]

### Step 2. N-(3'-bromo-2-fluoro-4'-methoxy-[1,1'-biphenyl]-4-yl)-4-ethoxy-1-(4-fluorophenyl)-2-oxo-1,2-dihydropyridin-3-carboxamide

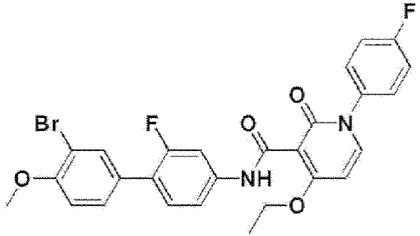

According to Step 5 of Example 1, by using 3'-bromo-2-fluoro-4'-methoxy(1,1'-biphenyl)-4-amine instead of 4-(4'-amino-4-(benzyloxy)-2'-fluoro-[1,1'-biphenyl]-3-yl)pyrimidin-2-amine obtained in Step 1 above and by a similar method as Example 1, the title compound (275 mg, yield: 71%) was obtained.
MS *m*/*z*: 555 [M], 557[M+2]

### Step 3. N-(3'-(2-aminopyrimidin-5-yl)-2-fluoro-4'-methoxy-[1,1'-biphenyl]-4-yl)-4-ethoxy-1-(4-fluorophenyl)-2-oxo-1,2-dihydropyridin-3-carboxamide

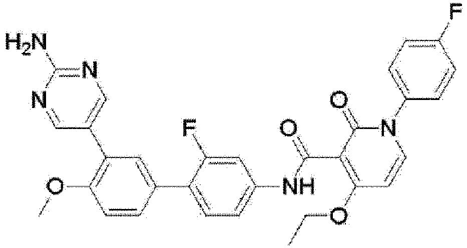

According to Step 4 of Example 1, by using N-(3'-bromo-2-fluoro-4'-methoxy-[1,1'-biphenyl]-4-yl)-4-ethoxy-1-(4-fluorophenyl)-2-oxo-1,2-dihydropyridin-3-carboxamide (40 mg, 0.07 mmol) obtained in Step 2 and (2-aminopyrimidin-5-yl)boronic acid (11 mg, 0,08 mmol) and by a similar method as Example 1, the title compound (10.9 mg, yield: 27%) was obtained.
¹H NMR (400 MHz, MeOH-d₄) δ 8.46 (s, 2H), 7.77 (m, 2H), 7.54 (d, 1H), 7.47-7.39 (m, 5H), 7.29 (m, 2H), 7.16 (d, 1H), 6.59 (d, 1H), 4.34 (q, 2H), 3.89 (s, 3H), 1.46(t, 3H); MS *m*/*z*: 570 [M+H]

### Example 18. N-(3'-(6-aminopyridin-3-yl)-2-fluoro-4'-methoxy-[1,1'-biphenyl]-4-yl)-4-ethoxy-1-(4-fluorophenyl)-2-oxo-1,2-dihydropyridin-3-carboxamide

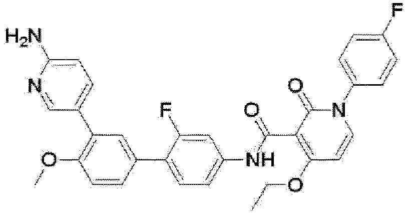

According to Step 3 of Example 17, by using (6-aminopyridin-3-yl)boronic acid instead of (2-aminopyrimidin-5-yl)boronic acid and by a similar method as Example 17, the title compound (22.1 mg, yield: 62%) was obtained.
¹H NMR (400 MHz, MeOH-d₄) δ 8.19 (s, 1H), 7.74 (m, 2H), 7.68 (d, 1H), 7.47-7.39 (m, 6H), 7.28 (m, 2H), 7.13 (d, 1H), 6.65 (d, 1H), 6.58 (d, 1H), 4.33 (q, 2H), 3.86 (s, 3H), 1.45(t, 3H);
MS *m*/*z:* 569[M+H]

### Example 19. N-(3'-(2-aminopyridin-4-yl)-2-fluoro-4'-methoxy-[1,1'-biphenyl]-4-yl)-4-ethoxy-1-(4-fluorophenyl)-2-oxo-1,2-dihydropyridin-3-carboxamide

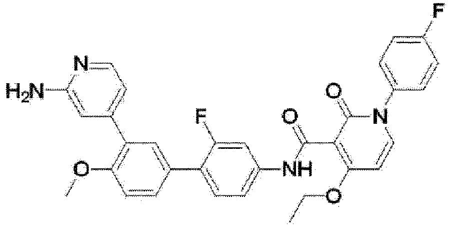

According to Step 3 of Example 17, by using (2-aminopyridin-4-yl)boronic acid instead of (2-aminopyrimidin-5-yl)boronic acid and by a similar method as Example 17, the title compound (5.4 mg, yield: 15%) was obtained.
¹H NMR (400 MHz, MeOH-d₄) δ 7.92 (m, 1H), 7.76 (m, 2H), 7.59 (d, 1H), 7.50-7.40 (m, 5H), 7.31 (m, 2H), 7.19 (d, 1H), 6.83 (m, 2H), 6.60 (d, 1H), 4.35 (q, 2H), 3.89 (s, 3H), 1.47(t, 3H);
MS *m*/*z*: 569[M+H]

### Example 20. N-(3'-(2-aminopyridin-3-yl)-2-fluoro-4'-methoxy-[1,1'-biphenyl]-4-yl)-4-ethoxy-1-(4-fluorophenyl)-2-oxo-1,2-dihydropyridin-3-carboxamide

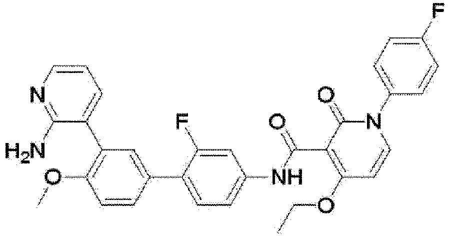

According to Step 3 of Example 17, by using 3-(4,4,5,5-tetramethyl-1,3,2-dioxaborolan-2-yl)pyridin-2-amine instead of (2-aminopyrimidin-5-yl)boronic acid and by a similar method as Example 17, the title compound (10.1 mg, yield: 28%) was obtained.
¹H NMR (400 MHz, MeOH-d₄) δ 7.98 (m, 1H), 7.80 (m, 2H), 7.62 (d, 1H), 746-7.39 (m, 6H), 7.30 (m, 2H), 7.21 (d, 1H), 6.77 (m, 1H), 6.59 (d, 1H), 4.34 (q, 2H), 3.87 (s, 3H), 1.46(t, 3H);
MS *m*/*z:* 569[M+H]

### Example 21. 4-Ethoxy-N-(2-fluoro-4'-methoxy-3'-(quinolin-6-yl)-[1,1'-biphenyl]-4-yl)-1-(4-fluorophenyl)-2-oxo-1,2-dihydropyridin-3-carboxamide

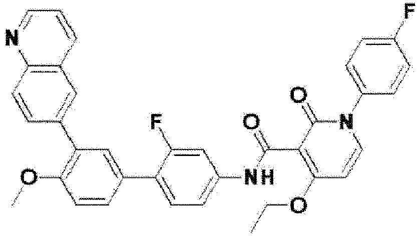

According to Step 3 of Example 17, by using quinolin-6-ylboronic acid instead of ((2-aminopyrimidin-5-yl)boronic acid and by a similar method as Example 17, the title compound (7.7 mg, yield: 20%) was obtained.
¹H NMR (400 MHz, MeOH-d₄) δ 8.84 (m, 1H), 8.42 (d, 1H), 8.06 (m, 2H), 7.98 (m, 1H), 7.78 (m, 2H), 7.61 (m, 3H), 7.48-7.38 (m, 4H), 7.30 (m, 2H), 7.23 (d, 1H), 6.60 (d, 1H), 4.35 (q, 2H), 3.90 (s, 3H), 1.46(t, 3H);
MS *m*/*z:* 604[M+H]

### Example 22. 4-Ethoxy-N-(2-fluoro-3'-(isoquinolin-6-yl)-4'-methoxy-[1,1'-biphenyl]-4-yl)-1-(4-fluorophenyl)-2-oxo-1,2-dihydropyridin-3-carboxamide

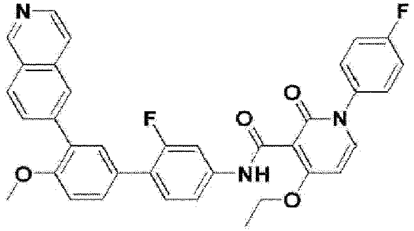

According to Step 3 of Example 17, by using isoquinolin-6-ylboronic acid instead of (2-aminopyrimidin-5-yl)boronic acid and by a similar method as Example 17, the title compound (4.8 mg, yield: 13%) was obtained.
¹H NMR (400 MHz, MeOH-d₄) δ 9.25 (s, 1H), 8.45 (m, 1H), 8.14 (d, 1H), 8.08 (s, 1H), 7.93 (m, 1H), 7.88 (m, 1H), 7.79 (m, 2H), 7.64 (m, 2H), 7.50-7.41 (m, 4H), 7.31 (m, 2H), 7.27 (m, 1H), 6.60 (d, 1H), 4.35 (q, 2H), 3.91 (s, 3H), 1.47(t, 3H);
MS *m*/*z:* 604[M+H]

### Example 23. 4-Ethoxy-N-(2-fluoro-4'-methoxy-3'-(1H-pyrrolo[2,3-b]pyridin-4-yl)-[1,1'-biphenyl]-4-yl)-1-(4-fluorophenyl)-2-oxo-1,2-dihydropyridin-3-carboxamide

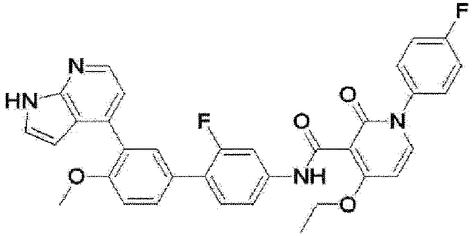

According to Step 13 of Example 17, by using 4-(4,4,5,5-tetramethyl-1,3,2-dioxaborolan-2yl)-1H-pyrrolo[2,3-b]pyridine instead of (2-aminopyrimidin-5-yl)boronic acid and by a similar method as Example 17, the title compound (7.3 mg, yield: 20%) was obtained.
¹H NMR (400 MHz, MeOH-d₄) δ 8.26 (m, 1H), 7.74 (m, 2H), 7.64 (m, 2H), 7.48-7.39 (m, 5H), 7.30 (m, 3H), 7.22 (m, 1H), 6.59 (d, 1H), 6.42 (d, 1H), 4.34 (q, 2H), 3.86 (s, 3H), 1.46(t, 3H);
MS *m*/*z:* 593[M+H]

### Example 24. N-(3'-bromo-2-fluoro-4'-hydroxy-[1,1'-biphenyl]-4-yl)-4-ethoxy-1-(4-fluorophenyl)-2-oxo-1,2-dihydropyridin-3-carboxamide

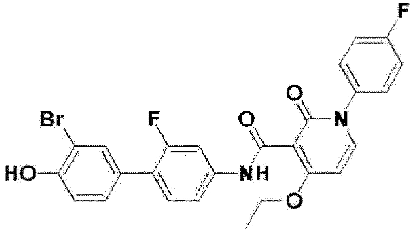

Hereinafter, the compound of Example 24 was prepared according to Reaction 7 below.

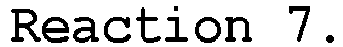

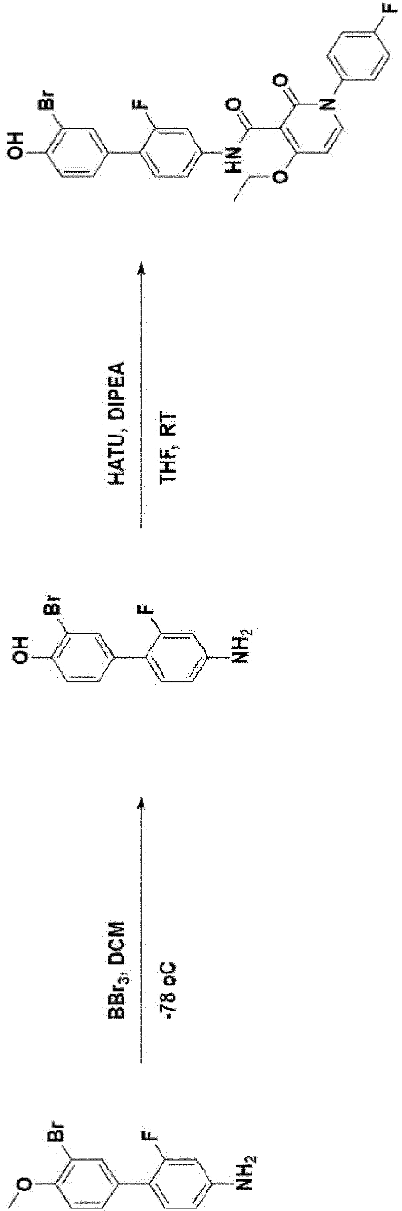

### Step 1. 4'-Amino-3-bromo-2'-fluoro-[1,1'-biphenyl]-4-ol

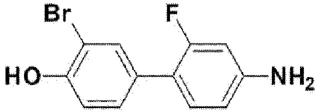

3'-Bromo-2-fluoro-4'-methoxy-[1,1'-biphenyl]-4-amine (220 mg, 0.74 mmol) was dissolved in 5 ml of DCM and then, stirred at -78°C under nitrogen. To this solution, 1 M BBr₃ (7.43 ml, 7.43 mmol) was slowly added. The solution mixture was stirred at -78°C for 15 minutes, and further stirred at room temperature for 4 hours. To the reaction mixture, 50 ml of water was added, and then, stirred at room temperature for 15 minutes. The mixture was extracted with DCM/MeOH (10:1), and an organic layer was washed with water and brine, and dried in vacuum. The residue was purified by silica gel chromatography to obtain the title compound (190 mg, yield: 91%) .
MS *m*/*z:* 282 [M], 284[M+2]

### Step 2. N-(3'-bromo-2-fluoro-4'-hydroxy-[1,1'-biphenyl]-4-yl)-4-ethoxy-1-(4-fluorophenyl)-2-oxo-1,2-dihydropyridin-3-carboxamide

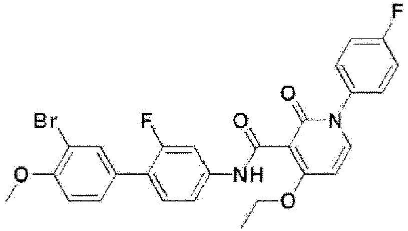

The compound obtained in Step 1 above of 4'-amino-3-bromo-2'-fluoro-[1,1'-biphenyl]-4-ol (100 mg, 0.35 mmol) was dissolved in 10 ml of THF, and 4-ethoxy-1-(4-fluorophenyl)-2-oxo-1,2-dihydropyridin-3-carboxylic acid (118 mg, 0.43 mmol), HATU (202 mg, 0.53 mmol), and DIPEA (124 µl, 0.71 mmol) were added thereto, followed by stirring at room temperature for 12 hours. The reaction solution was diluted with water, extracted with EA three times, and concentrated. The residue was purified by silica gel chromatography to obtain the title compound (180 mg, 94%).
¹H NMR (400 MHz, CDCl₃) δ 11.58 (s, 1H), 7.86 (d, 1H), 7.68 (s, 1H), 7.52 (d, 1H), 7.44 (m, 3H), 7.31-7.28 (m, 3H), 7.08 (d, 1H), 6.36 (d, 1H), 4.36 (q, 2H), 3.89 (s, 3H), 1.52(t, 3H);
MS *m*/*z:* 541 [M], 543[M+2]

### Example 25. N-(3'-(2-aminopyrimidin-5-yl)-2-fluoro-4'-hydroxy-[1,1'-biphenyl]-4-yl)-4-ethoxy-1-(4-fluorophenyl)-2-oxo-1,2-dihydropyridin-3-carboxamide

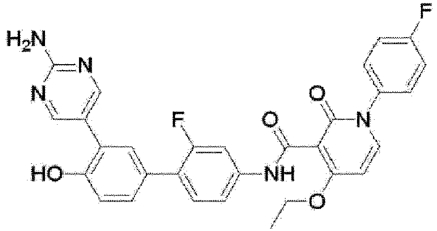

Hereinafter, the compound of Example 25 was prepared according to Reaction 8 below.

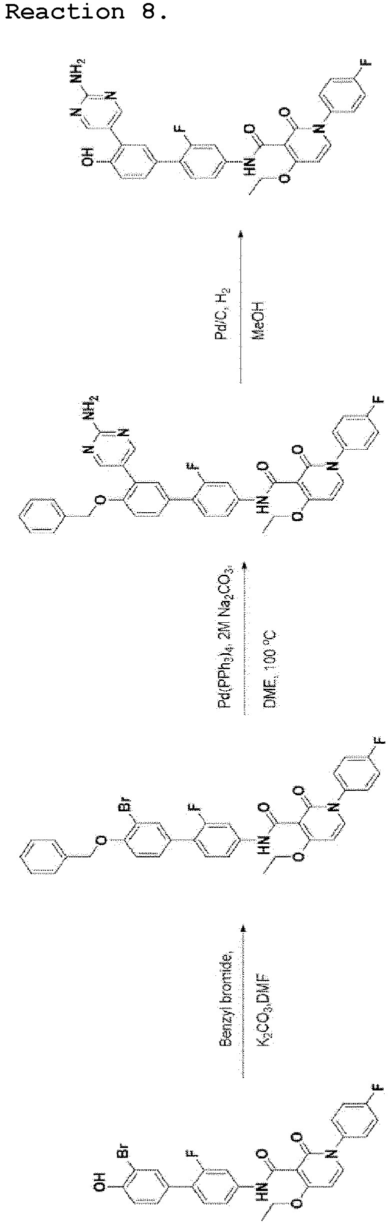

### Step 1. N-(4'-(benzyloxy)-3'-bromo-2-fluoro-[1,1'-biphenyl]-4-yl)-4-ethoxy-1-(4-fluorophenyl)-2-oxo-1,2-dihydropyridin-3-carboxamide

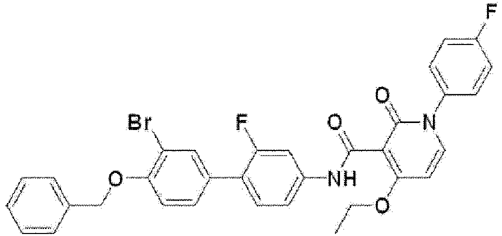

According to Step 1 of Example 1, by using the compound obtained in Example 24 of N-(3'-bromo-2-fluoro-4'-hydroxy-[1,1'-biphenyl]-4-yl)-4-ethoxy-1-(4-fluorophenyl)-2-oxo-1,2-dihydropyridin-3-carboxamide (180 mg, 0.33 mmol), benzyl bromide (47 ul, 0,40 mmol), and K₂CO₃ (69 mg, 0.50 mmol) and by a similar method, the title compound (200 mg, yield: 95%) was obtained.
MS *m*/*z:* 631[M+H]

### Step 2. N-(3'-(2-aminopyrimidin-5-yl)-4'-(benzyloxy)-2-fluoro-[1,1'-biphenyl]-4-yl)-4-ethoxy-1-(4-fluorophenyl)-2-oxo-1,2-dihydropyridin-3-carboxamide

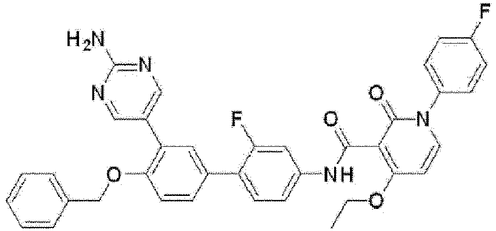

According to Step 4 of Reaction 1, by using the compound obtained in Step 1 above of N-(4'-(benzyloxy)-3'-bromo-2-fluoro-[1,1'-biphenyl]-4-yl)-4-ethoxy-1-(4-fluorophenyl)-2-oxo-1,2-dihydropyridin-3-carboxamide (50 mg, 0.08 mmol) and (2-aminopyrimidin-5-yl)boronic acid (17 mg, 0,12 mmol) and by a similar method, the title compound (21.0 mg, yield: 41%) was obtained.
MS *m*/*z:* 646[M+H]

### Step 3. N-(3'-(2-aminopyrimidin-5-yl)-2-fluoro-4'-hydroxy-[1,1'-biphenyl]-4-yl)-4-ethoxy-1-(4-fluorophenyl)-2-oxo-1,2-dihydropyridin-3-carboxamide

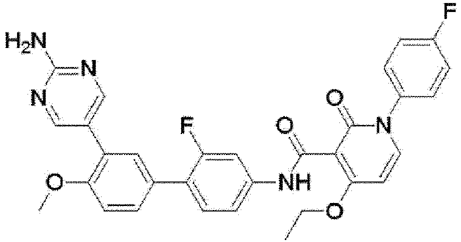

According to Step 6 of Example 1, by using the compound obtained in Step 2 above of N-(3'-(2-aminopyrimidin-5-yl)-4'-(benzyloxy)-2-fluoro-[1,1'-biphenyl]-4-yl)-4-ethoxy-1-(4-fluorophenyl)-2-oxo-1,2-dihydropyridin-3-carboxamide (21 mg, 0.03 mmol) and by a similar method as Example 1, the title compound (9.3 mg, yield: 52%) was obtained.
¹H NMR (400 MHz, MeOH-d₄) δ 8.56 (s, 2H), 7.68 (m, 2H), 7.44 (m, 4H), 7.36 (m, 2H), 7.28 (m, 2H), 7.06 (d, 1H), 6.58 (d, 1H), 4.31 (q, 2H), 1.42(t, 3H);
MS *m*/*z:* 556[M+H]

### Example 26. N-(3'-(2-aminopyridin-4-yl)-2-fluoro-4'-hydroxy-[1,1'-biphenyl]-4-yl)-4-ethoxy-1-(4-fluorophenyl)-2-oxo-1,2-dihydropyridin-3-carboxamide

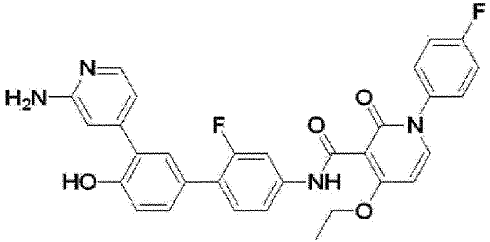

In Step 2 of Example 25, by using (2-aminopyridin-4-yl)boronic acid instead of (2-aminopyrimidin-5-yl)boronic acid and by a similar method as Example 25, the title compound (1.5 mg, yield: 16%) was obtained.
¹H NMR (400 MHz, MeOH-d₄) δ 7.94 (m, 1H), 7.79 (m, 1H), 7.76 (m, 1H), 7.51-7.39 (m, 5H), 7.30 (m, 2H), 7.02 (m, 1H), 6.88 (m, 2H), 6.62 (d, 1H), 4.36 (q, 2H), 1.48(t, 3H);
MS *m*/*z*: 555[M+H]

### Example 27. 4-Ethoxy-N-(2-fluoro-4'-hydroxy-3'-(1-methyl-1H-pyrazol-4-yl)-[1,1'-biphenyl]-4-yl)-1-(4-fluorophenyl)-2-oxo-1,2-dihydropyridin-3-carboxamide

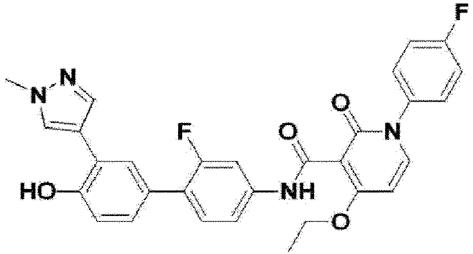

In Step 2 of Example 25, by using 1-methyl-4-(4,4,5,5-tetramethyl-1,3,2-dioxaborolan-2-yl)-1H-pyrazole instead of (2-aminopyrimidin-5-yl)boronic acid and by a similar method as Example 25, the title compound (3.7 mg, yield: 43%) was obtained.
¹H NMR (400 MHz, MeOH-d₄) δ 8.12 (s, 1H), 7.94 (s, 1H), 7.80 (d, 1H), 7.74 (m, 2H), 7.47 (m, 3H), 7.43 (m, 1H), 7.31-7.25 (m, 3H), 6.95 (d, 1H), 6.61 (m, 1H), 4.36 (q, 2H), 3.95 (s, 3H), 1.47(t, 3H);
MS *m*/*z:* 543[M+H]

### Example 28. N-(4-((3-(2-aminopyrimidin-4-yl)-4-hydroxyphenyl)amino)-3-fluorophenyl)-4-ethoxy-1-(4-fluorophenyl)-2-oxo-1,2-dihydropyridin-3-carboxamide

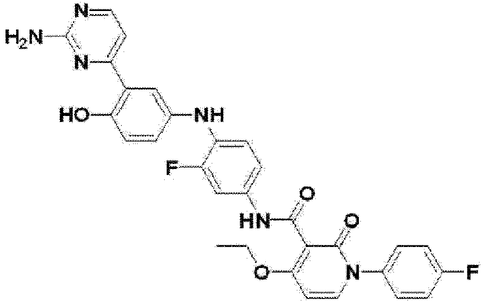

Hereinafter, the compound of Example 28 was prepared according to Reaction 9 below.

### Reaction 9.

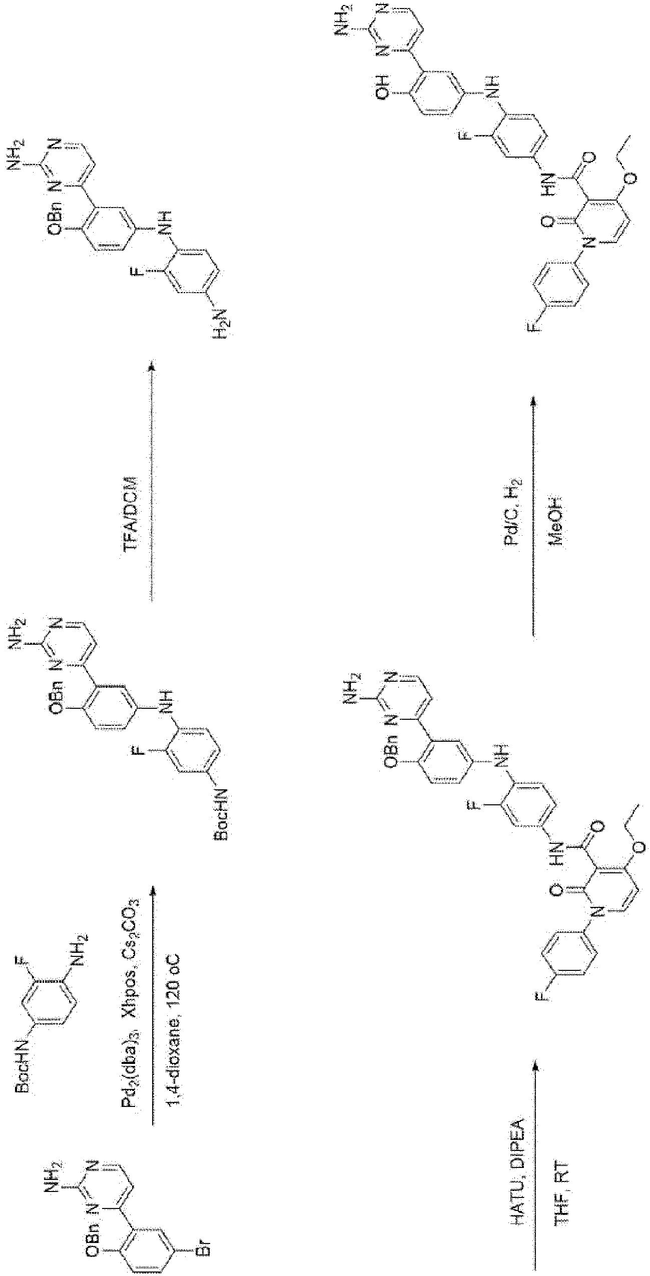

### Step 1. tert-butyl(4-((3-(2-aminopyrimidin-4-yl)-4-(benzyloxy)phenyl)amino)-3-fluorophenyl)carbamate

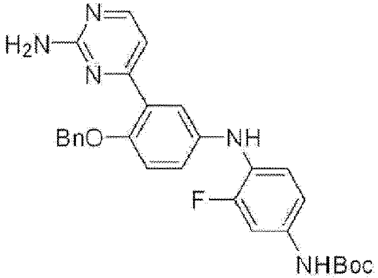

4-(2-(Benzyloxy)-5-bromophenyl)pyrimidin-2-amine (100 mg, 0.28 mmol) was dissolved in 5 ml of 1,4-dioxane, and tert-butyl(4-amino-3-fluorophenyl)carbamate (76 mg, 0.34 mmol), Cs₂CO₃ (183 mg, 0.56 mmol), Pd₂(dba)₃ (13 mg, 0.01 mmol), and Xphos (13.4 mg, 0.02 mmol) were added thereto, followed by stirring at 130°C for 2 hours. The solution was cooled to room temperature, a catalyst was filtered through celite, and the filtrate was dried in vacuum. The residue was purified by silica gel chromatography to obtain the title compound (105 mg, yield: 75%).
MS *m*/*z:* 502[M+H]

### Step 2. N1-(3-(2-aminopyrimidin-4-yl)-4-(benzyloxy)phenyl)-2-fluorobenzene-1,4-diamine

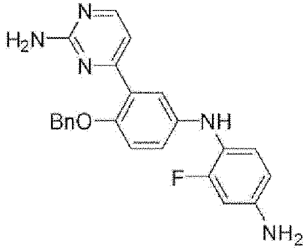

The compound obtained in Step 1 above of tert-butyl(4-((3-(2-aminopyrimidin-4-yl)-4-(benzyloxy)phenyl)amino)-3-fluorophenyl)carbamate (100 mg, 0.20 mmol) was dissolved in 3 ml of DCM, and 3 ml of TFA was added thereto. The mixture was stirred at room temperature for 2 hours and concentrated. The residue was dissolved in DCM and concentrated twice further, and then, the residue was diluted with water and EA, and extracted with EA three times. An organic layer was concentrated to obtain the title compound (60 mg, yield: 79%).
MS *m*/*z:* 402[M+H]

### Step 3. N-(4-((3-(2-aminopyrimidin-4-yl)-4-(benzyloxy)phenyl)amino)-3-fluorophenyl)-4-ethoxy-1-(4-fluorophenyl)-2-oxo-1,2-dihydropyridin-3-carboxamide

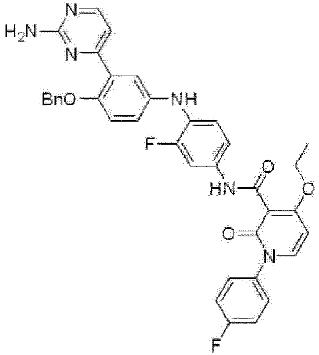

According to Step 5 of Example 1, by using the compound obtained in Step 2 above of N1-(3-(2-aminopyrimidin-4-yl)-4-(benzyloxy)phenyl)-2-fluorobenzene-1,4-diamine (23 mg, 0.06 mmol) and 4-ethoxy-1-(4-fluorophenyl)-2-oxo-1,2-dihydropyridin-3-carboxylic acid (43 mg, 0.16 mmol) and by a similar method, the title compound (20 mg, yield: 51%) was obtained.
MS *m*/*z:* 661[M+H]

### Step 4. N-(4-((3-(2-aminopyrimidin-4-yl)-4-hydroxyphenyl)amino)-3-fluorophenyl)-4-ethoxy-1-(4-fluorophenyl)-2-oxo-1,2-dihydropyridin-3-carboxamide

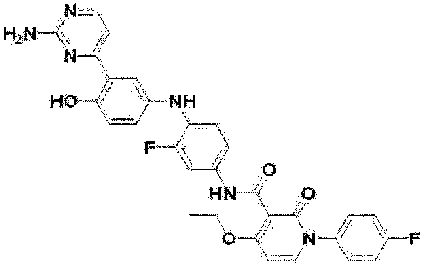

According to Step 6 of Example 1, by using the compound obtained in Step 3 above of N-(4-((3-(2-aminopyrimidin-4-yl)-4-(benzyloxy)phenyl)amino)-3-fluorophenyl)-4-ethoxy-1-(4-fluorophenyl)-2-oxo-1,2-dihydropyridin-3-carboxamide and by a similar method as Example 1, the title compound (6.9 mg, yield: 40%) was obtained.
¹H NMR (400 MHz, MeOH-d₄) δ 8.32 (d, 1H), 7.76 (d, 1H), 7.64 (m, 1H), 7.56 (m, 1H), 7.44 (m, 2H), 7.30 (m, 2H), 7.26 (m, 2H), 7.18 (d, 1H), 7.06, (m, 1H), 6.92 (d, 1H), 6.58 (d, 1H), 4.33 (q, 2H), 1.46(t, 3H);
MS *m*/*z:* 571[M+H]

### Example 29. N-(4-((3-(2-aminopyrimidin-4-yl)-4-hydroxyphenyl)amino)-3-fluorophenyl)-1-(4-fluorophenyl)-4-methoxy-2-oxo-1,2-dihydropyridin-3-carboxamide

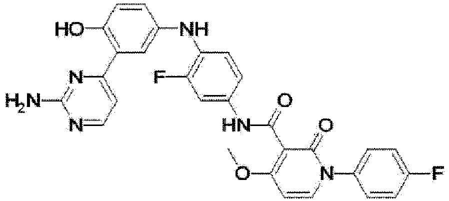

In Step 3 of Example 28, by using 1-(4-fluorophenyl)-4-methoxy-2-oxo-1,2-dihydropyridin-3-carboxylic acid instead of 4-ethoxy-1-(4-fluorophenyl)-2-oxo-1,2-dihydropyridin-3-carboxylic acid and by a similar method as Example 28, the title compound (20.7 mg, yield: 44%) was obtained.
¹H NMR (400 MHz, MeOH-d₄) δ 8.26 (d, 1H), 7.78 (d, 1H), 7.65 (m, 1H), 7.57 (m, 1H), 7.45 (m, 2H), 7.29 (m, 2H), 7.18 (m, 2H), 7.15 (m, 1H), 7.09, (m, 1H), 6.91 (d, 1H), 6.60 (d, 1H), 4.01 (s, 3H);
MS *m*/*z:* 557[M+H]

### Example 30. N-(4-((3-(2-aminopyrimidin-4-yl)-4-hydroxyphenyl)amino)-3-fluorophenyl)-1-(4-fluorophenyl)-6-methyl-2-oxo-1,2-dihydropyridin-3-carboxamide

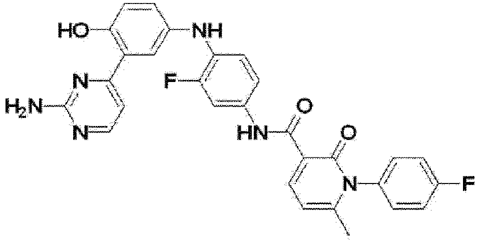

In Step 3 of Example 28, by using 1-(4-fluorophenyl)-6-methyl-2-oxo-1,2-dihydropyridin-3-carboxylic acid instead of 4-ethoxy-1-(4-fluorophenyl)-2-oxo-1,2-dihydropyridin-3-carboxylic acid and by a similar method as Example 28, the title compound (2.5 mg, yield: 8%) was obtained.
¹H NMR (400 MHz, MeOH-d₄) δ 8.56 (d, 1H), 8.32 (m, 1H), 7.72 (d, 1H), 7.58 (m, 1H), 7.39 (m, 4H), 7.19 (m, 1H), 7.12 (m, 2H), 7.08 (m, 1H), 6.91 (d, 1H), 6.76 (d, 1H), 2.15 (s, 3H);
MS *m*/*z:* 541[M+H]

### Example 31. N-(4-((3-(2-aminopyrimidin-4-yl)-4-hydroxyphenyl)amino)-3-fluorophenyl)-N-(4-fluorophenyl)cyclopropan-1,1-dicarboxamide

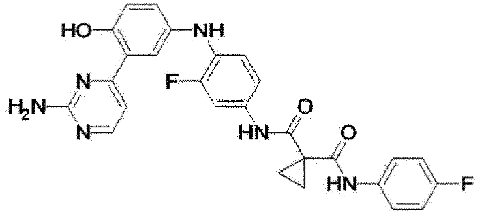

In Step 3 of Example 28, by using 1-((4-fluorophenyl)carbamoyl)cyclopropan-1-carboxylic acid instead of 4-ethoxy-1-(4-fluorophenyl)-2-oxo-1,2-dihydropyridin-3-carboxylic acid and by a similar method as Example 28, the title compound (1.2 mg, yield: 5%) was obtained.
¹H NMR (400 MHz, MeOD-d₄) δ 8.34 (m, 1H), 7.59 (m, 3H), 7.57 (m, 1H), 7.20 (m, 1H), 7.13-7.01 (m, 5H), 6.92 (d, 1H), 2.28 (s, 3H), 1.62(m, 4H);
MS *m*/*z:* 517[M+H]

### Example 32. N-(4-((3-(2-aminopyrimidin-4-yl)-4-hydroxyphenyl)amino)-3-fluorophenyl)-1,5-dimethyl-3-oxo-2-phenyl-2,3-dihydro-1H-pyrazol-4-carboxamide

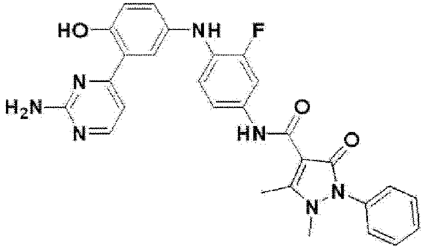

In Step 3 of Example 28, by using 1,5-dimethyl-3-oxo-2-phenyl-2,3-dihydro-1H-pyrazol-4-carboxylic acid instead of 4-ethoxy-1-(4-fluorophenyl)-2-oxo-1,2-dihydropyridin-3-carboxylic acid and by a similar method as Example 28, the title compound (30 mg, yield: 36%) was obtained.
¹H NMR (400 MHz, DMSO-d₆) δ 13.27 (s, 1H), 10.67 (s, 1H), 8.35 (m, 1H), 7.75 (d, 1H), 7.61 (m, 2H), 7.53 (m, 3H), 7.43 (m, 2H), 7.13 (m, 3H), 7.08 (m, 3H), 6.86 (d, 1H), 3.35 (s, 3H), 2.70 (s, 3H);
MS *m*/*z:* 526[M+H]

### Example 33. N-(4-((3-(2-aminopyrimidin-4-yl)-4-hydroxyphenyl)amino)-3-fluorophenyl)-1-phenyl-5-(trifluoromethyl)-1H-imidazol-4-carboxamide

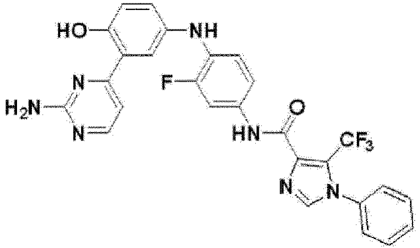

In Step 3 of Example 28, by using 1-phenyl-5-(trifluoromethyl)-1H-imidazol-4-carboxylic acid instead of 4-ethoxy-1-(4-fluorophenyl)-2-oxo-1,2-dihydropyridin-3-carboxylic acid and by a similar method as Example 28, the title compound (46 mg, yield: 52%) was obtained.
¹H NMR (400 MHz, DMSO-d₆) δ 13.30 (s, 1H), 10.42 (s, 1H), 8.37 (m, 2H), 7.80 (m, 1H), 7.63 (m, 7H), 7.48 (m, 1H), 7.13 (m, 5H), 6.88 (m, 1H);
MS *m*/*z:* 550[M+H]

### Example 34. N-(4-((3-(2-aminopyrimidin-4-yl)-4-hydroxyphenyl)amino)-2-fluorophenyl)-4-ethoxy-1-(4-fluorophenyl)-2-oxo-1,2-dihydropyridin-3-carboxamide

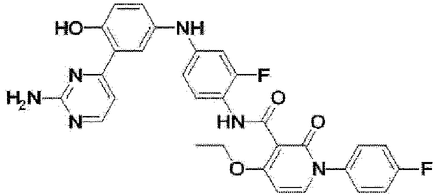

Hereinafter, the compound of Example 34 was prepared according to Reaction 10 below.

### Reaction 10.

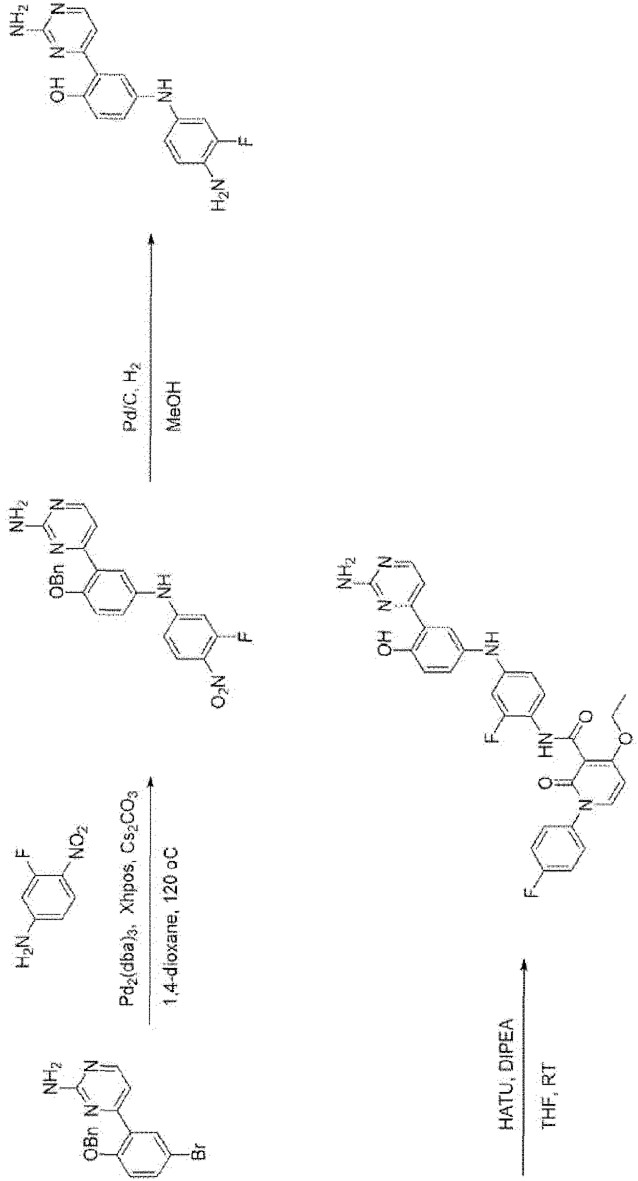

### Step 1. 4-(2-(Benzyloxy)-5-((3-fluoro-4-nitrophenyl)amino)phenyl)pyrimidin-2-amine

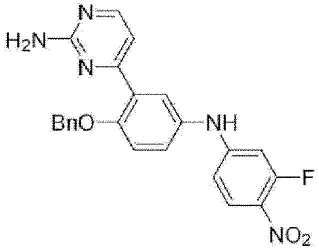

In Step 2 of Example 28, by using 3-fluoro-4-nitroaniline instead of tert-butyl(4-amino-3-fluorophenyl)carbamate and by a similar method as Example 28, the title compound (390 mg, yield: 72%) was obtained.
MS *m*/*z:* 432[M+H]

### Step 2. 4-((4-Amino-3-fluorophenyl)amino)-2-(2-aminopyrimidin-4-yl)phenol

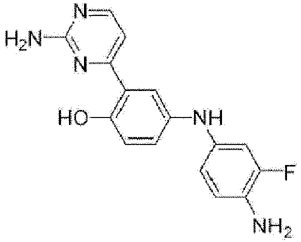

The compound obtained in Step 1 above of 4-(2-(benzyloxy)-5-((3-fluoro-4-nitrophenyl)amino)phenyl)pyrimidin-2-amine (390 mg, 0.90 mmol) was dissolved in 10 ml of methanol, and 10% Pd/C was added thereto, followed by stirring under a hydrogen atmosphere. A catalyst was filtered through celite, and the filtrate was dried in vacuum. The residue was purified by silica gel chromatography to obtain the title compound (200 mg, yield: 710) .
MS *m*/*z:* 312[M+H]

### Step 3. N-(4-((3-(2-aminopyrimidin-4-yl)-4-hydroxyphenyl)amino)-2-fluorophenyl)-4-ethoxy-1-(4-fluorophenyl)-2-oxo-1,2-dihydropyridin-3-carboxamide

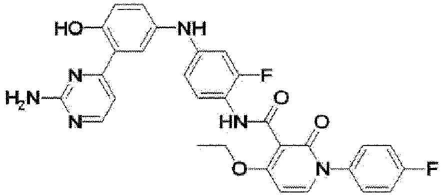

According to Step 5 of Example 1, by using the compound obtained in Step 2 above of 4-((4-amino-3-fluorophenyl)amino)-2-(2-aminopyrimidin-4-yl)phenol (50 mg, 0.16 mmol) and 4-ethoxy-1-(4-fluorophenyl)-2-oxo-1,2-dihydropyridin-3-carboxylic acid (53 mg, 0.19 mmol) and by a similar method as Example 1, the title compound (36.3 mg, yield: 40%) was obtained.
¹H NMR (400 MHz, DMSO-d₆) δ 10.12 (s, 1H), 8.35 (m 1H), 7.98 (s, 1H), 7.84 (d, 1H), 7.73 (m, 1H), 7.60 (m, 1H), 7.47 (m, 2H), 7.38 (m, 2H), 7.26 (m, 4H), 6.90, (d, 1H), 6.69 (m, 2H), 6.49 (d, 1H), 4.26 (q, 2H), 1.34(t, 3H);
MS *m*/*z:* 571[M+H]

### Example 35. N-(4-((3-(2-aminopyrimidin-4-yl)-4-hydroxyphenyl)amino)phenyl)-4-ethoxy-1-(4-fluorophenyl)-2-oxo-1,2-dihydropyridin-3-carboxamide

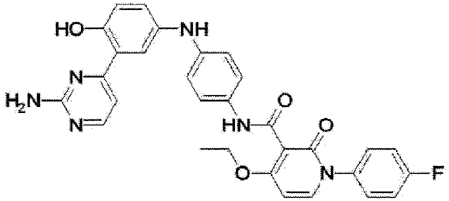

In Step 1 of Example 28, by using benzene-1,4-diamine instead of tert-butyl(4-amino-3-fluorophenyl)carbamate and by a similar method as Example 28, the title compound (8.2 mg, yield: 24%) was obtained.
¹H NMR (400 MHz, MeOD-d₄) δ 8.28 (m 1H), 7.73 (m, 1H), 7.59 (m, 1H), 7.49 (d, 2H), 7.45 (m, 2H), 7.29 (m, 2H), 7.20 (m, 1H), 7.10 (m, 1H), 6.95, (d, 1H), 6.90 (m, 2H), 6.57 (d, 1H), 4.33 (q, 2H), 1.46(t, 3H);
MS *m*/*z:* 553[M+H]

### Example 36. N-(4-(3-acetyl-4-hydroxyphenoxy)-3-fluorophenyl)-4-ethoxy-1-(4-fluorophenyl)-2-oxo-1,2-dihydropyridin-3-carboxamide

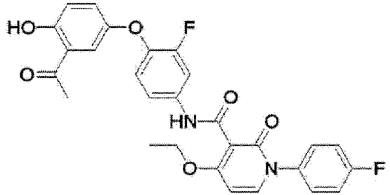

Hereinafter, the compound of Example 36 was prepared according to Reaction 11 below.

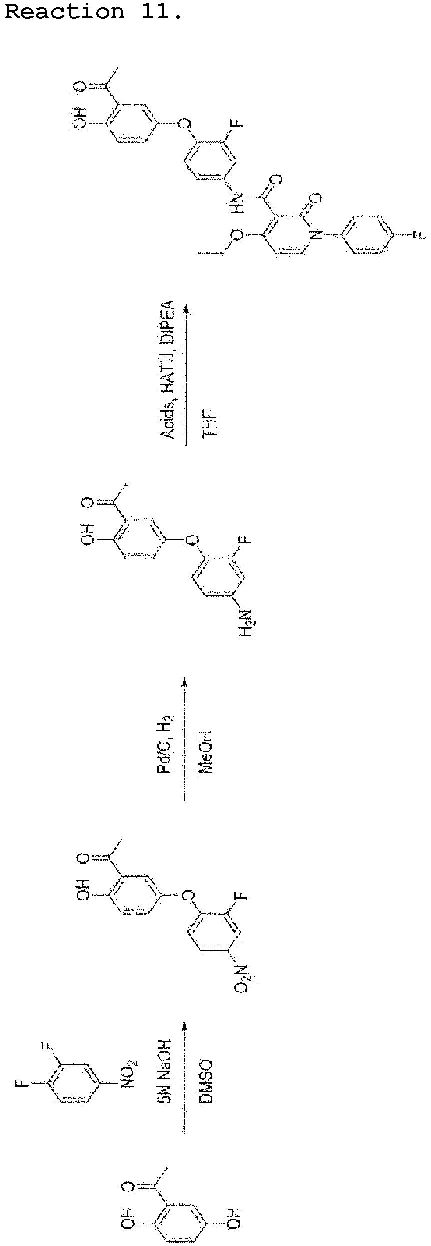

### Step 1. 1-(5-(2-Fluoro-4-nitrophenoxy)-2-hydroxyphenyl)ethan-1-one

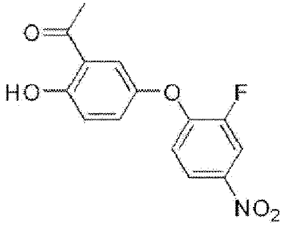

To a reaction vessel containing 12 ml of DMSO, 1.5 ml of 5 N NaOH was put and stirred at room temperature for 10 minutes, and 1-(2,5-dihydroxyphenyl)ethan-1-one (459 mg, 3.02 mmol) was added thereto, followed by further stirring for 30 minutes. 1,2-difluoro-4-nitrobenzene was slowly added thereto, followed by stirring at room temperature for 5 hours. The resultant was acidified using 5 N HCl, extracted with EA three times, and then, concentrated. The residue was purified by silica gel chromatography to obtain the title compound (600 mg, yield: 82%).
MS *m*/*z:* 292[M+H]

### Step 2. 1-(5-(4-Amino-2-fluorophenoxy)-2-hydroxyphenyl)ethan-1-one

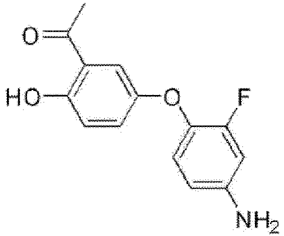

The compound obtained in Step 1 above of 1-(5-(2-fluoro-4-nitrophenoxy)-2-hydroxyphenyl)ethan-1-one (100 mg, 0.34 mmol) was dissolved in 7 ml of MeOH, and 10% Pd/C was added thereto, followed by stirring under a hydrogen atmosphere. A catalyst was filtered through celite, and the filtrate was dried in vacuum. The residue was purified by silica gel chromatography to obtain the title compound (80 mg, yield: 89%) .
MS *m*/*z:* 262[M+H]

### Step 3. N-(4-(3-acetyl-4-hydroxyphenoxy)-3-fluorophenyl)-4-ethoxy-1-(4-fluorophenyl)-2-oxo-1,2-dihydropyridin-3-carboxamide

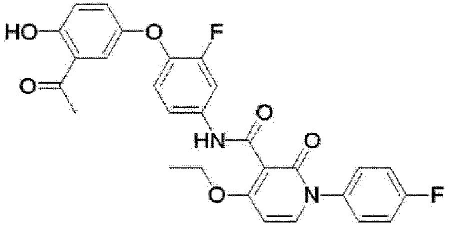

According to Step 5 of Example 1, by using the compound obtained in Step 2 above of 1-(5-(4-amino-2-fluorophenoxy)-2-hydroxyphenyl)ethan-1-one (40 mg, 0.15 mmol) and 4-ethoxy-1-(4-fluorophenyl)-2-oxo-1,2-dihydropyridin-3-carboxylic acid (51 mg, 0.18 mmol) and by a similar method as Example 1, the title compound (20.0 mg, yield: 25%) was obtained.
¹H NMR (400 MHz, DMSO-d₆) δ 11.5 (s, 1H), 10.45 (s, 1H), 7.85 (m, 2H), 7.46 (m, 3H), 7.39 (m, 3H), 7.22 (m, 1H), 7.10 (m, 1H), 6.98 (m, 1H), 6.51 (d, 1H), 4.27 (q, 2H), 2.60 (s, 3H), 1.30(t, 3H);
MS *m*/*z:* 521[M+H]

### Example 37. N-(4-(3-acetyl-4-hydroxyphenoxy)-3-fluorophenyl)-1-(4-fluorophenyl)-6-methyl-2-oxo-1,2-dihydropyridin-3-carboxamide

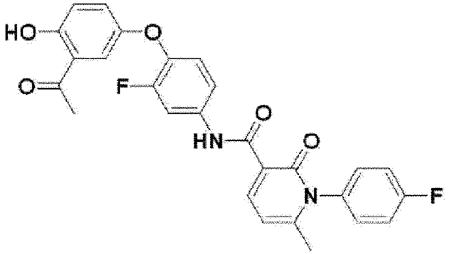

In Step 3 of Example 36, by using 1-(4-fluorophenyl)-6-methyl-2-oxo-1,2-dihydropyridin-3-carboxylic acid instead of 4-ethoxy-1-(4-fluorophenyl)-2-oxo-1,2-dihydropyridin-3-carboxylic acid and by a similar method as Example 36, the title compound (50 mg, yield: 59%) was obtained.
¹H NMR (400 MHz, DMSO-d₆) δ 11.97 (s, 1H), 8.49 (m, 1H), 7.96 (d, 1H), 7.50-7.42 (m, 5H), 7.34 (m, 1H), 7.23 (m, 1H), 7.06 (t, 1H), 6.99 (m, 1H), 6.71 (d, 1H), 2.60 (s, 3H), 2.07 (s, 3H);
MS *m*/*z:* 490[M+H]

### Example 38. (E)-N-(3-fluoro-4-(4-hydroxy-3-(1-(hydroxyimino)ethyl)phenoxy)phenyl)-1-(4-fluorophenyl)-6-methyl-2-oxo-1,2-dihydropyridin-3-carboxamide

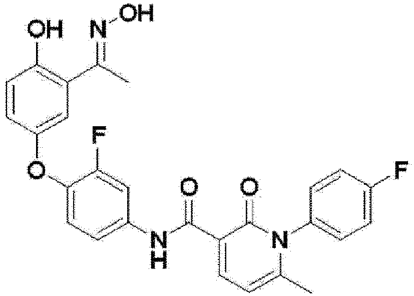

Hereinafter, the compound of Example 38 was prepared according to Reaction 12 below.

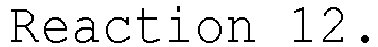

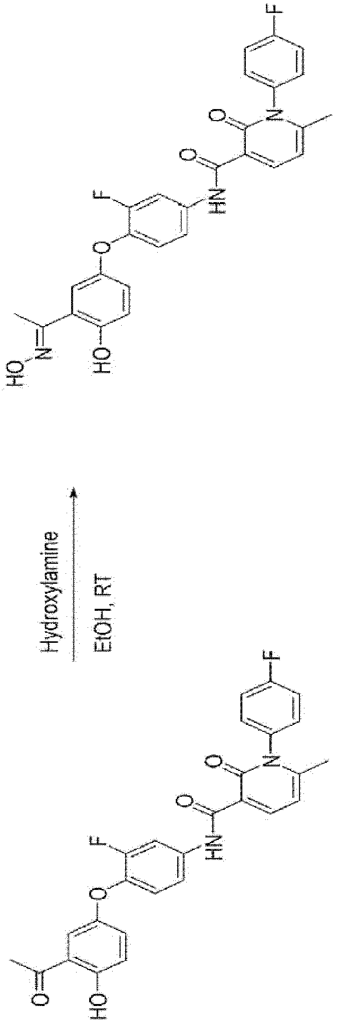

To the compound obtained in Example 37, N-(4-(3-acetyl-4-hydroxyphenoxy)-3-fluorophenyl)-1-(4-fluorophenyl)-6-methyl-2-oxo-1,2-dihydropyridin-3-carboxamide (20 mg, 0.04 mmol) was dissolved in 2 ml of EtOH, and hydroxylamine HCl (2.83 mg, 0.04 mmol) dissolved in 0.2 ml of water was added, followed by stirring at 50°C for 12 hours. The reaction mixture was cooled to room temperature, and the reaction solution was diluted with water, extracted with EA three times, and concentrated. The residue was purified by silica gel chromatography to obtain the title compound (14 mg, yield: 68%) .
¹H NMR (400 MHz, DMSO-d₆) δ 11.94 (s, 1H), 11.60 (s, 1H), 11.26 (brs, 1H), 8.48 (m, 1H), 7.95 (d, 1H), 7.50-7.41 (m, 4H), 7.32 (m, 1H), 7.16 (m, 1H), 7.01 (t, 1H), 6.92 (m, 2H), 6.71 (d, 1H), 2.19 (s, 3H), 2.07 (s, 3H);
MS *m*/*z:* 506[M+H]

### Example 39. N-(4-(3-(2-aminopyrimidin-4-yl)-4-hydroxyphenoxy)-3-fluorophenyl)-4-ethoxy-1-(4-fluorophenyl)-2-oxo-1,2-dihydropyridin-3-carboxamide

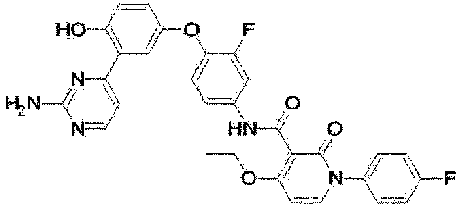

Hereinafter, the compound of Example 39 was prepared according to Reaction 13 below.

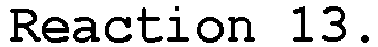

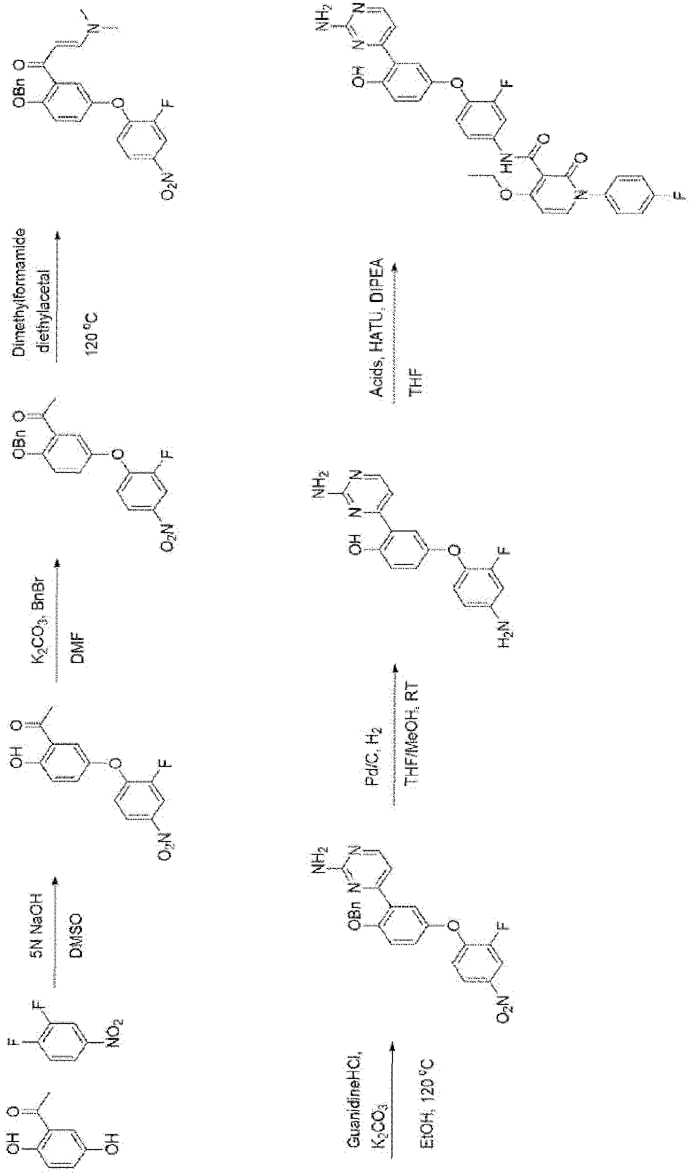

### Step 1. 1-(2-(Benzyloxy)-5-(2-fluoro-4-nitrophenoxy)phenyl)ethan-1-one

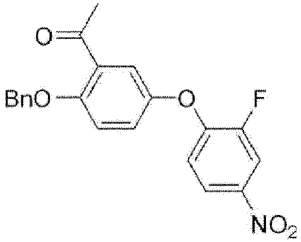

To the DMF solution of the compound obtained in Step 1 of Example 36 of 1-(5-(2-fluoro-4-nitrophenoxy)-2-hydroxyphenyl)ethan-1-one (8 g, 27.4 mmol), K₂CO₃ (7.6 g, 54.8 mmol) and benzyl bromide (7 g, 41.1 mmol) were added, followed by stirring at room temperature for 12 hours. The reaction solution was diluted with water and extracted with EA three times, and an organic layer was washed with brine and concentrated. The residue was purified by silica gel chromatography to obtain the title compound (10.1 g, yield: 96%) .
MS *m*/*z:* 382[M+H]

### Step 2. (E)-1-(2-(benzyloxy)-5-(2-fluoro-4-nitrophenoxy)phenyl)-3-(dimethylamino)prop-2-en-1-one

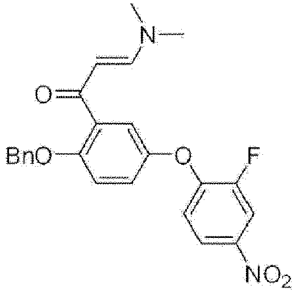

According to Step 2 of Example 1, by using the compound obtained in Step 1 above of 1-(2-(benzyloxy)-5-(2-fluoro-4-nitrophenoxy)phenyl)ethan-1-one (10 g, 26.2 mmol) instead of 1-(2-(benzyloxy)-5-bromophenyl)ethan-1-one, the title compound (11 g, yield: 96%) was obtained.
MS *m*/*z:* 437[M+H]

### Step 3. 4-(2-(Benzyloxy)-5-(2-fluoro-4-nitrophenoxy)phenyl)pyrimidin-2-amine

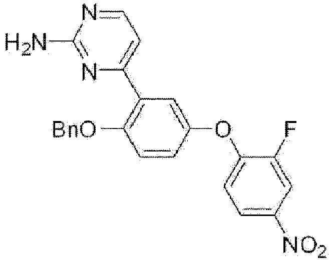

According to Step 3 of Example 1, by using the compound obtained in Step 2 above of (E)-1-(2-(benzyloxy)-5-(2-fluoro-4-nitrophenoxy)phenyl)-3-(dimethylamino)prop-2-en-1-one (11 g, 25.2 mmol) instead of (E)-1-(2-(benzyloxy)-5-bromophenyl)-3-(dimethylamino)prop-2-en-1-one and by a similar method, the title compound (8.3 g, yield: 76%) was obtained.
MS *m*/*z:* 433[M+H]

### Step 4. 4-(5-(4-Amino-2-fluorophenoxy)-2-(benzyloxy)phenyl)pyrimidin-2-amine

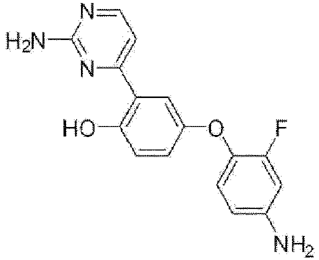

According to Step 4 of Example 1, by using the compound obtained in Step 3 above of 4-(2-(benzyloxy)-5-(2-fluoro-4-nitrophenoxy)phenyl)pyrimidin-2-amine (6g, 13.8 mmol) instead of 4-(2-(benzyloxy)-5-bromophenyl)pyrimidin-2-amine and by a similar method, the title compound (3.6 g, yield: 65%) was obtained.
MS *m*/*z:* 313[M+H]

### Step 5. N-(4-(3-(2-aminopyrimidin-4-yl)-4-hydroxyphenoxy)-3-fluorophenyl)-4-ethoxy-1-(4-fluorophenyl)-2-oxo-1,2-dihydropyridin-3-carboxamide

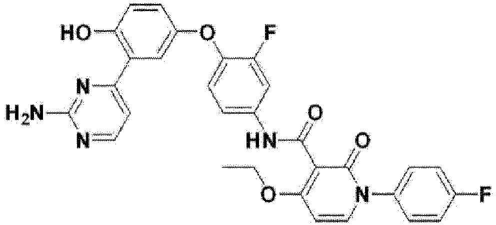

According to Step 5 of Example 1, by using the compound obtained in Step 4 above of 4-(5-(4-amino-2-fluorophenoxy)-2-(benzyloxy)phenyl)pyrimidin-2-amine (40 mg, 0.13 mmol) and 4-ethoxy-1-(4-fluorophenyl)-2-oxo-1,2-dihydropyridin-3-carboxylic acid (42 mg, 0.16 mmol) and by a similar method as Example 1, the title compound (40.4 mg, yield: 55%) was obtained.
¹H NMR (400 MHz, DMSO-d₆) δ 13.56 (s, 1H), 10.43 (s, 1H), 8.35 (d, 1H), 7.85 (m, 2H), 7.63 (m, 1H), 7.46 (m, 2H), 7.38 (m, 3H), 7.20 (m, 3H), 7.06 (m, 2H), 6.92 (m, 1H), 6.51 (d, 1H), 4.26 (q, 2H), 1.30(t, 3H);
MS *m*/*z:* 572[M+H]

### Example 40. N-(4-(3-(2-aminopyrimidin-4-yl)-4-hydroxyphenoxy)-3-fluorophenyl)-1-(4-fluorophenyl)-6-methyl-2-oxo-1,2-dihydropyridin-3-carboxamide

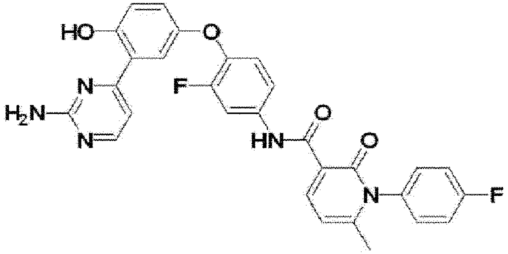

By using 1-(4-fluorophenyl)-6-methyl-2-oxo-1,2-dihydropyridin-3-carboxylic acid instead of 4-ethoxy-1-(4-fluorophenyl)-2-oxo-1,2-dihydropyridin-3-carboxylic acid of Example 39 and by a similar method as Example 39, the title compound (41.1 mg, yield: 59%) was obtained.
¹H NMR (400 MHz, DMSO-d₆) δ 13.59 (s, 1H), 11.95 (s, 1H), 8.48 (m, 1H), 8.34 (m, 1H), 7.96 (d, 1H), 7.67 (s, 1H), 7.50-7.41 (m, 5H), 7.32 (m, 1H), 7.21 (m, 2H), 7.06 (m, 2H), 6.98 (m, 1H), 6.71 (d, 1H), 2.07 (s, 3H), 2.07 (s, 3H);
MS *m*/*z:* 542[M+H]

### Example 41. N-(4-(3-(2-aminopyrimidin-4-yl)-4-hydroxyphenoxy)-3-fluorophenyl)-N-(4-fluorophenyl)cyclopropan-1,1-dicarboxamide

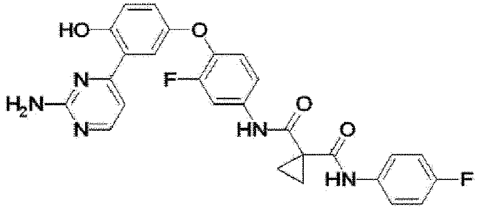

By using 1-((4- fluorophenyl)carbamoyl)cyclopropan-1-carboxylic acid instead of 4-ethoxy-1-(4-fluorophenyl)-2-oxo-1,2-dihydropyridin-3-carboxylic acid of Example 39 and by a similar method as Example 39, the title compound (12 mg, yield: 180) was obtained.
¹H NMR (400 MHz, MeOH-d₄) δ 8.29 (m, 1H), 7.68 (d, 1H), 7.58 (m, 3H), 7.25 (d, 1H), 7.09-6.99 (m, 5H), 6.94 (m, 1H), 1.62 (brs, 4H);
MS *m*/*z:* 518[M+H]

### Example 42. N-(4-(3-(2-aminopyrimidin-4-yl)-4-hydroxyphenoxy)-3-fluorophenyl)-1-(4-fluorophenyl)-4-methoxy-2-oxo-1,2-dihydropyridin-3-carboxamide

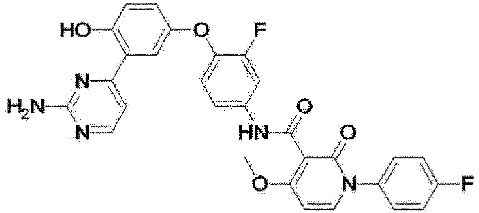

By using 1-(4-fluorophenyl)-4-methoxy-2-oxo-1,2-dihydropyridin-3-carboxylic acid instead of 4-ethoxy-1-(4-fluorophenyl)-2-oxo-1,2-dihydropyridin-3-carboxylic acid of Example 39 and by a similar method as Example 39, the title compound (20.1 mg, yield: 25%) was obtained.
¹H NMR (400 MHz, DMSO-d₆) δ 13.56 (s, 1H), 10.49 (s, 1H), 8.36 (s, 1H), 7.89 (m, 2H), 7.63 (s, 1H), 7.48 (m, 2H), 7.39 (m, 3H), 7.20 (m, 3H), 7.08 (m, 2H), 6.93 (d, 1H), 6.54 (d, 1H), 3.92 (s, 3H);
MS *m*/*z:* 558[M+H]

### Example 43. N-(4-(3-(2-aminopyrimidin-4-yl)-4-hydroxyphenoxy)-3-fluorophenyl)-1,5-dimethyl-3-oxo-2-phenyl-2,3-dihydro-1H-pyrazol-4-carboxamide

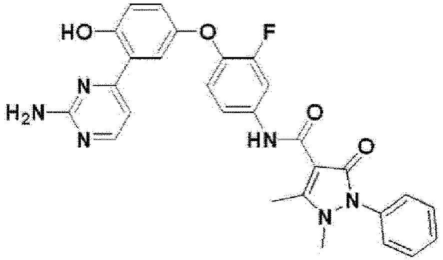

By using 1,5-dimethyl-3-oxo-2-phenyl-2,3-dihydro-1H-pyrazol-4-carboxylic acid instead of 4-ethoxy-1-(4-fluorophenyl)-2-oxo-1,2-dihydropyridin-3-carboxylic acid of Example 39 and by a similar method as Example 39, the title compound (42 mg, yield: 50%) was obtained.
¹H NMR (400 MHz, DMSO-d₆) δ 13.54 (s, 1H), 10.86 (s, 1H), 8.35 (m, 1H), 7.90 (d, 1H), 7.64 (m, 1H), 7.56 (m, 2H), 7.49 (m, 1H), 7.38 (m, 2H), 7.22 (m, 4H), 7.02 (m, 2H), 6.92 (d, 1H), 3.38 (s, 3H), 2.70 (s, 3H);
MS *m*/*z:* 527[M+H]

### Example 44. N-(4-(3-(2-aminopyrimidin-4-yl)-4-hydroxyphenoxy)-3-fluorophenyl)-5-bromo-1-(4-fluorophenyl)-2-oxo-1,2-dihydropyridin-3-carboxamide

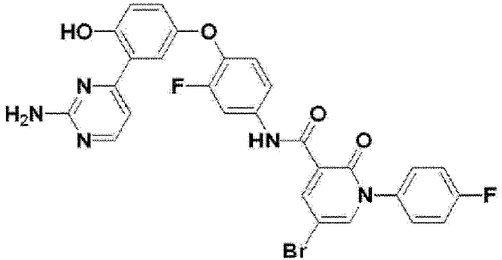

By using 5-bromo-1-(4-fluorophenyl)-2-oxo-1,2-dihydropyridin-3-carboxylic acid instead of 4-ethoxy-1-(4-fluorophenyl)-2-oxo-1,2-dihydropyridin-3-carboxylic acid of Example 39 and by a similar method as Example 39, the title compound (65 mg, yield: 40%) was obtained.
¹H NMR (400 MHz, DMSO-d₆) δ 13.60 (brs, 1H), 11.83 (s, 1H), 8.52 (m, 2H), 8.36 (m, 1H), 7.96 (m, 1H), 7.69 (m, 1H), 7.64 (m, 2H), 7.44 (m, 3H), 7.36 (m, 1H), 7.23 (m, 2H), 7.08 (m, 2H), 6.94 (m, 1H);
MS *m*/*z:* 606[M], 608[M+2]

### Example 45. N-(4-(3-(2-aminopyrimidin-4-yl)-4-hydroxyphenoxy)-3-fluorophenyl)-1-phenyl-5-(trifluoromethyl)-1H-imidazol-4-carboxamide

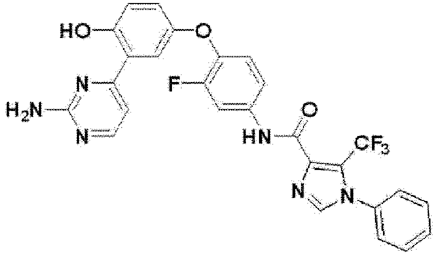

By using 1-phenyl-5-(trifluoromethyl)-1H-imidazol-4-carboxylic acid instead of 4-ethoxy-1-(4-fluorophenyl)-2-oxo-1,2-dihydropyridin-3-carboxylic acid of Example 39 and by a similar method as Example 39, the title compound (41 mg, yield: 47%) was obtained.
¹H NMR (400 MHz, DMSO-d₆) δ 13.59 (s, 1H), 10.65 (s, 1H), 8.36 (m, 2H), 7.96 (d, 1H), 7.68 (m, 1H), 7.59 (m, 6H), 7.23 (m, 3H), 7.10 (m, 2H), 6.95 (d, 1H);
MS *m*/*z:* 551[M+H]

### Example 46. N-(4-(3-(2-aminopyrimidin-4-yl)-4-hydroxyphenoxy)-3-fluorophenyl)-1-(4-fluorophenyl)-2-oxo-1,2-dihydropyridin-3-carboxamide

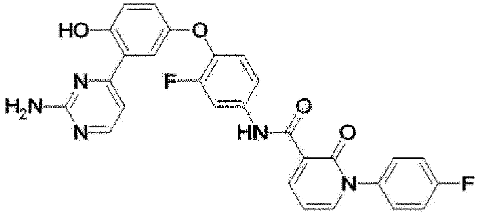

The compound obtained in Example 44 of N-(4-(3-(2-aminopyrimidin-4-yl)-4-hydroxyphenoxy)-3-fluorophenyl)-5-bromo-1-(4-fluorophenyl)-2-oxo-1,2-dihydropyridin-3-carboxamide (30 mg, 0.05 mmol) was dissolved in 3 ml of methanol, and 10% Pd/C was added thereto, followed by stirring under a hydrogen atmosphere. A catalyst was filtered through celite, and the filtrate was dried in vacuum. The residue was purified by prep-HPLC (0.1% formic acid in water/acetonitrile) to obtain the title compound (3.5 mg, yield: 130).
¹H NMR (400 MHz, CDCl₃) δ 11.90 (s, 1H), 8.76 (m, 1H), 8.32 (m, 1H), 7.91 (m, 1H), 7.69 (m, 1H), 7.44 (m, 3H), 7.29 (m, 2H), 7.15 (m, 5H), 6.66 (m, 1H), 5.81 (brs, 2H);
MS *m*/*z:* 528[M+H]

### Example 47. N-(4-(3-(2-aminopyrimidin-4-yl)-4-hydroxyphenoxy)-3-fluorophenyl)-1-(4-fluorophenyl)-5-(1-methyl-1H-pyrazol-4-yl)-2-oxo-1,2-dihydropyridin-3-carboxamide

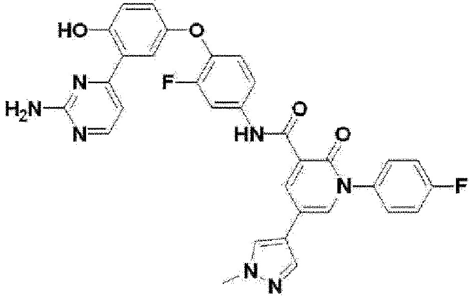

By using 1-(4-fluorophenyl)-5-(1-methyl-1H-pyrazol-4-yl)-2-oxo-1,2-dihydropyridin-3-carboxylic acid instead of 4-ethoxy-1-(4-fluorophenyl)-2-oxo-1,2-dihydropyridin-3-carboxylic acid of Example 39 and by a similar method as Example 39, the title compound (7 mg, yield: 120) was obtained.
¹H NMR (400 MHz, DMSO-d₆) δ 13.60 (s, 1H), 12.10 (s, 1H), 8.74 (m, 1H), 8.39 (m, 2H), 8.24 (s, 1H), 7.99 (m, 1H), 7.91 (m, 1H), 7.69 (m, 3H), 7.46 (m, 2H), 7.36 (m, 1H), 7.23 (m, 3H), 7.08 (m, 2H), 6.95 (d, 1H), 3.86 (s, 3H);
MS *m*/*z:* 608[M+H]

### Example 48. N-(4-(3-(2-aminopyrimidin-4-yl)-4-hydroxyphenoxy)-3-fluorophenyl)-5-cyclopropyl-1-(4-fluorophenyl)-2-oxo-1,2-dihydropyridin-3-carboxamide

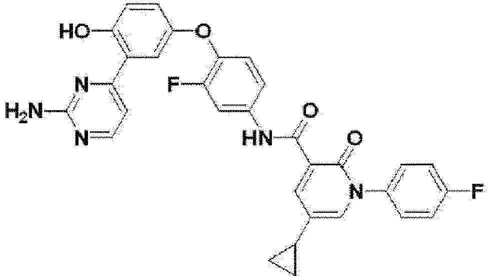

To a toluene/water (10:1) solution of the compound obtained in Example 44 of N-(4-(3-(2-aminopyrimidin-4-yl)-4-hydroxyphenoxy)-3-fluorophenyl)-5-bromo-1-(4-fluorophenyl)-2-oxo-1,2-dihydropyridin-3-carboxamide (30 mg, 0.05 mmol), cyclopropylboronic acid (6.4 mg, 0.07 mmol), tricyclohexylphosphine (1.4 mg, 0.005 mmol), K3PO4 (26.3 mg, 0.12 mmol), and PdOAc (1 mg, 0.005 mmol) were added, followed by stirring at 140°C for 30 minutes. The reaction solution was diluted with 50 ml of water, extracted with ethyl acetate three times, and concentrated. The residue was purified by prep-HPLC (0.1% formic acid in water/acetonitrile) to obtain the title compound (5.0 mg, yield: 180).
¹H NMR (400 MHz, DMSO-d₆) δ 13.56 (brs, 1H), 12.14 (s, 1H), 8.38 (d, 1H), 8.30 (s, 1H), 7.99 (m, 2H), 7.71 (m, 1H), 7.63 (m, 2H), 7.45 (m, 2H), 7.27 (m, 3H), 7.10 (m, 2H), 6.96 (d, 1H), 1.96 (m, 1H), 0.94 (m, 2H), 0.74 (m, 2H);
MS *m*/*z:* 568[M+H]

### Example 49. N-(4-(3-(2-aminopyrimidin-4-yl)-4-hydroxyphenoxy)-3-fluorophenyl)-1-(4-fluorophenyl)-2-oxo-6-(trifluoromethyl)-1,2-dihydropyridin-3-carboxamide

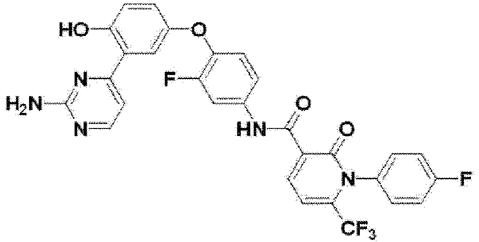

By using 1-(4-fluorophenyl)-2-oxo-6-(trifluoromethyl)-1,2-dihydropyridin-3-carboxylic acid instead of 4-ethoxy-1-(4-fluorophenyl)-2-oxo-1,2-dihydropyridin-3-carboxylic acid of Example 39 and by a similar method as Example 39, the title compound (31 mg, yield: 54%) was obtained.
¹H NMR (400 MHz, DMSO-d₆) δ 13.60 (s, 1H), 11.64 (s, 1H), 8.63 (d, 1H), 8.35 (d, 1H), 7.96 (m, 1H), 7.68 (s, 1H), 7.59 (m, 1H), 7.44 (m, 2H), 7.38 (m, 2H), 7.30 (m, 2H), 7.22 (m, 2H), 7.07 (m, 2H), 6.93 (m, 1H);
MS *m*/*z:* 596[M+H]

### Example 50. N-(4-(3-(2-aminopyrimidin-4-yl)-4-hydroxyphenoxy)-3-fluorophenyl)-1-(4-fluorophenyl)-6-(hydroxymethyl)-2-oxo-1,2-dihydropyridin-3-carboxamide

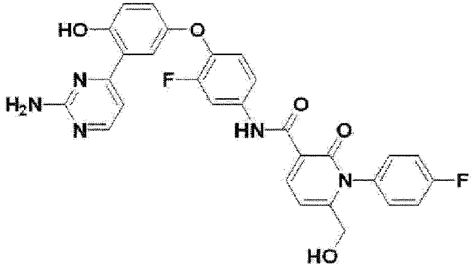

By using 1-(4-fluorophenyl)-6-(hydroxymethyl)-2-oxo-1,2-dihydropyridin-3-carboxylic acid instead of 4-ethoxy-1-(4-fluorophenyl)-2-oxo-1,2-dihydropyridin-3-carboxylic acid of Example 39 and by a similar method as Example 39, the title compound (27.9 mg, yield: 39%) was obtained.
¹H NMR (400 MHz, DMSO-d₆) δ 13.58 (s, 1H), 11.95 (s, 1H), 8.62 (d, 1H), 8.35 (s, 1H), 7.97 (d, 1H), 7.67 (s, 1H), 7.51 (m, 2H), 7.42 (m, 2H), 7.32 (m, 1H), 7.22 (m, 1H), 7.16 (brs, 2H), 7.07 (m, 2H), 6.94 (m, 1H), 6.88 (d, 1H), 5.79 (brs, 1H), 4.01 (s, 2H);
MS *m*/*z:* 558[M+H]

### Example 51. N-(4-(3-(2-aminopyrimidin-4-yl)-4-hydroxyphenoxy)-3-fluorophenyl)-1-phenyl-5-(tri fluoromethyl)-1H-pyrazol-4-carboxamide

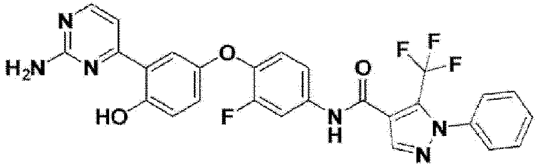

By using 1-phenyl-5-(trifluoromethyl)-1H-pyrazol-4-carboxylic acid instead of 4-ethoxy-1-(4-fluorophenyl)-2-oxo-1,2-dihydropyridin-3-carboxylic acid of Example 39 and by a similar method as Example 39, the title compound (40.3 mg, yield: 46%) was obtained.
¹H NMR (400 MHz, DMSO-d₆) δ 13.59 (s, 1H), 10.70 (s, 1H), 8.37 (m, 1H), 8.32 (s, 1H), 7.86 (d, 1H), 7.68 (m, 1H), 7.63 (m, 3H), 7.56 (m, 2H), 7.44 (m, 1H), 7.23 (m, 1H), 7.16 (brs, 2H), 7.09 (m, 2H), 6.95 (d, 1H) ;
MS *m*/*z:* 551[M+H]

### Example 52. N-(4-(3-(2-aminopyrimidin-4-yl)-4-hydroxyphenoxy)-3-fluorophenyl)-1-(2-hydroxy-2-methylpropyl)-5-methyl-3-oxo-2-phenyl-2,3-dihydro-1H-pyrazol-4-carboxamide

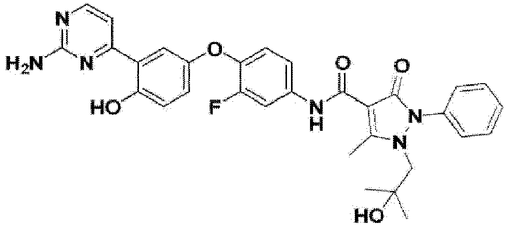

By using 1-(2-hydroxy-2-methylpropyl)-5-methyl-3-oxo-2-phenyl-2,3-dihydro-1H-pyrazol-4-carboxylic acid instead of 4-ethoxy-1-(4-fluorophenyl)-2-oxo-1,2-dihydropyridin-3-carboxylic acid of Example 39 and by a similar method as Example 39, the title compound (52.9 mg, 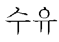 : 57%) was obtained.
¹H NMR (400 MHz, DMSO-d₆) δ 13.55 (s, 1H), 10.82 (s, 1H), 8.35 (m, 1H), 7.89 (m, 1H), 7.66 (d, 1H), 7.58 (m, 2H), 7.47 (m, 1H), 7.35 (m, 2H), 7.44 (m, 1H), 7.22 (m, 3H), 7.06 (m, 2H), 6.93 (d, 1H), 4.82 (brs, 1H), 3.86 (brs, 2H), 2.79 (s, 3H), 0.96 (s, 6H);
MS *m*/*z:* 585[M+H]

### Example 53. N-(4-(3-(2-aminopyrimidin-4-yl)-4-hydroxyphenoxy)-3-fluorophenyl)-5-ethyl-1-(pyrimidin-5-yl)-1H-pyrazol-4-carboxamide

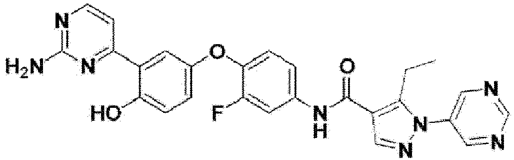

By using 5-ethyl-1-(pyrimidin-5-yl)-1H-pyrazol-4-carboxylic acid instead of 4-ethoxy-1-(4-fluorophenyl)-2-oxo-1,2-dihydropyridin-3-carboxylic acid of Example 39 and by a similar method as Example 39, the title compound (27 mg, 33%) was obtained.
¹H NMR (400 MHz, DMSO-d₆) δ 13.57 (s, 1H), 10.11 (s, 1H), 9.37 (s, 1H), 9.12 (s, 2H), 8.44 (m, 1H), 8.36 (m, 1H), 7.91 (d, 2H), 7.67 (m, 1H), 7.48 (m, 1H), 7.22 (m, 2H), 7.09 (m, 2H), 6.95 (d, 1H), 3.05 (m, 2H), 1.12 (t, 3H);
MS *m*/*z:* 513[M+H]

### Example 54. 4-Ethoxy-N-(3-fluoro-4-(4-hydroxy-3-(pyrimidin-4-yl)phenoxy)phenyl)-1-(4-fluorophenyl)-2-oxo-1,2-dihydropyridin-3-carboxamide

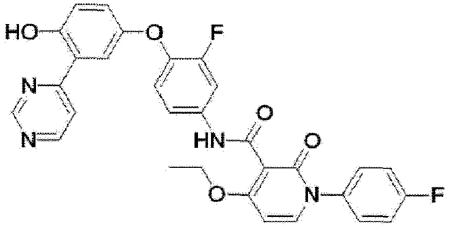

Hereinafter, the compound of Example 54 was prepared according to Reaction 14 below.

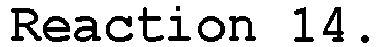

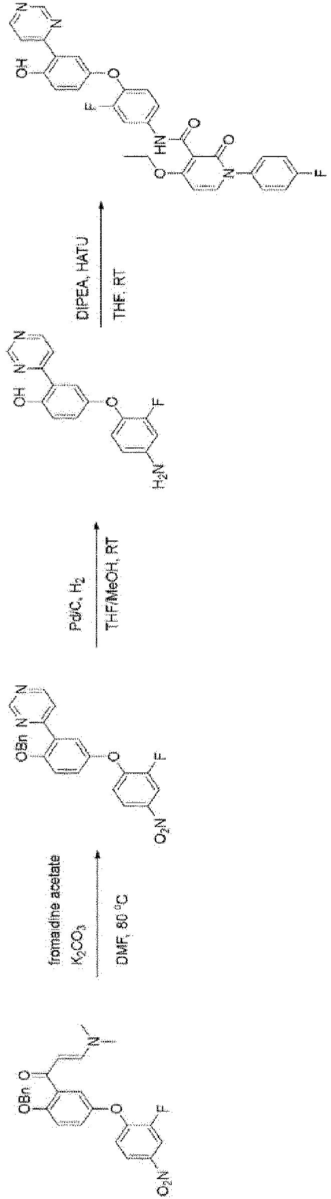

### Step 1. 4-(2-(Benzyloxy)-5-(2-fluoro-4-nitrophenoxy)phenyl)pyrimidine

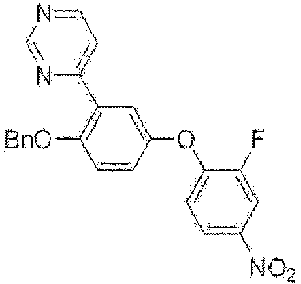

The compound obtained in Step 2 of Example 39 of (E)-1-(2-(benzyloxy)-5-(2-fluoro-4-nitrophenoxy)phenyl)-3-(dimethylamino)prop-2-en-1-one (200 mg, 0.46 mmol) was dissolved in 7 ml of DMF, and formamidine acetate (57.3 mg, 0.55 mmol) and TEA (0.1 ml, 0.69 mmol) were added thereto, followed by stirring at 80°C for 12 hours. The reaction mixture was cooled to room temperature, diluted with water, extracted with DCM three times, and concentrated. The residue was purified by column chromatography to obtain the title compound (180 mg, yield: 94%).
MS *m*/*z:* 418[M+H]

### Step 2. 4-(4-Amino-2-fluorophenoxy)-2-(pyrimidin-4-yl)phenol

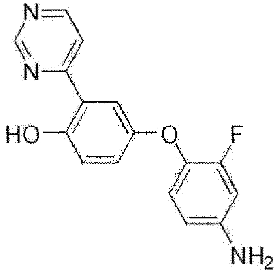

According to Step 4 of Example 1, by using the compound obtained in Step 1 above of 4-(2-(benzyloxy)-5-(2-fluoro-4-nitrophenoxy)phenyl)pyrimidine (180 mg, 0.43 mmol) instead of 4-(2-(benzyloxy)-5-bromophenyl)pyrimidin-2-amine and by a similar method, the title compound (80 mg, yield: 63%) was obtained.
MS *m*/*z:* 298[M+H]

### Step 3. 4-Ethoxy-N-(3-fluoro-4-(4-hydroxy-3-(pyrimidin-4-yl)phenoxy)phenyl)-1-(4-fluorophenyl)-2-oxo-1,2-dihydropyridin-3-carboxamide

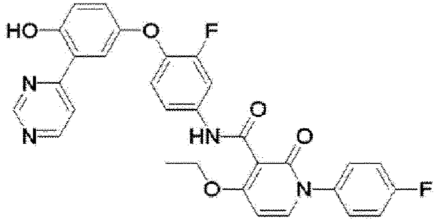

According to Step 5 of Example 1, by using the compound obtained in Step 2 above of 4-(4-amino-2-fluorophenoxy)-2-(pyrimidin-4-yl)phenol (30 mg, 0.10 mmol) and 4-ethoxy-1-(4-fluorophenyl)-2-oxo-1,2-dihydropyridin-3-carboxylic acid (33.6 mg, 0.12 mmol) and by a similar method as Example 1, the title compound (3.5 mg, yield: 6%) was obtained.
¹H NMR (400 MHz, DMSO-d₆) δ 12.20 (brs, 1H), 10.46 (s, 1H), 9.24 (s, 1H), 8.87 (m, 1H), 8.26 (m, 1H), 7.85 (m, 2H), 7.76 (s, 1H), 7.47 (m, 2H), 7.38 (m, 3H), 7.13 (m, 2H), 7.00 (m, 1H), 6.51 (d, 1H), 4.27 (q, 2H), 1.31(t, 3H);
MS *m*/*z:* 557[M+H]

### Example 55. N-(3-Fluoro-4-(4-hydroxy-3-(pyrimidin-4-yl)phenoxy)phenyl)-1-(4-fluorophenyl)-6-methyl-2-oxo-1,2-dihydropyridin-3-carboxamide

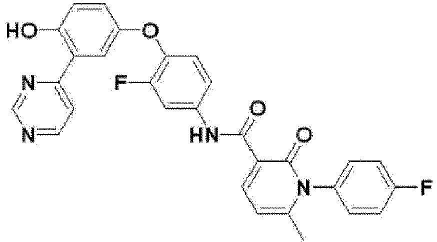

By using 1-(4-fluorophenyl)-6-methyl-2-oxo-1,2-dihydropyridin-3-carboxylic acid instead of 4-ethoxy-1-(4-fluorophenyl)-2-oxo-1,2-dihydropyridin-3-carboxylic acid of Example 54 and by a similar method as Example 54, the title compound (18.3 mg, yield: 26%) was obtained.
¹H NMR (400 MHz, DMSO-d₆) δ 12.62 (brs, 1H), 11.96 (s, 1H), 9.23 (s, 1H), 8.87 (m, 1H), 8.48 (m, 1H), 8.27 (m, 1H), 7.97 (d, 1H), 7.79 (m, 1H), 7.51 (m, 2H), 7.43 (m, 2H), 7.35 (m, 1H), 7.14 (m, 1H), 7.07 (m, 2H), 6.72 (d, 1H), 2.07 (s, 3H) ;
**MS *m*/*z:* 527[M+H]**

### Example 56. N-(3-fluoro-4-(4-hydroxy-3-(pyrimidin-4-yl)phenoxy)phenyl)-N-(4-fluorophenyl)cyclopropan-1,1-dicarboxamide

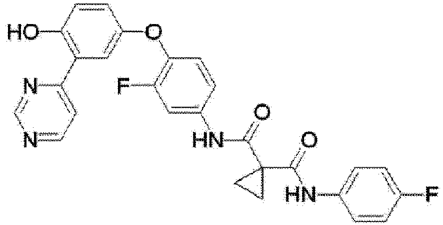

By using 1-((4-fluorophenyl)carbamoyl)cyclopropan-1-carboxylic acid instead of 4-ethoxy-1-(4-fluorophenyl)-2-oxo-1,2-dihydropyridin-3-carboxylic acid of Example 54 and by a similar method as Example 54, the title compound (2.5 mg, yield: 8%) was obtained.
¹H NMR (400 MHz, MeOH-d₄) δ 9.20 (s, 1H), 8.78 (m, 1H), 7.71 (m, 1H), 7.57 (m, 2H), 7.36 (m, 1H), 7.29 (m, 1H), 7.09 (m, 4H), 6.97 (m, 1H), 6.60 (m, 1H), 1.71(brs, 4H);
MS *m*/*z:* 503[M+H]

### Example 57. 4-Ethoxy-N-(3-fluoro-4-(4-hydroxy-3-(1H-pyrazol-3-yl)phenoxy)phenyl)-1-(4-fluorophenyl)-2-oxo-1,2-dihydropyridin-3-carboxamide

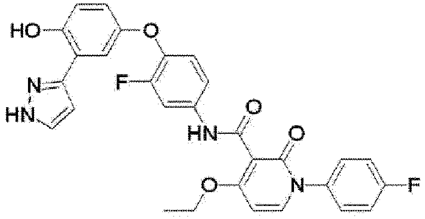

Hereinafter, the compound of Example 57 was prepared according to Reaction 15 below.

### Reaction 15.

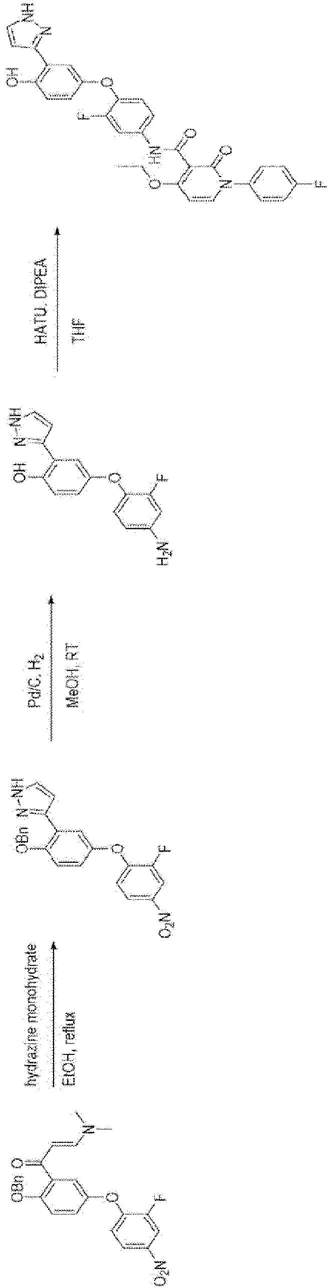

### Step 1. 3-(2-(Benzyloxy)-5-(2-fluoro-4-nitrophenoxy)phenyl)-1H-pyrazole

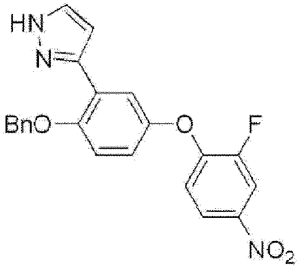

The compound obtained in Step 2 of Example 39 of (E)-1-(2-(benzyloxy)-5-(2-fluoro-4-nitrophenoxy)phenyl)-3-(dimethylamino)prop-2-en-1-one (250 mg, 0.57 mmol) was dissolved in 10 ml of ethanol, and hydrazine monohydrate (143 mg, 2.86 mmol) was added thereto, followed by stirring at 100°C for 2 hours. The reaction mixture was cooled to room temperature, diluted with water, extracted with DCM three times, and concentrated. The residue was purified by column chromatography to obtain the title compound (170 mg, yield: 95%) .
MS *m*/*z:* 316[M+H]

### Step 2. 4-(4-Amino-2-fluorophenoxy)-2-(1H-pyrazol-3-yl)phenol

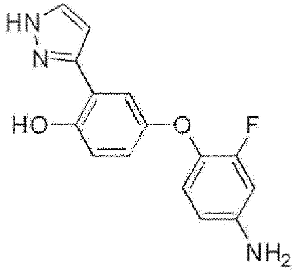

According to Step 4 of Example 1, by using the compound obtained in Step 1 above of 3-(2-(benzyloxy)-5-(2-fluoro-4-nitrophenoxy)phenyl)-1H-pyrazole (170 mg, 0.54 mmol) instead of 4-(2-(benzyloxy)-5-bromophenyl)pyrimidin-2-amine and by a similar method, the title compound (100 mg, yield: 65%) was obtained.
MS *m*/*z:* 286[M+H]

### Step 3. 4-Ethoxy-N-(3-fluoro-4-(4-hydroxy-3-(1H-pyrazol-3-yl)phenoxy)phenyl)-1-(4-fluorophenyl)-2-oxo-1,2-dihydropyridin-3-carboxamide

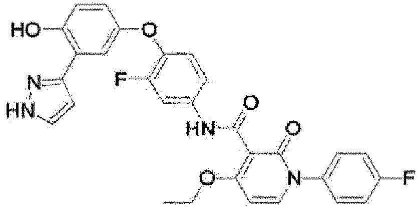

According to Step 5 of Example 1, by using the compound obtained in Step 2 above of 4-(4-amino-2-fluorophenoxy)-2-(1H-pyrazol-3-yl)phenol (33 mg, 0.11 mmol) and 4-ethoxy-1-(4-fluorophenyl)-2-oxo-1,2-dihydropyridin-3-carboxylic acid (38.5 mg, 0.13 mmol) and by a similar method as Example 1, the title compound (30 mg, yield: 48%) was obtained.
¹H NMR (400 MHz, DMSO-d₆) δ 10.42 (s, 1H), 7.85 (m, 3H), 7.47 (m, 3H), 7.38 (m, 3H), 7.07 (m, 1H), 6.92 (m, 2H), 6.82 (m, 1H), 6.51 (d, 1H), 4.27 (q, 2H), 1.31(t, 3H);
MS *m*/*z:* 545[M+H]

### Example 58. N-(3-Fluoro-4-(4-hydroxy-3-(1H-pyrazol-3-yl)phenoxy)phenyl)-1-(4-fluorophenyl)-6-methyl-2-oxo-1,2-dihydropyridin-3-carboxamide

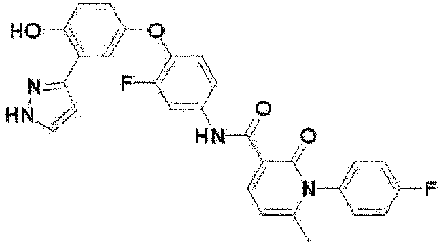

By using 1-(4-fluorophenyl)-6-methyl-2-oxo-1,2-dihydropyridin-3-carboxylic acid instead of 4-ethoxy-1-(4-fluorophenyl)-2-oxo-1,2-dihydropyridin-3-carboxylic acid of Example 57 and by a similar method as Example 57, the title compound (14.0 mg, yield: 24%) was obtained.
¹H NMR (400 MHz, DMSO-d₆) δ 11.95 (s, 1H), 8.49 (m, 1H), 7.96 (m, 1H), 7.51 (m, 3H), 7.45 (m, 3H), 7.32 (m, 1H), 7.01 (m, 1H), 6.93 (m, 1H), 6.85 (m, 2H), 6.72 (d, 1H), 2.08 (s, 3H);
MS *m*/*z:* 515[M+H]

### Example 59. N-(3-Fluoro-4-(4-hydroxy-3-(isoxazol-5-yl)phenoxy)phenyl)-1-(4-fluorophenyl)-6-methyl-2-oxo-1,2-dihydropyridin-3-carboxamide

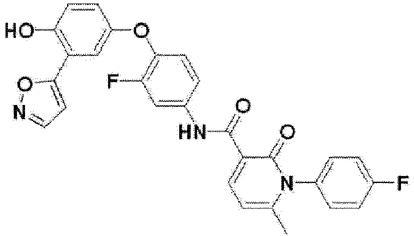

Hereinafter, the compound of Example 59 was prepared according to Reaction 16 below.

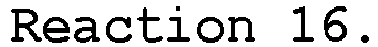

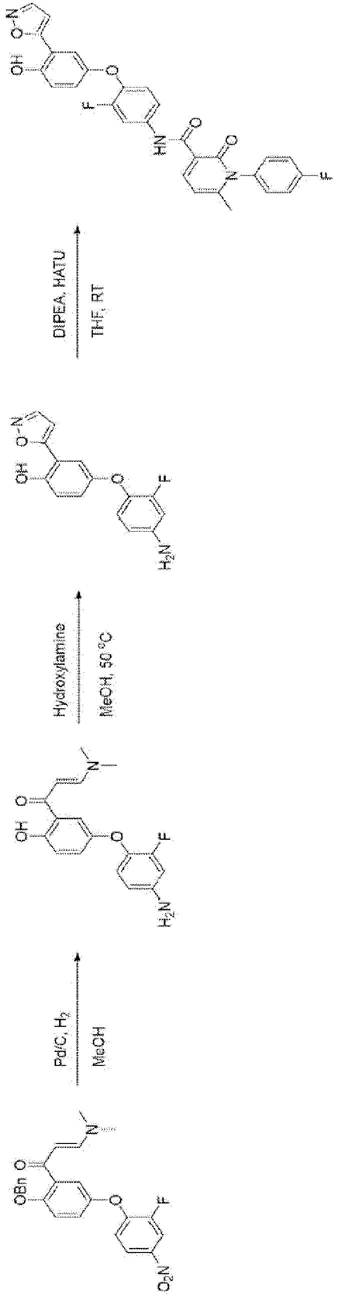

### Step 1. (E)-1-(5-(4-amino-2-fluorophenoxy)-2-hydroxyphenyl)-3-(dimethylamino)prop-2-en-1-one

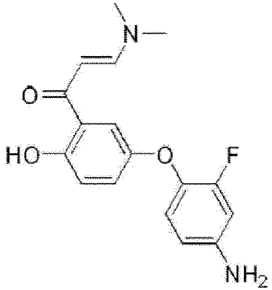

According to Step 4 of Example 1, by using the compound obtained in Step 2 of Example 39, (E)-1-(2-(benzyloxy)-5-(2-fluoro-4-nitrophenoxy)phenyl)-3-(dimethylamino)prop-2-en-1-one (100 mg, 0.23 mmol) instead of 4-(2-(benzyloxy)-5-bromophenyl)pyrimidin-2-amine and by a similar method, the title compound (50 mg, yield: 69%) was obtained.
MS *m*/*z:* 317[M+H]

### Step 2. 4-(4-Amino-2-fluorophenoxy)-2-(isoxazol-5-yl)phenol

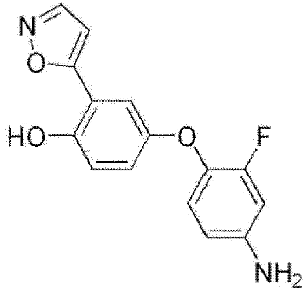

The compound obtained in Step 1 above of ((E)-1-(5-(4-amino-2-fluorophenoxy)-2-hydroxyphenyl)-3-(dimethylamino)prop-2-en-1-one (50 mg, 1.16 mmol) was dissolved in 5 ml of methanol, and hydroxylamine hydrochloride (16.5 mg, 0.24 mmol) was added thereto, followed by stirring at 50°C for 12 hours. The reaction mixture was cooled to room temperature, diluted in water, extracted with DCM three times, and concentrated. The residue was purified by column chromatography to obtain the title compound (25 mg, yield: 55%) .
MS *m*/*z:* 287[M+H]

### Step 3. N-(3-Fluoro-4-(4-hydroxy-3-(isoxazol-5-yl)phenoxy)phenyl)-1-(4-fluorophenyl)-6-methyl-2-oxo-1,2-dihydropyridin-3-carboxamide

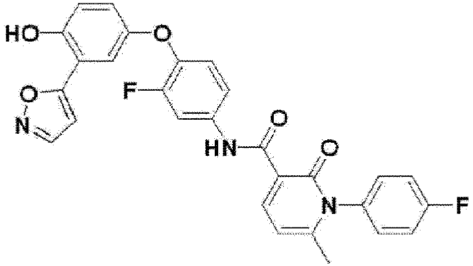

According to Step 5 of Example 1, by using the compound obtained in Step 2 above of 4-(4-amino-2-fluorophenoxy)-2-(isoxazol-5-yl)phenol (25 mg, 0.09 mmol) and 1-(4-fluorophenyl)-6-methyl-2-oxo-1,2-dihydropyridin-3-carboxylic acid (26 mg, 0.11 mmol) and by a similar method as Example 1, the title compound (14.0 mg, yield: 310) was obtained.
¹H NMR (400 MHz, DMSO-d₆) δ 11.98 (s, 1H), 10.57 (brs, 1H), 8.61 (m, 1H), 8.49 (d, 1H), 7.98 (m, 1H), 7.51 (m, 2H), 7.45 (m, 2H), 7.37 (m, 2H), 7.13 (m, 1H), 7.04 (m, 2H), 6.92 (s, 1H), 6.72 (d, 1H), 2.08 (s, 3H); MS *m*/*z:* 516[M+H]

### Example 60. N-(4-(3-(2-aminopyrimidin-5-yl)-4-hydroxyphenoxy)-3-fluorophenyl)-4-ethoxy-1-(4-fluorophenyl)-2-oxo-1,2-dihydropyridin-3-carboxamide

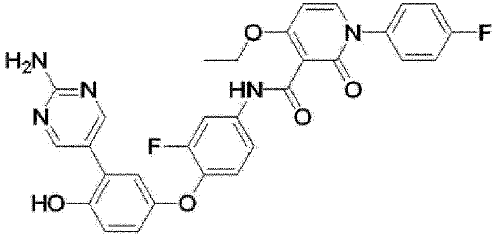

Hereinafter, the compound of Example 60 was prepared according to Reaction 17 below.

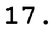

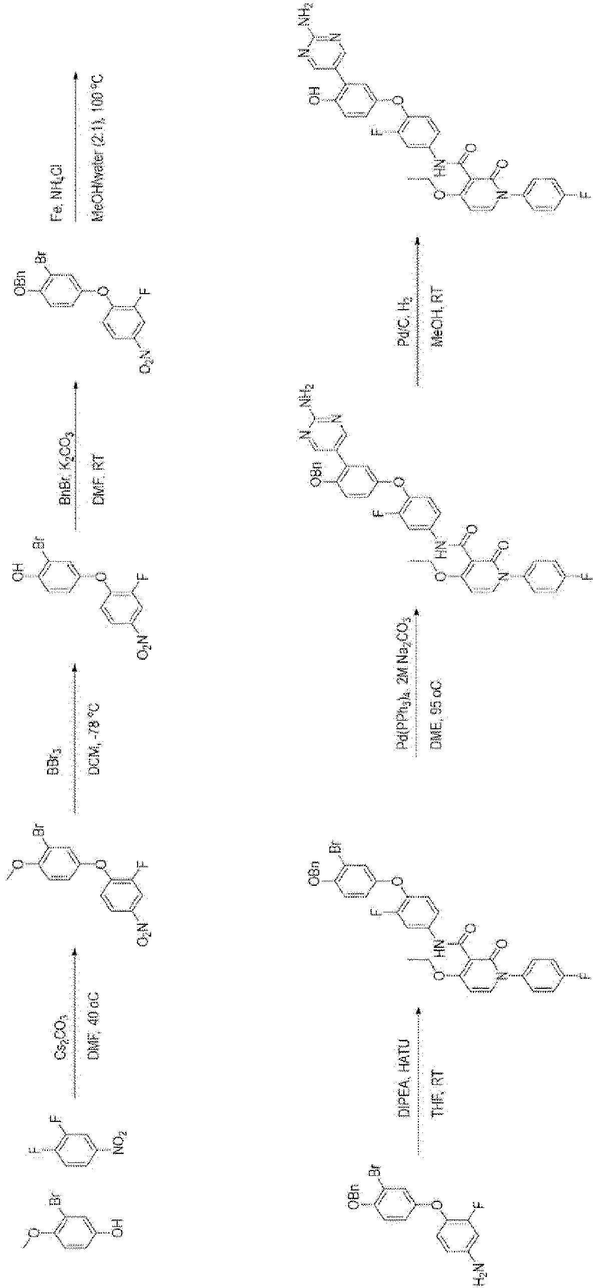

### Step 1. 2-Bromo-4-(2-fluoro-4-nitrophenoxy)-1-methoxybenzene

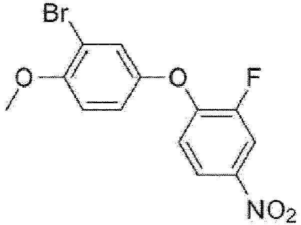

To a DMF solution of 3-bromo-4-methoxyphenol (1 g, 4.93 mmol), Cs₂CO₃ (3.2 g, 9.85 mmol) and 1,2-difluoro-4-nitrobenzene (0.86 g, 5.42 mmol) was added, followed by stirring at room temperature for 4 hours. The reaction solution was diluted with water and extracted with EA three times, and an organic layer was washed with brine and concentrated. The residue was purified by silica gel chromatography to obtain the title compound (1.4 g, yield: 83%) .
MS *m*/*z:* 342[M], 344[M+2]

### Step 2. 2-Bromo-4-(2-fluoro-4-nitrophenoxy)phenol

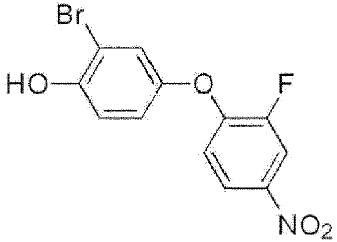

According to Step 1 of Example 24, by using the compound obtained in Step 1 above of 2-bromo-4-(2-fluoro-4-nitrophenoxy)-1-methoxybenzene (600 mg, 1.75 mmol) and 1 M BBr₃ (8.77 ml, 8.77 mmol) and by a similar method, the title compound (190 mg, yield: 33%) was obtained.
MS *m*/*z:* 328[M], 330[M+2]

### Step 3. 1-(Benzyloxy)-2-bromo-4-(2-fluoro-4-nitrophenoxy)benzene

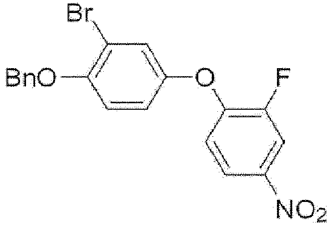

According to Step 1 of Example 1, by using the compound obtained in Step 2 above of 2-bromo-4-(2-fluoro-4-nitrophenoxy)phenol (190 mg, 0.58 mmol), benzyl bromide (83 µl, 0,70 mmol), and K₂CO₃ (120 mg, 0.87 mmol) and by a similar method, the title compound (196 mg, yield: 810) was obtained.
MS *m*/*z:* 418[M], 420[M+2]

### Step 4. 4-(4-(Benzyloxy)-3-bromophenoxy)-3-fluoroaniline

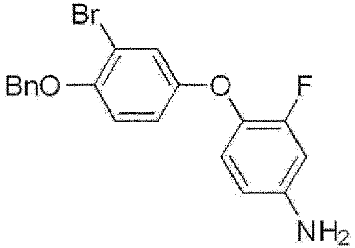

The compound obtained in Step 3 above of 1-(benzyloxy)-2-bromo-4-(2-fluoro-4-nitrophenoxy)benzene (196 mg, 0.47 mmol) was dissolved in 5 ml of methanol/water (2:1), and iron (79 mg, 1.41 mmol) and NH₄Cl (125 mg, 2.35 mmol) were added thereto, followed by stirring at 100°C for 2 hours. The reaction mixture was cooled to room temperature, diluted with EA, and filtered through celite. The filtrate was washed with brine and concentrated. The residue was purified by column chromatography to obtain the title compound (167 mg, yield: 92%) .
MS *m*/*z:* 388[M], 390[M+2]

### Step 5. N-(4-(4-(benzyloxy)-3-bromophenoxy)-3-fluorophenyl)-4-ethoxy-1-(4-fluorophenyl)-2-oxo-1,2-dihydropyridin-3-carboxamide

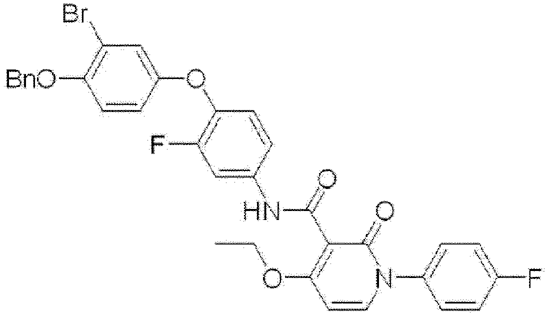

According to Step 5 of Example 1, by using the compound obtained in Step 4 above of 4-(4-(benzyloxy)-3-bromophenoxy)-3-fluoroaniline (167 mg, 0.43 mmol) and 4-ethoxy-1-(4-fluorophenyl)-2-oxo-1,2-dihydropyridin-3-carboxylic acid (143 mg, 0.52 mmol) and by a similar method, the title compound (182 mg, yield: 65%) was obtained.
MS *m*/*z:* 647[M], 649[M+2]

### Step 6. N-(4-(3-(2-aminopyrimidin-5-yl)-4-(benzyloxy)phenoxy)-3-fluorophenyl)-4-ethoxy-1-(4-fluorophenyl)-2-oxo-1,2-dihydropyridin-3-carboxamide

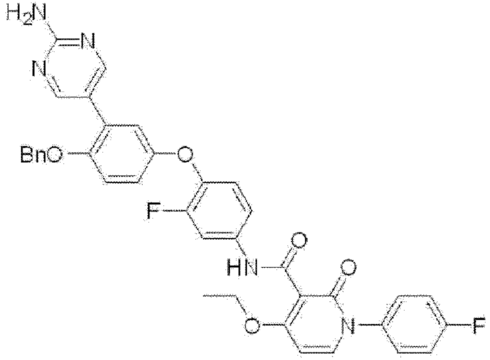

According to Step 4 of Example 1, by using the compound obtained in Step 5 above of N-(4-(4-(benzyloxy)-3-bromophenoxy)-3-fluorophenyl)-4-ethoxy-1-(4-fluorophenyl)-2-oxo-1,2-dihydropyridin-3-carboxamide (45 mg, 0.07 mmol) and (2-aminopyrimidin-5-yl)boronic acid (15 mg, 0,11 mmol) and by a similar method, the title compound (20.0 mg, yield: 44%) was obtained.
MS *m*/*z:* 662[M+H]

### Step 7. N-(4-(3-(2-aminopyrimidin-5-yl)-4-hydroxyphenoxy)-3-fluorophenyl)-4-ethoxy-1-(4-fluorophenyl)-2-oxo-1,2-dihydropyridin-3-carboxamide

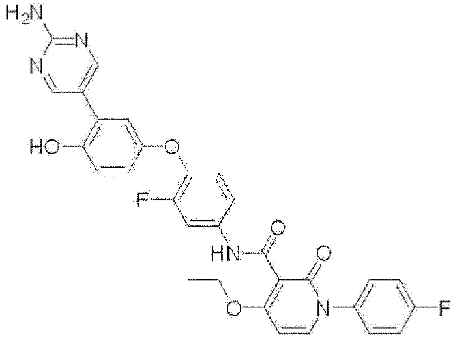

According to Step 6 of Example 1, by using the compound obtained in Step 6 above of N-(4-(3-(2-aminopyrimidin-5-yl)-4-(benzyloxy)phenoxy)-3-fluorophenyl)-4-ethoxy-1-(4-fluorophenyl)-2-oxo-1,2-dihydropyridin-3-carboxamide (20 mg, 0.03 mmol) and by a similar method as Example 1, the title compound (8.0 mg, yield: 46%) was obtained.
¹H NMR (400 MHz, DMSO-d₆) δ 10.41 (s, 1H), 9.59 (brs, 1H), 8.43 (s, 2H), 7.85 (m, 2H), 7.47 (m, 2H), 7.38 (m, 3H), 7.09 (m, 1H), 7.01 (m, 1H), 6.91 (m, 1H), 6.77 (m, 1H), 6.67 (m, 1H), 6.51 (d, 1H), 4.27 (q, 2H), 1.31(t, 3H);
MS *m*/*z:* 572[M+H]

### Example 61. N-(4-(3-(2-aminopyridin-4-yl)-4-hydroxyphenoxy)-3-fluorophenyl)-4-ethoxy-1-(4-fluorophenyl)-2-oxo-1,2-dihydropyridin-3-carboxamide

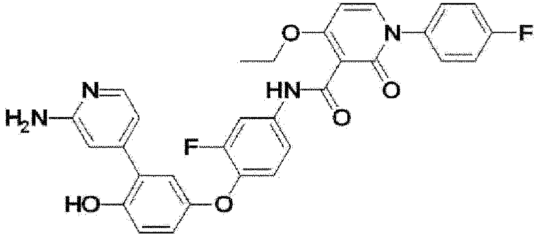

In Step 6 of Example 60, by using (2-aminopyridin-4-yl)boronic acid instead of (2-aminopyrimidin-5-yl)boronic acid and by a similar method as Example 60, the title compound (5.4 mg, yield: 37%) was obtained.
¹H NMR (400 MHz, DMSO-d₆) δ 10.43 (s, 1H), 9.56 (brs, 1H), 7.89 (m, 3H), 7.47 (m, 2H), 7.38 (m, 3H), 7.10 (m, 1H), 6.94 (d, 1H), 6.87 (m, 2H), 6.65 (m, 2H), 6.40 (d, 1H), 5.85 (brs, 2H), 4.25 (q, 2H), 1.31(t, 3H);
MS *m*/*z:* 571[M+H].

### Experimental Example 1. Evaluation of inhibitory activity to RON and MET

The evaluation results on the inhibitory activity of the title compounds are shown in Table 2 below.

**[Table 2]**

| | RON (IC₅₀, nM) | MET (IC₅₀, nM) |
|---|---|---|
| Example 1 | 144 | 612 |
| Example 2 | 131 | 583 |
| Example 7 | 942 | 2234 |
| Example 8 | 834 | 2093 |
| Example 10 | 855 | 1709 |
| Example 13 | 1330 | 2940 |
| Example 17 | 2300 | >10000 |
| Example 28 | 1170 | 180 |
| Example 30 | 248 | 49 |
| Example 34 | 2280 | 552 |
| Example 37 | 6970 | 954 |
| Example 38 | 2220 | 354 |
| Example 39 | 86 | 27 |
| Example 40 | 56 | 4 |
| Example 41 | 362 | 71 |
| Example 42 | 355 | 53 |
| Example 43 | 279 | 48 |
| Example 44 | 137 | 46 |
| Example 46 | 241 | 54 |
| Example 47 | 882 | 179 |
| Example 48 | 23 | 64 |
| Example 49 | 163 | 67 |
| Example 50 | 133 | 12 |
| Example 52 | 876 | 115 |
| Example 54 | 351 | 67 |
| Example 56 | 3350 | 281 |
| Example 57 | 4480 | 160 |
| Example 58 | 1450 | 99 |

## Claims

1. A compound of the following Formula 1 or a pharmaceutically acceptable salt thereof: 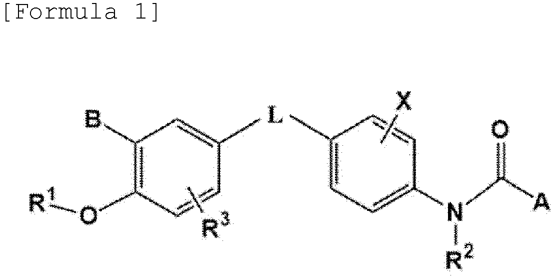 in Formula 1,
L is a direct linkage, O or NR⁴,
R¹ is hydrogen, C₁-C₆ alkyl, substituted C₁-C₆ alkyl, C₅-C₇ aryl, or substituted C₅-C₇ aryl, where a substituent of the substituted C₁-C₆ alkyl and the substituted C₅-C₇ aryl is selected from the group consisting of C₁-C₆ alkyl, C₆-C₁₀ aryl, halogen, C₁-C₆ haloalkyl, C₁-C₆ alkoxy, C₁-C₆ alkylthio, hydroxy, -COOH, C₁-C₆ alkylcarbonyl, C₁-C₆ alkyloxycarbonyl, C₁-C₆ alkylcarbonyloxy, -NH₂, carbamoyl, urea and -SH,
R² is hydrogen, C₁-C₆ alkyl or substituted C₁-C₆ alkyl, where a substituent of the substituted C₁-C₆ alkyl is selected from the group consisting of C₁-C₆ alkyl, C₆-C₁₀ aryl, halogen, C₁-C₆ haloalkyl, C₁-C₆ alkoxy, C₁-C₆ alkylthio, hydroxy, -COOH, C₁-C₆ alkylcarbonyl, C₁-C₆ alkyloxycarbonyl, C₁-C₆ alkylcarbonyloxy, -NH₂, carbamoyl, urea and -SH,
R³ is hydrogen, halogen, C₁-C₆ alkyl or substituted C₁-C₆ alkyl, where a substituent of the substituted C₁-C₆ alkyl is selected from the group consisting of C₁-C₆ alkyl, C₆-C₁₀ aryl, halogen, C₁-C₆ haloalkyl, C₁-C₆ alkoxy, C₁-C₆ alkylthio, hydroxy, -COOH, C₁-C₆ alkylcarbonyl, C₁-C₆ alkyloxycarbonyl, C₁-C₆ alkylcarbonyloxy, -NH₂, carbamoyl, urea and -SH,
R⁴ is hydrogen, C₁-C₆ alkyl or substituted C₁-C₆ alkyl, where a substituent of the substituted C₁-C₆ alkyl is selected from the group consisting of C₁-C₆ alkyl, C₆-C₁₀ aryl, halogen, C₁-C₆ haloalkyl, C₁-C₆ alkoxy, C₁-C₆ alkylthio, hydroxy, -COOH, C₁-C₆ alkylcarbonyl, C₁-C₆ alkyloxycarbonyl, C₁-C₆ alkylcarbonyloxy, -NH₂, carbamoyl, urea and -SH,
X is hydrogen, C₁-C₆ alkyl, substituted C₁-C₆ alkyl, or halogen, where a substituent of the substituted C₁-C₆ alkyl is selected from the group consisting of C₁-C₆ alkyl, C₆-C₁₀ aryl, halogen, C₁-C₆ haloalkyl, C₁-C₆ alkoxy, C₁-C₆ alkylthio, hydroxy, -COOH, C₁-C₆ alkylcarbonyl, C₁-C₆ alkyloxycarbonyl, C₁-C₆ alkylcarbonyloxy, -NH₂, carbamoyl, urea and -SH,
A is 5-member to 7-member, substituted or unsubstituted heteroaryl containing 1 to 3 heterocycle-forming atoms selected from the group consisting of nitrogen, sulfur and oxygen, or 3-member to 7-member, substituted or unsubstituted cycloalkyl, where a substituent of the substituted heteroaryl and the substituted cyclolakyl is hydrogen, halogen, C₁-C₆ alkyl, C₁-C₆ alkyl, substituted C₁-C₆ alkyl, C₃-C₆ cycloalkyl, C₁-C₅ alkoxy, 5-member to 12-member aryl, C₁-C₆ alkyl, substituted 5-member to 12-member aryl, 5-member to 7-member heteroaryl containing 1 to 3 heterocycle-forming atoms selected from the group consisting of nitrogen, sulfur and oxygen, or C₁-C₆ alkyl, substituted 5-member to 7-member heteroaryl, or oxo,
B is C₁-C₆ alkylcarbonyl, C₁-C₆ alkylimine, N-hydroxy-substituted C₁-C₆ alkylimine, halogen, 5-member to 7-member monoaryl, substituted 5-member to 7-member monoaryl, 5-member to 7-member heteromonoaryl containing 1 to 3 heterocycle-forming atoms selected from the group consisting of nitrogen, sulfur and oxygen, substituted 5-member to 7-member heteromonoaryl, 9-member to 10-member fused biaryl, substituted 9-member to 10-member fused biaryl, 9-member to 10-member fused heterobiaryl containing 1 to 3 heterocycle-forming atoms selected from the group consisting of nitrogen, sulfur and oxygen, or substituted 9-member to 10-member fused heterobiaryl, where substituents of the substituted monoaryl, heteromonoaryl, biaryl and heterobiaryl are 1 to 3 substituents independently selected from the group consisting of C₁-C₃ alkyl, halogen, hydroxy, -NH₂ and -SH, and
the halogen is independently selected from the group consisting of F, Cl, Br and I.

2. The compound or the pharmaceutically acceptable salt thereof according to claim 1, wherein A has a structure of the following Formula (IIa), Formula (IIb) or Formula (IIc): 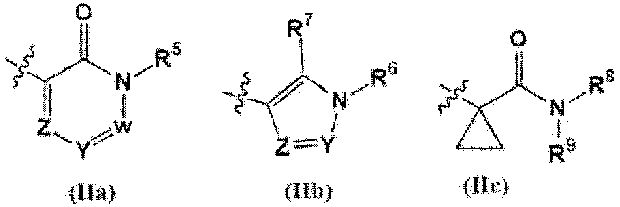 in Formula (IIa), Formula (IIb) and Formula (IIc),
Y and Z are each independently CR¹⁰, COR¹⁰ or NR¹⁰,
W is a direct linkage, CR¹⁰ or COR¹⁰,
R⁵, R⁶ and R⁸ are each independently 5-member to 7-member aryl, substituted 5-member to 7-member aryl, 5-member to 7-member heteroaryl containing 1 to 3 heterocycle-forming atoms selected from the group consisting of nitrogen, sulfur and oxygen, or substituted 5-member to 7-member heteroaryl, where substituents in the substituted 5-member to 7-member aryl or the 5-member to 7-member heteroaryl are each independently C₁-C₆ alkyl, halogen or hydroxy,
R⁷ and R⁹ are each independently hydrogen, C₁-C₆ alkyl or halogen-substituted C₁-C₆ alkyl, and
R¹⁰ is hydrogen, halogen, C₁-C₆ alkyl, substituted C₁-C₆ alkyl, C₃-C₆ cycloalkyl, substituted C₃-C₆ cycloalkyl, 5-member to 7-member aryl, substituted 5-member to 7-member aryl, 5-member to 7-member heteroaryl containing 1 to 3 heterocycle-forming atoms selected from the group consisting of nitrogen, sulfur and oxygen, or substituted 5-member to 7-member heteroaryl, where substituents in the substituted C₁-C₆ alkyl, C₃-C₆ cycloalkyl, 5-member to 7-member aryl, or 5-member to 7-member heteroaryl are each independently C₁-C₆ alkyl, hydroxy or halogen.

3. The compound or the pharmaceutically acceptable salt thereof according to claim 1, wherein B is a compound having the following structures:

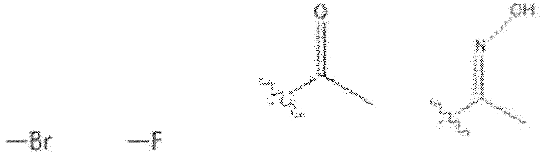

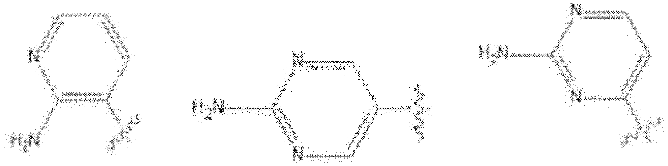

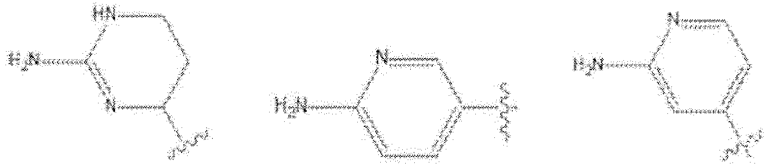

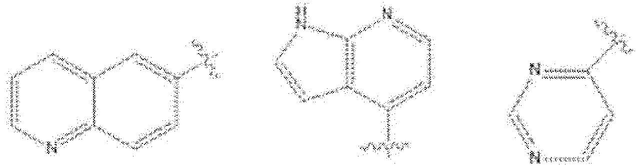

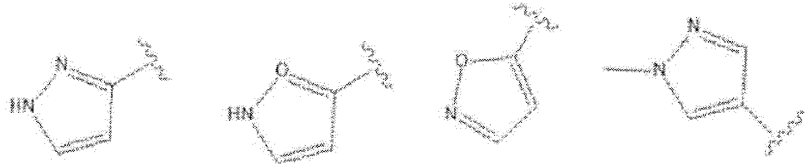

4. The compound or the pharmaceutically acceptable salt thereof according to claim 1, wherein L is a direct linkage, O or NH.

5. The compound or the pharmaceutically acceptable salt thereof according to claim 1, wherein R¹ is hydrogen, methyl, ethyl or benzyl.

6. The compound or the pharmaceutically acceptable salt thereof according to claim 1, wherein R² is hydrogen or methyl.

7. The compound or the pharmaceutically acceptable salt thereof according to claim 1, wherein R³ is hydrogen.

8. The compound or the pharmaceutically acceptable salt thereof according to claim 1, wherein X is hydrogen, methyl or fluorine.

9. The compound or the pharmaceutically acceptable salt thereof according to claim 1, wherein the compound is selected from the group consisting of
N-(3'-(2-aminopyrimidin-4-yl)-2-fluoro-4'-hydroxy-[1,1'-biphenyl]-4-yl)-4-ethoxy-1-(4-fluorophenyl)-2-oxo-1,2-dihydropyridin-3-carboxamide,
N-(3'-(2-aminopyrimidin-4-yl)-2-fluoro-4'-hydroxy-[1,1'-biphenyl]-4-yl)-1-(4-fluorophenyl)-4-methoxy-2-oxo-1,2-dihydropyridin-3-carboxamide,
N-(3'-(2-aminopyrimidin-4-yl)-2-fluoro-4'-hydroxy-[1,1'-biphenyl]-4-yl)-1-(4-fluorophenyl)-6-methyl-2-oxo-1,2-dihydropyridin-3-carboxamide,
N-(3'-(2-aminopyrimidin-4-yl)-2-fluoro-4'-hydroxy-[1,1'-biphenyl]-4-yl)-1,5-dimethyl-3-oxo-2-phenyl-2,3-dihydro-1H-pyrazol-4-carboxamide,
N-(3'-(2-aminopyrimidin-4-yl)-2-fluoro-4'-hydroxy-[1,1'-biphenyl]-4-yl)-1-phenyl-5-(trifluoromethyl)-1H-imidazol-4-carboxamide,
N-(3'-(2-aminopyrimidin-4-yl)-2-fluoro-4'-hydroxy-[1,1'-biphenyl]-4-yl)-5-bromo-1-(4-fluorophenyl)-2-oxo-1,2-dihydropyridin-3-carboxamide,
N-(3'-(2-aminopyrimidin-4-yl)-2-fluoro-4'-hydroxy-[1,1'-biphenyl]-4-yl)-1-(4-fluorophenyl)-5-(1-methyl-1H-pyrazol-4-yl)-2-oxo-1,2-dihydropyridin-3-carboxamide,
N-3'-(2-aminopyrimidin-4-yl)-2-fluoro-4'-hydroxy-[1,1'-biphenyl]-4-yl)-5-cyclopropyl-1-(4-fluorophenyl)-2-oxo-1,2-dihydropyridin-3-carboxamide,
N-(3'-(2-aminopyrimidin-4-yl)-2-fluoro-4'-hydroxy-[1,1'-biphenyl]-4-yl)-1-(4-fluorophenyl)-2-oxo-6-(trifluoromethyl)-1,2-dihydropyridin-3-carboxamide,
N-(3'-(2-aminopyrimidin-4-yl)-2-fluoro-4'-hydroxy-[1,1'-biphenyl]-4-yl)-1-(4-fluorophenyl)-6-(hydroxymethyl)-2-oxo-1,2-dihydropyridin-3-carboxamide,
N-(3'-(2-aminopyrimidin-4-yl)-4'-(benzyloxy)-2-methyl-[1,1'-biphenyl]-4-yl)-4-ethoxy-1-(4-fluorophenyl)-2-oxo-1,2-dihydropyridin-3-carboxamide,
N-(3'-(2-aminopyrimidin-4-yl)-4'-(benzyloxy)-2-methyl-[1,1'-biphenyl]-4-yl)-N-(4-fluorophenyl)cyclopropan-1,1-dicarboxamide,
N-(3'-(2-aminopyrimidin-4-yl)-4'-hydroxy-2-methyl-[1,1'-biphenyl]-4-yl)-4-ethoxy-1-(4-fluorophenyl)-2-oxo-1,2-dihydropyridin-3-carboxamide,
N-(3'-(2-aminopyrimidin-4-yl)-4'-hydroxy-2-methyl-[1,1'-biphenyl]-4-yl)-1-(4-fluorophenyl)-6-methyl-2-oxo-1,2-dihydropyridin-3-carboxamide,
N-(3'-(2-aminopyrimidin-4-yl)-4'-hydroxy-2-methyl-[1,1'-biphenyl]-4-yl)-N-(4-fluorophenyl)cyclopropan-1,1-dicarboxamide,
N-(3'-(2-amino-1,4,5,6-tetrahydropyrimidin-4-yl)-4'-hydroxy-2-methyl-[1,1'-biphenyl]-4-yl)-N-(4-fluorophenyl)cyclopropan-1,1-dicarboxamide,
N-(3'-(2-aminopyrimidin-5-yl)-2-fluoro-4'-methoxy-[1,1'-biphenyl]-4-yl)-4-ethoxy-1-(4-fluorophenyl)-2-oxo-1,2-dihydropyridin-3-carboxamide,
N-(3'-(6-aminopyridin-3-yl)-2-fluoro-4'-methoxy-[1,1'-biphenyl]-4-yl)-4-ethoxy-1-(4-fluorophenyl)-2-oxo-1,2-dihydropyridin-3-carboxamide,
N-(3'-(2-aminopyridin-4-yl)-2-fluoro-4'-methoxy-[1,1'-biphenyl]-4-yl)-4-ethoxy-1-(4-fluorophenyl)-2-oxo-1,2-dihydropyridin-3-carboxamide,
N-(3'-(2-aminopyridin-3-yl)-2-fluoro-4'-methoxy-[1,1'-biphenyl]-4-yl)-4-ethoxy-1-(4-fluorophenyl)-2-oxo-1,2-dihydropyridin-3-carboxamide,
4-ethoxy-N-(2-fluoro-4'-methoxy-3'-(quinolin-6-yl)-[1,1'-biphenyl]-4-yl)-1-(4-fluorophenyl)-2-oxo-1,2-dihydropyridin-3-carboxamide,
4-ethoxy-N-(2-fluoro-3'-(isoquinolin-6-yl)-4'-methoxy-[1,1'-biphenyl]-4-yl)-1-(4-fluorophenyl)-2-oxo-1,2-dihydropyridin-3-carboxamide,
4-ethoxy-N-(2-fluoro-4'-methoxy-3'-(1H-pyrrolo[2,3-b]pyridin-4-yl)-[1,1'-biphenyl]-4-yl)-1-(4-fluorophenyl)-2-oxo-1,2-dihydropyridin-3-carboxamide,
N-(3'-bromo-2-fluoro-4'-hydroxy-[1,1'-biphenyl]-4-yl)-4-ethoxy-1-(4-fluorophenyl)-2-oxo-1,2-dihydropyridin-3-carboxamide,
N-(3'-(2-aminopyrimidin-5-yl)-2-fluoro-4'-hydroxy-[1,1'-biphenyl]-4-yl)-4-ethoxy-1-(4-fluorophenyl)-2-oxo-1,2-dihydropyridin-3-carboxamide,
N-(3'-(2-aminopyridin-4-yl)-2-fluoro-4'-hydroxy-[1,1'-biphenyl]-4-yl)-4-ethoxy-1-(4-fluorophenyl)-2-oxo-1,2-dihydropyridin-3-carboxamide,
4-ethoxy-N-(2-fluoro-4'-hydroxy-3'-(1-methyl-1H-pyrazol-4-yl)-[1,1'-biphenyl]-4-yl)-1-(4-fluorophenyl)-2-oxo-1,2-dihydropyridin-3-carboxamide,
N-(4-((3-(2-aminopyrimidin-4-yl)-4-hydroxyphenyl)amino)-3-fluorophenyl)-4-ethoxy-1-(4-fluorophenyl)-2-oxo-1,2-dihydropyridin-3-carboxamide,
N-(4-((3-(2-aminopyrimidin-4-yl)-4-hydroxyphenyl)amino)-3-fluorophenyl)-1-(4-fluorophenyl)-4-methoxy-2-oxo-1,2-dihydropyridin-3-carboxamide,
N-(4-((3-(2-aminopyrimidin-4-yl)-4-hydroxyphenyl)amino)-3-fluorophenyl)-1-(4-fluorophenyl)-6-methyl-2-oxo-1,2-dihydropyridin-3-carboxamide,
N-(4-((3-(2-aminopyrimidin-4-yl)-4-hydroxyphenyl)amino)-3-fluorophenyl)-N-(4-fluorophenyl)cyclopropan-1,1-dicarboxamide,
N-(4-((3-(2-aminopyrimidin-4-yl)-4-hydroxyphenyl)amino)-3-fluorophenyl)-1,5-dimethyl-3-oxo-2-phenyl-2,3-dihydro-1H-pyrazol-4-carboxamide,
N-(4-((3-(2-aminopyrimidin-4-yl)-4-hydroxyphenyl)amino)-3-fluorophenyl)-1-phenyl-5-(trifluoromethyl)-1H-imidazol-4-carboxamide,
N-(4-((3-(2-aminopyrimidin-4-yl)-4-hydroxyphenyl)amino)-2-fluorophenyl)-4-ethoxy-1-(4-fluorophenyl)-2-oxo-1,2-dihydropyridin-3-carboxamide,
N-(4-((3-(2-aminopyrimidin-4-yl)-4-hydroxyphenyl)amino)phenyl)-4-ethoxy-1-(4-fluorophenyl)-2-oxo-1,2-dihydropyridin-3-carboxamide,
N-(4-(3-acetyl-4-hydroxyphenoxy)-3-fluorophenyl)-4-ethoxy-1-(4-fluorophenyl)-2-oxo-1,2-dihydropyridin-3-carboxamide,
N-(4-(3-acetyl-4-hydroxyphenoxy)-3-fluorophenyl)-1-(4-fluorophenyl)-6-methyl-2-oxo-1,2-dihydropyridin-3-carboxamide,
(E)-N-(3-fluoro-4-(4-hydroxy-3-(1-(hydroxyimino)ethyl)phenoxy)phenyl)-1-(4-fluorophenyl)-6-methyl-2-oxo-1,2-dihydropyridin-3-carboxamide,
N-(4-(3-(2-aminopyrimidin-4-yl)-4-hydroxyphenoxy)-3-fluorophenyl)-4-ethoxy-1-(4-fluorophenyl)-2-oxo-1,2-dihydropyridin-3-carboxamide,
N-(4-(3-(2-aminopyrimidin-4-yl)-4-hydroxyphenoxy)-3-fluorophenyl)-1-(4-fluorophenyl)-6-methyl-2-oxo-1,2-dihydropyridin-3-carboxamide,
N-(4-(3-(2-aminopyrimidin-4-yl)-4-hydroxyphenoxy)-3-fluorophenyl)-N-(4-fluorophenyl)cyclopropan-1,1-dicarboxamide,
N-(4-(3-(2-aminopyrimidin-4-yl)-4-hydroxyphenoxy)-3-fluorophenyl)-1-(4-fluorophenyl)-4-methoxy-2-oxo-1,2-dihydropyridin-3-carboxamide,
N-(4-(3-(2-aminopyrimidin-4-yl)-4-hydroxyphenoxy)-3-fluorophenyl)-1,5-dimethyl-3-oxo-2-phenyl-2,3-dihydro-1H-pyrazol-4-carboxamide,
N-(4-(3-(2-aminopyrimidin-4-yl)-4-hydroxyphenoxy)-3-fluorophenyl)-5-bromo-1-(4-fluorophenyl)-2-oxo-1,2-dihydropyridin-3-carboxamide,
N-(4-(3-(2-aminopyrimidin-4-yl)-4-hydroxyphenoxy)-3-fluorophenyl)-1-phenyl-5-(trifluoromethyl)-1H-imidazol-4-carboxamide,
N-(4-(3-(2-aminopyrimidin-4-yl)-4-hydroxyphenoxy)-3-fluorophenyl)-1-(4-fluorophenyl)-2-oxo-1,2-dihydropyridin-3-carboxamide,
N-(4-(3-(2-aminopyrimidin-4-yl)-4-hydroxyphenoxy)-3-fluorophenyl)-1-(4-fluorophenyl)-5-(1-methyl-1H-pyrazol-4-yl)-2-oxo-1,2-dihydropyridin-3-carboxamide,
N-(4-(3-(2-aminopyrimidin-4-yl)-4-hydroxyphenoxy)-3-fluorophenyl)-5-cyclopropyl-1-(4-fluorophenyl)-2-oxo-1,2-dihydropyridin-3-carboxamide,
N-(4-(3-(2-aminopyrimidin-4-yl)-4-hydroxyphenoxy)-3-fluorophenyl)-1-(4-fluorophenyl)-2-oxo-6-(trifluoromethyl)-1,2-dihydropyridin-3-carboxamide,
N-(4-(3-(2-aminopyrimidin-4-yl)-4-hydroxyphenoxy)-3-fluorophenyl)-1-(4-fluorophenyl)-6-(hydroxymethyl)-2-oxo-1,2-dihydropyridin-3-carboxamide,
N-(4-(3-(2-aminopyrimidin-4-yl)-4-hydroxyphenoxy)-3-fluorophenyl)-1-phenyl-5-(tri fluoromethyl)-1H-pyrazol-4-carboxamide,
N-(4-(3-(2-aminopyrimidin-4-yl)-4-hydroxyphenoxy)-3-fluorophenyl)-1-(2-hydroxy-2-methylpropyl)-5-methyl-3-oxo-2-phenyl-2,3-dihydro-1H-pyrazol-4-carboxamide,
N-(4-(3-(2-aminopyrimidin-4-yl)-4-hydroxyphenoxy)-3-fluorophenyl)-5-ethyl-1-(pyrimidin-5-yl)-1H-pyrazol-4-carboxamide,
4-ethoxy-N-(3-fluoro-4-(4-hydroxy-3-(pyrimidin-4-yl)phenoxy)phenyl)-1-(4-fluorophenyl)-2-oxo-1,2-dihydropyridin-3-carboxamide,
N-(3-fluoro-4-(4-hydroxy-3-(pyrimidin-4-yl)phenoxy)phenyl)-1-(4-fluorophenyl)-6-methyl-2-oxo-1,2-dihydropyridin-3-carboxamide,
N-(3-fluoro-4-(4-hydroxy-3-(pyrimidin-4-yl)phenoxy)phenyl)-N-(4-fluorophenyl)cyclopropan-1,1-dicarboxamide,
4-ethoxy-N-(3-fluoro-4-(4-hydroxy-3-(1H-pyrazol-3-yl)phenoxy)phenyl)-1-(4-fluoro phenyl)-2-oxo-1,2-dihydropyridin-3-carboxamide,
N-(3-fluoro-4-(4-hydroxy-3-(1H-pyrazol-3-yl)phenoxy)phenyl)-1-(4-fluorophenyl)-6-methyl-2-oxo-1,2-dihydropyridin-3-carboxamide,
N-(3-fluoro-4-(4-hydroxy-3-(isoxazol-5-yl)phenoxy)phenyl)-1-(4-fluorophenyl)-6-methyl-2-oxo-1,2-dihydropyridin-3-carboxamide,
N-(4-(3-(2-aminopyrimidin-5-yl)-4-hydroxyphenoxy)-3-fluorophenyl)-4-ethoxy-1-(4-fluorophenyl)-2-oxo-1,2-dihydropyridin-3-carboxamide, and
N-(4-(3-(2-aminopyridin-4-yl)-4-hydroxyphenoxy)-3-fluorophenyl)-4-ethoxy-1-(4-fluorophenyl)-2-oxo-1,2-dihydropyridin-3-carboxamide.

10. A pharmaceutical composition comprising: the compound or the pharmaceutically acceptable salt thereof according to any one of claim 1 to claim 9; and a pharmaceutically acceptable carrier.
